# EUROPEAN PATENT SPECIFICATION

(11) **EP 3 256 113 B1**
(45) Date of publication and mention of the grant of the patent: **02.04.2025**
(21) Application number: 16748831.1
(22) Date of filing: 09.02.2016
(51) Int. Cl.: A61K 31/166, A61K 38/29, A61K 38/55, A61K 47/12, A61P 5/18, A61P 19/08, A61K 9/20

(54) **TREATMENT OF HYPOPARATHYROIDISM**
BEHANDLUNG VON HYPOPARATHYREOIDISMUS
TRAITEMENT DE L'HYPOPARATHYROÏDIE

(30) Priority: 09.02.2015 US 201562113619 P; 09.02.2015 US 201562113625 P; 09.02.2015 US 201562113638 P; 09.02.2015 US 201562113673 P; 09.02.2015 US 201562113604 P; 09.02.2015 US 201562113629 P; 09.02.2015 US 201562113600 P
(43) Date of publication of application: 20.12.2017
(73) Proprietor: Entera Bio Ltd., 9112002 Jerusalem (IL)
(72) Inventor: BURSHTEIN, Gregory, 7177625 Modiin (IL); ROTHNER, Ariel, 9350614 Jerusalem (IL); SCHWARTZ, Phillip M., 9322823 Jerusalem (IL); GALITZER, Hillel, 7681200 Yad Binyamin (IL)
(74) Representative: Mathys & Squire
(86) International application number: PCT/IL2016/050151
(87) International publication number: WO 2016/128970

(56) References cited:
- WO-A1-00/59863
- WO-A1-03/015822
- WO-A1-2006/076692
- WO-A1-2010/020978
- WO-A1-2010/020978
- US-A1- 2005 054 557
- CUSANO NATALIE E ET AL: "Parathyroid hormone therapy for hypoparathyroidism", BEST PRACTICE & RESEARCH CLINICAL ENDOCRINOLOGY & METABOLISM, BAILLIERE TINDALL, LONDON, GB, vol. 29, no. 1, 10 September 2014 (2014-09-10), pages 47 - 55, XP029189965, ISSN: 1521-690X, DOI: 10.1016/J.BEEM.2014.09.001
- LEONE-BAY A ET AL: "ORAL DELIVERY OF BIOLOGICALLY ACTIVE PARATHYROID HORMONE", PHARMACEUTICAL RESEARCH, SPRINGER NEW YORK LLC, US, vol. 18, no. 7, 1 July 2001 (2001-07-01), pages 964 - 970, XP001106035, ISSN: 0724-8741, DOI: 10.1023/A:1010936227570
- MAHER, S. ET AL.: "Overcoming poor permeability: translating permeation enhancers for oral peptide delivery", DRUG DISCOVERY TODAY: TECHNOLOGIES, vol. 9, no. 2, 31 August 2012 (2012-08-31), pages e113 - 9, XP055127722, DOI: doi:10.1016/j.ddtec.2011.11.006
- WERLE, MARTIN ET AL.: "Oral Protein Delivery: A Patent Review Of Academic And Industrial Approaches", RECENT PATENTS ON DRUG DELIVERY & FORMULATION, vol. 3, no. 2, 1 June 2009 (2009-06-01), pages 94 - 104, XP009134347

## Description

### FIELD AND BACKGROUND

The present invention, in some embodiments thereof, relates to therapy, and more particularly, to compositions for the treatment of hypoparathyroidism by oral administration.

Parathyroid hormone (PTH) is secreted by the parathyroid gland as a polypeptide containing 84 amino acids. PTH regulates serum calcium levels by enhancing release of calcium from bones (bone resorption), and by enhancing absorption of calcium in the intestines.

Teriparatide is a recombinant form of the first 34 amino acids of human PTH (PTH (1-34)), and is used for treatment of osteoporosis. Administration is by subcutaneous injection once per day at a dose of 20 µg [Riek & Towler, Mo Med 2011, 108:118-123].

PTH (including PTH (1-34)) has been reported to enhance bone growth provided that it is administered intermittently, with circulating levels returning to control levels within 3 hours [Martin, J Bone Metab 2014, 21:8-20]. In contrast, prolonged elevated PTH levels deplete bones by enhancing bone resorption.

Hypoparathyroidism involves underproduction of PTH, leading to low levels of calcium in the blood, which can cause cramping and tetany.

Intravenous calcium is used to treat the symptom of acute hypocalcemia, and oral calcium, vitamin D and/or its active metabolites (e.g., calcitriol) are used for chronic management of hypoparathyroidism. Although calcium and vitamin D administration can normalize serum calcium, they do not correct diminished bone turnover or restore PTH-dependent renal calcium reabsorption, which can lead to hypercalciuria and eventually to renal damage [Winer et al., J Clin Endocrinol Metab 2012, 97:391-399; Bilezikian et al., J Bone Miner Res 2011, 26:2317-2337]. Thiazide diuretics may be used to enhance renal calcium reabsorption, but can cause hypokalemia and/or hyponatremia [Bilezikian et al., J Bone Miner Res 2011, 26:2317-2337].

Winer et al. [J Clin Endocrinol Metab 2003, 88:4214-4220] describes treatment of hypoparathyroidism by twice-daily subcutaneous injection of PTH (1-34), and reported that normal urinary calcium levels were observed, in contrast to high urinary calcium levels in subjects treated by calcitriol administration.

Winer et al. [J Clin Endocrinol Metab 2008, 93:3389-3395] reported that twice-daily injection of PTH (1-34) is more effective than once-daily injection, as it results in less variation in serum calcium levels, and allows for a lower total daily dose.

Winer et al. [J Clin Endocrinol Metab 2012, 97:391-399] describe use of an insulin pump to administer PTH (1-34) by subcutaneous infusion, and reported that this resulted in less fluctuation in serum calcium, more than 50 % reduction in urine calcium, and a 65 % reduction in the PTH dose needed to maintain normal serum calcium levels (from 37 µg per day to 13 µg per day), as compared with twice-daily subcutaneous injection of PTH (1-34). In addition, pump therapy was reported to result in normal bone turnover, whereas subcutaneous injection of PTH (1-34) twice per day resulted in chronic elevation of bone turnover markers due to overstimulation by the PTH.

Rubin et al. [Osteoporos Int 2010, 21:1927-1934] describes treatment of hypoparathyroidism by subcutaneous injection of 100 µg intact PTH (PTH (1-84)) every other day, in combination with calcium and vitamin D supplementation, and reported that the PTH administration reduced the required doses of calcium and vitamin D.

Oral administration of peptide pharmaceuticals is problematic due to degradation of peptides in the digestive system and poor absorption of large molecules.

U.S. Patent Application Publication No. 2007/0087957 describes compositions for oral administration of a protein, the compositions comprising a protein and an omega-3 fatty acid, as well as the use of such compositions for oral administration of insulin.

Qi & Ping [J Microencapsulation 2004, 21:37-45] describe administration of enteric microspheres containing insulin with SNAC (sodium 8-N-(2-hydroxybenzoyl)aminocaprylate). The enteric microspheres are for protecting the insulin from digestive enzymes of the stomach and small intestine, and the SNAC is for enhancing absorption.

U.S. Patent Application Publication No. 2011/0142800 describes compositions for oral administration of a protein, comprising a protein having a molecular weight of up to 100,000 Da, a protease inhibitor, and an absorption enhancer, such as SNAC, N-(10-[2-hydroxybenzoyl]amino)decanoic acid (SNAD), 8-[N-(2-hydroxy-4-methoxybenzoyl)amino]caprylic acid (4-MOAC), 8-[N-(2-hydroxy-5-chlorobenzoyl)amino]caprylic acid (5-CNAC) and 4-[(4-chloro-2-hydroxybenzoyl)amino]butanoic acid (4-CNAB) and sodium salts thereof.

U.S. Patent No. 8,110,547 describes compositions for buccal administration of parathyroid hormone (PTH). The composition comprises PTH or a fragment or analog thereof, as well as a delivery agent such as 4-MOAC, SNAC, SNAD, 5-CNAC and 4-CNAB.

Additional background art includes Puig-Domingo et al. [Eur J Endocrinol 2008, 159:653-657]; Qi et al. [Acta Pharm Sinica 2004, 39:844-848]; International Patent Application Publications WO 00/50386, WO 01/32130, WO 01/32596, WO 03/045306 and WO 2007/121471; Japanese Patent Application Nos. 2005281231 and 2006111558; and U.S. Patent Application Publication Nos. 2006/0234913 and 2013/0224300 ,WO 03/015822, WO 2006/076692, US 2005/054557, WO 00/59863, WO 2010/020978, Maher et al. [Drug Discovery Today: Technologies (2012) 9(2):E113-E119], Werle et al. [Recent Patents on Drug Delivery and Formulation (2009) 3(2):94-104], Cusano et al. [Best Practice & Research Clinical Endocrinology and Metabolism (2014) 29(1):47-55, which describes treatment of hypoparathyroidism via subcutaneous administration] and Leone-Bay et al. [Pharmaceutical Research (2001) 18(7):964-970, which describes a preliminary study comparing the effect of various absorption enhancers when added to a single administration by gavage to anaesthetised rats.].

### SUMMARY OF THE INVENTION

The claimed invention provides a pharmaceutical composition for use in the treatment of hypoparathyroidism by administration of the composition to a subject in need thereof via oral administration, the composition comprising: teriparatide; at least one protease inhibitor; and SNAC (sodium 8-N-(2-hydroxybenzoyl)aminocaprylate); wherein said composition is for administration via oral administration at least three times per day, and said oral administration is effected at least 4 hours after the most recent food intake.

The at least one protease inhibitor may comprise at least one trypsin inhibitor. The at least one trypsin inhibitor may be selected from the group consisting of lima bean trypsin inhibitor, aprotinin, soybean trypsin inhibitor and ovomucoid trypsin inhibitor. The at least one trypsin inhibitor may comprise soybean trypsin inhibitor.

The treatment may comprise administration via oral administration of said teriparatide in an amount 400 µg to 20 mg per day. The treatment may comprise administration via oral administration of said teriparatide in an amount in a range of from 100 to 3000 µg, such as in a range of from 750 to 3000 µg.

The composition may be for administration via oral administration three or four times per day.

The composition may be formulated as a tablet.

### DETAILED DESCRIPTION

The invention is set out in the appended set of claims. According to the disclosure, there is provided a pharmaceutical composition for use in the treatment of hypoparathyroidism by oral administration of the composition to a subject in need thereof, the composition comprising:
parathyroid hormone or a fragment thereof; and
SNAC (sodium 8-N-(2-hydroxybenzoyl)aminocaprylate).

According to some instances of the disclosure , there is provided a use of a composition in the preparation of a medicament for the treatment of hypoparathyroidism by oral administration of the composition to a subject in need thereof, the composition comprising:
parathyroid hormone or a fragment thereof; and
SNAC (sodium 8-N-(2-hydroxybenzoyl)aminocaprylate).

According to an aspect of some instances of the disclosure , there is provided a method of treating hypoparathyroidism in a subject in need thereof, the method comprising orally administering to the subject a composition comprising:
parathyroid hormone or a fragment thereof; and
SNAC (sodium 8-N-(2-hydroxybenzoyl)aminocaprylate).

As claimed, the composition comprises teriparatide.

As claimed, the composition further comprises at least one protease inhibitor.

According to some embodiments of the invention, the at least one protease inhibitor comprises at least one trypsin inhibitor.

According to some embodiments of the invention, the at least one trypsin inhibitor is selected from the group consisting of lima bean trypsin inhibitor, aprotinin, soybean trypsin inhibitor and ovomucoid trypsin inhibitor.

According to some embodiments of the invention, the at least one trypsin inhibitor comprises soybean trypsin inhibitor.

According to some embodiments of the invention, the composition is formulated such that absorption of the parathyroid hormone or fragment thereof following oral administration of the composition is characterized by a Cmax in a range of from 25 pg/ml to 1000 pg/ml.

According to some embodiments of the invention, the method and/or treatment comprises oral administration of the parathyroid hormone or fragment thereof in an amount in a range of from 100 to 3000 µg.

According to some embodiments of the invention, the method and/or treatment comprises oral administration of the parathyroid hormone or fragment thereof in an amount in a range of from 750 to 3000 µg.

According to some instances of the disclosure , the composition is for oral administration at least twice per day.

According to some instances of the disclosure , the oral administration is effected at least twice per day.

According to some embodiments of the invention, the composition is formulated as an extended-release formulation and/or a multimodal release formulation.

According to some instances of the disclosure , the composition is for oral administration once or twice per day.

According to some instances of the disclosure , the oral administration is effected once or twice per day.

According to some embodiments of the invention, the method and/or treatment comprises oral administration of the parathyroid hormone or fragment thereof in an amount in a range of from 200 to 12000 µg.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a bar graph showing maximal plasma concentrations (Cmax) of parathyroid hormone(1-34) as a function of time after oral administration of 200, 400, 680, 1400 or 1800 µg teriparatide according to some embodiments of the invention, and after subcutaneous administration of 20 µg teriparatide;
FIG. 2 is a graph showing plasma concentrations of parathyroid hormone(1-34) as a function of time after oral administration of 1800 µg teriparatide according to some embodiments of the invention, after subcutaneous administration of 20 µg teriparatide, or after administration of a placebo;
FIG. 3 is a graph showing plasma concentrations of cAMP as a function of time after oral administration of 680 µg teriparatide according to some embodiments of the invention, or after subcutaneous administration of 20 µg teriparatide;
FIG. 4 is a graph showing the change in dose of supplemental calcium intake in hypoparathyroidism patients, as a percentage of initial dose (baseline), over the course of a 16-week treatment with oral administration of teriparatide according to some embodiments of the invention (average of results for 17 patients, * indicates p < 0.05 relative to baseline);
FIG. 5 is a graph showing serum phosphorus levels in hypoparathyroidism patients before and 60 minutes after oral administration of teriparatide according to some embodiments of the invention, over the course of a 16-week treatment (average of results for 16 patients, * indicates p < 0.05 between pre-dose and post-dose values);
FIG. 6 is a graph showing teriparatide (PTH(1-34)) serum levels in hypoparathyroidism patients as a function of time following oral administration of teriparatide according to some embodiments of the invention before a meal and after a meal;
FIG. 7 is a bar graph showing average maximal teriparatide (PTH(1-34)) serum levels (average Cmax values) in hypoparathyroidism patients following oral administration of teriparatide according to some embodiments of the invention before a meal and after a meal;
FIG. 8 is a graph showing the dose of supplemental calcium intake (Ca) and urinary calcium levels (Urinary Ca) in an hypoparathyroidism patient, over the course of a 16-week treatment with oral administration of teriparatide according to some embodiments of the invention;
FIG. 9 is a graph showing serum phosphorus levels and albumin adjusted calcium (ACa) levels in an exemplary hypoparathyroidism patient, over the course of a 16-week treatment with oral administration of teriparatide according to some embodiments of the invention;
FIG. 10 is a graph showing teriparatide (PTH(1-34)) serum levels in an exemplary hypoparathyroidism patient as a function of time following oral administration of teriparatide according to some embodiments of the invention before a meal and after a meal;
FIG. 11 is a bar graph showing the time to full dissolution of exemplary 300 mg tablets comprising 1 mg teriparatide and 0 mg (A), 125 mg (B) or 187.5 mg (C) ethyl cellulose;
FIGs. 12A and 12B are bar graphs showing the time to full dissolution of exemplary tablets comprising 0.67 mg teriparatide with 30 weight percents ethyl cellulose (STEC-30) or without ethyl cellulose (ST), at pH 2 (FIG. 12A) and at pH 6 (FIG. 12B);
FIGs. 13A and 13B are bar graphs showing the time to full disintegration of exemplary tablets comprising 0.67 mg teriparatide with 30 weight percents ethyl cellulose (STEC-30) or without ethyl cellulose (ST), at pH 2 (FIG. 13A) and at pH 6 (FIG. 13B);
FIGs. 14A-14C depict exemplary unit dosage forms for use according to some embodiments of the invention;
FIGs. 15A-15C depict exemplary coated unit dosage forms for oral administration according to some embodiments of the invention;
FIG. 16 depicts an exemplary tablet for oral administration according to some embodiments of the invention;
FIG. 17 depicts an exemplary coated tablet for oral administration according to some embodiments of the invention;
FIG. 18 depicts an exemplary external layer of unit dosage forms for oral administration according to some embodiments of the invention;
FIG. 19 depicts an exemplary external layer of a unit dosage form for oral administration according to some embodiments of the invention;
FIG. 20 depicts an exemplary core of a unit dosage form for oral administration according to some embodiments of the invention;
FIG. 21 depicts an exemplary drug delivery system according to some embodiments of the invention;
FIGs. 22A-22C depict an exemplary drug delivery system according to some embodiments of the invention, prior to oral administration (FIG. 22A), and subsequent to oral administration in the stomach (FIG. 22B) and in the intestines (FIG. 22C);
FIG. 23 depicts a casing of an exemplary drug delivery system according to some embodiments of the invention;
FIG. 24 presents a graph showing the release of SNAC, as a function of time, from an exemplary tablet formulation comprising sodium bicarbonate in comparison with an exemplary tablet formulation without sodium bicarbonate; and
FIG. 25 presents a bar graph showing relative absorption of teriparatide from an exemplary oral formulation co-administered with 150 ml of water (H2O) or with an aqueous solution of 3 mg/ml sodium bicarbonate (H2O + NaCO3) (absorption upon co-administration with water defined as 100 %).

### FURTHER DETAILED DESCRIPTION

As claimed, the invention relates compositions for use in treating hypoparathyroidism which are administered via oral administration. Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

The present inventors have now uncovered that hypoparathyroidism can be advantageously treated using oral administration of parathyroid hormone (or a fragment thereof) rather than subcutaneous injection or infusion, by utilizing compositions designed for overcoming the poor absorption of parathyroid hormone upon oral administration.

As claimed, the invention relates compositions comprising teriparatide for use in treating hypoparathyroidism. While investigating the enhancement of absorption of parathyroid hormone by SNAC (sodium 8-N-(2-hydroxybenzoyl)aminocaprylate) upon oral administration, the present inventors have uncovered that compositions comprising SNAC can be used to obtain absorption of parathyroid hormone comparable to absorption obtained using subcutaneous injection or infusion. The present inventors have envisioned that oral administration of such compositions would be particularly useful for treating hypoparathyroidism, in which continuous supplementation of parathyroid hormone is generally advantageous, in a more convenient manner than regimens requiring repeated injections and/or continuous infusion of parathyroid hormone.

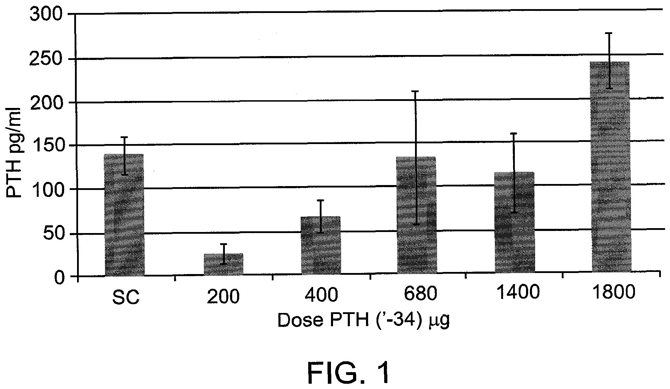

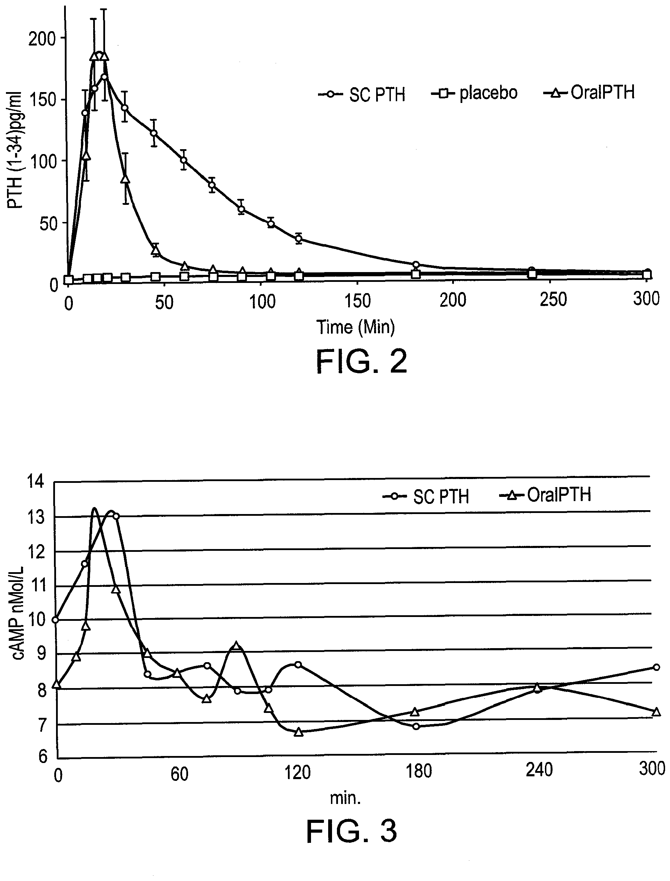

Referring now to the drawings, FIG. 1 shows that the plasma levels of parathyroid hormone (PTH) following oral administration are proportional to the orally administered dose. FIG. 2 shows that peak plasma levels (Cmax) of parathyroid hormone (PTH) following oral administration are comparable to those obtained when the PTH is administered subcutaneously. FIG. 3 shows that the absorbed PTH exhibits a biological effect.

These results indicate that oral administration of compositions comprising PTH as described herein is both an effective and convenient route for administering PTH.

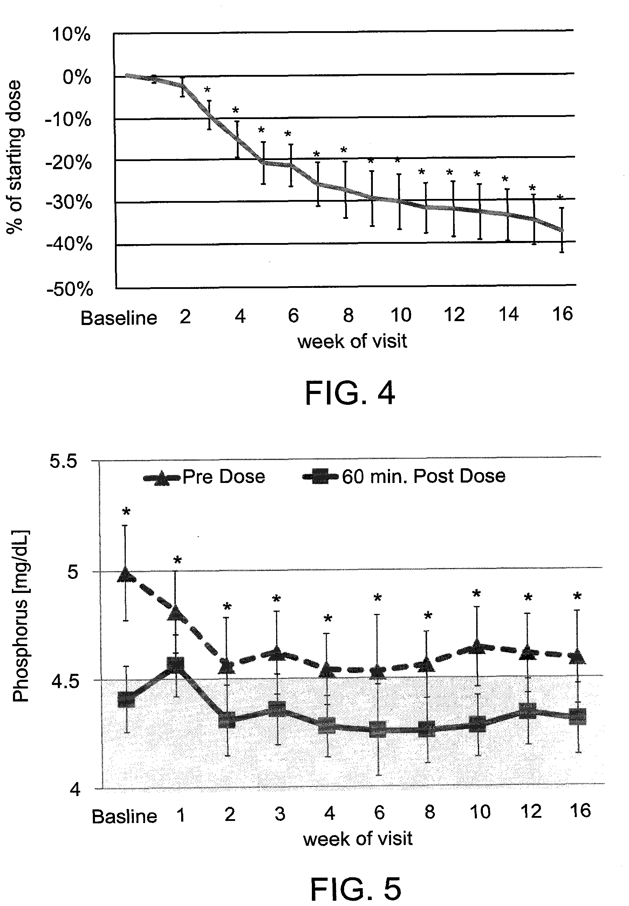

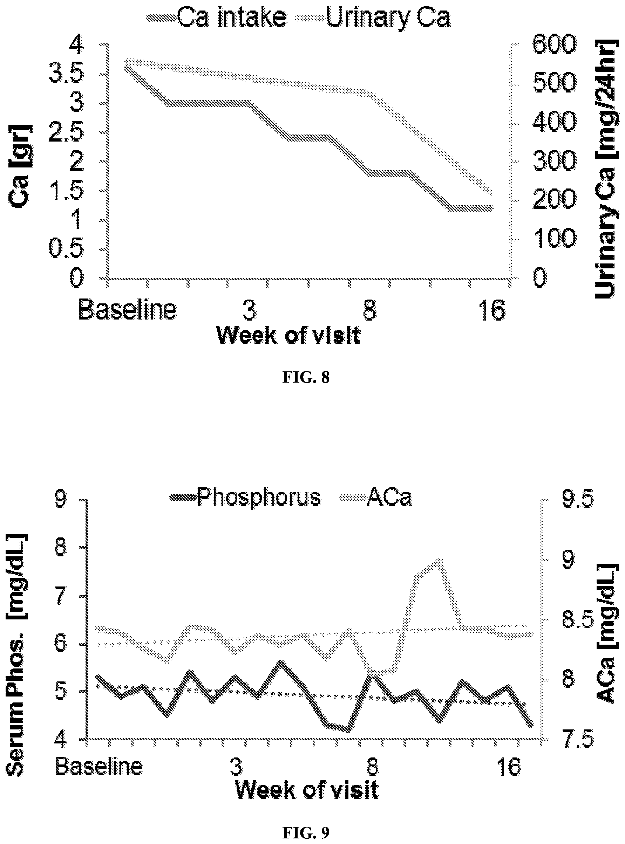

FIGs. 4, 5, 8 and 9 show that orally administered PTH increases serum calcium levels (FIG. 9), decreases serum phosphorus levels (FIGs. 5 and 9), and enables reductions in calcium supplementation doses and in urinary calcium levels (FIGs. 4 and 8) while treating hypoparathyroidism patients.

These results indicate that oral administration of compositions comprising PTH as described herein is effective at treating hypoparathyroidism and symptoms thereof.

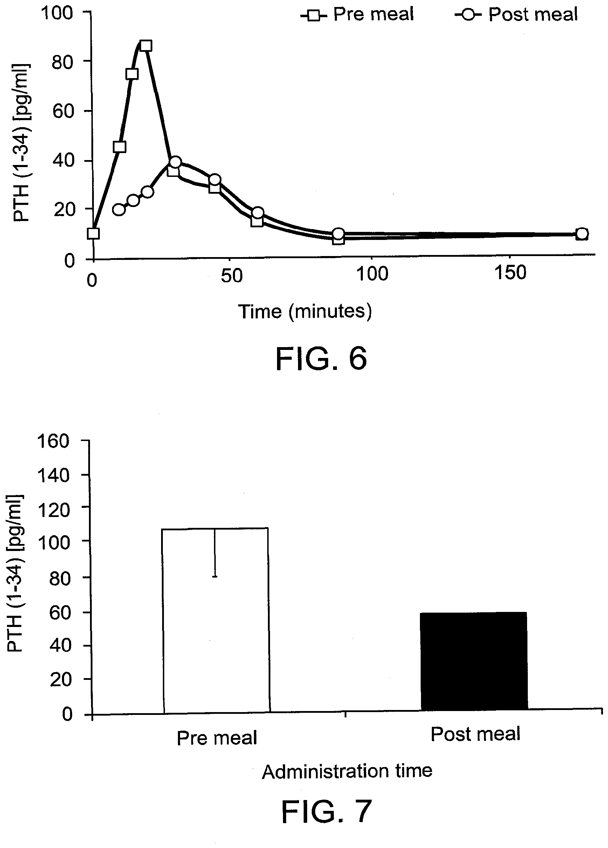

FIGs. 6, 7 and 10 show that the potency of compositions comprising PTH as described herein depends on whether administration is on a full or empty stomach.

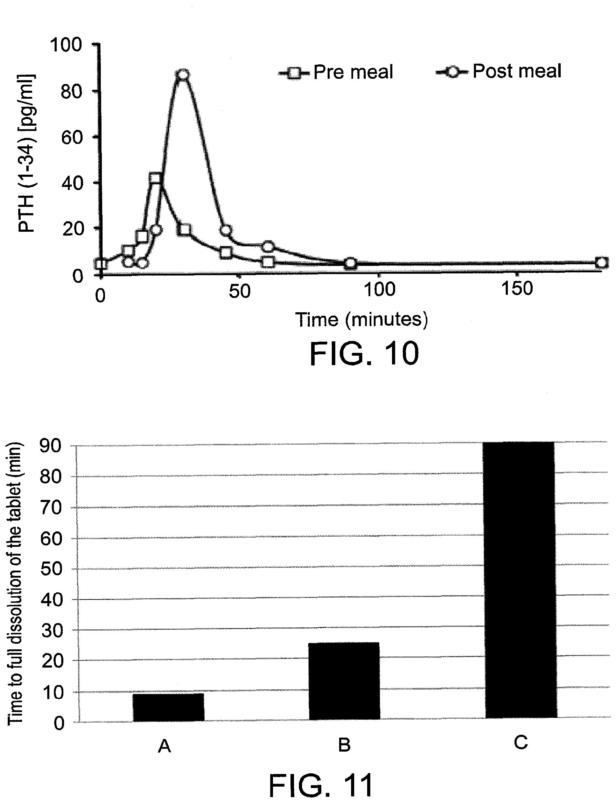

FIGs. 11-13B show that addition of ethyl cellulose to a solid composition containing PTH for oral administration delays the disintegration and dissolution of the composition under gastrointestinal conditions.

These results indicate that a pharmacokinetic profile of orally administered PTH can be controlled in order to be particularly suitable for treating hypoparathyroidism, for example, by releasing PTH gradually over a considerably period of time.

According to the disclosure, there is provided a pharmaceutical composition for use in the treatment of hypoparathyroidism.

The compositions are suitable for treating hypoparathyroidism by oral administration of the composition. That is, the compositions provide a therapeutic effect when administered orally.

The pharmaceutical composition comprises:
parathyroid hormone or a fragment thereof; and
SNAC (sodium 8-N-(2-hydroxybenzoyl)aminocaprylate).

As claimed, the pharmaceutical composition comprises teriparatide; at least one protease inhibitor; and SNAC, wherein said composition is for administration via oral administration at least three times per day, administration is effected at least 4 hours after the most recent food intake. According to the disclosure, there is provided a use of a composition as described herein in the preparation of a medicament for the treatment of hypoparathyroidism by oral administration of the medicament to a subject in need thereof.

According to the disclosure, there is provided a method of treating hypoparathyroidism in a subject in need thereof.

All disclosures herein of methods of treatment or uses of compositions in the manufacture of a medicament relate to compositions for use in the treatment of hypoparathyoridism according to the claimed invention. According to some embodiments of the present invention, the method is effected by orally administering to the subject a composition as described herein.

In some embodiments according to any of the aspects of embodiments described herein, the composition for administration is formulated as one or more unit dosage form(s).

Herein and in the art, the term "parathyroid hormone" refers to an 84-amino acid polypeptide hormone secreted by the parathyroid glands.

Herein, a "fragment" of parathyroid hormone refers to a polypeptide comprising a portion of the abovementioned 84 amino acids of parathyroid hormone. Preferably, the fragment is a fragment which exhibits a biological activity of parathyroid hormone.

Teriparatide is an example of a parathyroid hormone fragment, composed of amino acids 1-34 (i.e., an N-terminal portion) of the full parathyroid hormone polypeptide. The term "teriparatide" is used interchangeably herein with the terms "PTH(1-34)" and "parathyroid hormone(1-34)".

Herein, for the sake of brevity, the term "parathyroid hormone" or its abbreviation "PTH" encompasses parathyroid hormone (having a naturally occurring amino acid sequence, e.g., in humans), fragments thereof and homologs of the parathyroid hormone or the fragment thereof, except where indicated otherwise. For example, the terms "PTH(1-84)" and "parathyroid hormone(1-84)" (which are used interchangeably herein) refer herein in particular to the full 84-amino acid parathyroid hormone polypeptide, whereas the term "PTH(1-34)" refers herein to a particular fragment of parathyroid hormone (teriparatide).

Without being bound by any particular theory, it is believed that PTH tends to be poorly absorbed upon oral administration due to its relatively large molecular weight and/or due to its polarity; and therefore, their absorption is particularly susceptible to enhancement by SNAC activity.

### Methods and compositions:

As used herein, the phrase "pharmaceutical composition" (also referred to herein, for brevity, as "composition") refers to a preparation of a parathyroid hormone (PTH) described herein (e.g., PTH(1-34), PTH(1-84)) with other chemical components such as SNAC, and optionally additional ingredients such as described herein. The purpose of a pharmaceutical composition is to facilitate administration of the PTH.

Accordint to the claimed invention he PTH is teriparatide. According to the claimed invetion, the composition for oral administration further comprises at least one protease inhibitor. Optional species and amounts of protease inhibitor are described in detail herein. According to the claimed invention, the composition comprises a protease inhibitor. In some embodiments of any one of the embodiments described herein, a treatment or method according to any of the respective embodiments described herein comprises oral administration of at least 200 µg of PTH (e.g., PTH(1-34)) per day. In some embodiments, the treatment or method comprises oral administration of at least 400 µg of PTH (e.g., PTH(1-34)) per day. In some embodiments, the treatment or method comprises oral administration of at least 1000 µg of PTH (e.g., PTH(1-34)) per day. In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a treatment or method according to any of the respective embodiments described herein comprises oral administration of 20 mg or less of PTH (e.g., PTH(1-34)) per day. In some embodiments, the treatment or method comprises oral administration of 10 mg or less of PTH (e.g., PTH(1-34)) per day. In some embodiments, the treatment or method comprises oral administration of 6 mg (6000 µg) or less of PTH (e.g., PTH(1-34)) per day. In some embodiments, the treatment or method comprises oral administration of 3 mg (3000 µg) or less of PTH (e.g., PTH(1-34)) per day. In some embodiments, the treatment or method comprises oral administration of 2000 µg or less of PTH (e.g., PTH(1-34)) per day. In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a treatment or method according to any of the respective embodiments described herein comprises oral administration of 200 µg to 20 mg of PTH (e.g., PTH(1-34)) per day.

In some embodiments, the treatment or method comprises oral administration of 400 µg to 20 mg of PTH (e.g., PTH(1-34)) per day. In some embodiments, the treatment or method comprises oral administration of 400 µg to 10 mg of PTH (e.g., PTH(1-34)) per day. In some embodiments, the treatment or method comprises oral administration of from 400 to 6000 µg of PTH (e.g., PTH(1-34)) per day. In some embodiments, the treatment or method comprises oral administration of from 400 to 4000 µg of PTH (e.g., PTH(1-34)) per day. In some embodiments, the treatment or method comprises oral administration of from 1000 to 4000 µg of PTH (e.g., PTH(1-34)) per day. In some embodiments, the treatment or method comprises oral administration of from 2000 to 4000 µg of PTH (e.g., PTH(1-34)) per day. In some embodiments, the treatment or method comprises oral administration of about 3000 µg of PTH (e.g., PTH(1-34)) per day. In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, the oral administration according to any of the respective embodiments described herein is effected at least twice per day. In some embodiments, the composition (e.g., formulated as a unit dosage form) is for oral administration at least twice per day.

In some embodiments of any one of the embodiments described herein, the oral administration according to any of the respective embodiments described herein is effected at least 3 times per day. In some embodiments, the composition (e.g., formulated as a unit dosage form) is for oral administration at least 3 times per day.

As claimed, the composition is for oral administration at least 3 times per day.

In some embodiments of any one of the embodiments described herein, the oral administration according to any of the respective embodiments described herein is effected from 2 to 6 times per day. In some embodiments, the composition (e.g., formulated as a unit dosage form) is for oral administration from 2 to 6 times per day.

In some embodiments of any one of the embodiments described herein, the oral administration according to any of the respective embodiments described herein is effected from 3 to 6 times per day. In some embodiments, the composition (e.g., formulated as a unit dosage form) is for oral administration from 3 to 6 times per day.

In some embodiments of any one of the embodiments described herein, the oral administration according to any of the respective embodiments described herein is effected 3 times per day. In some embodiments, the composition (e.g., formulated as a unit dosage form) is for oral administration 3 times per day.

In some embodiments of any one of the embodiments described herein, the oral administration according to any of the respective embodiments described herein is effected at least 4 times per day (e.g., from 4 to 6 times per day). In some embodiments, the composition (e.g., formulated as a unit dosage form) is for oral administration at least 4 times per day (e.g., from 4 to 6 times per day).

In some embodiments of any one of the embodiments described herein, the oral administration according to any of the respective embodiments described herein is effected 4 times per day. In some embodiments, the composition (e.g., formulated as a unit dosage form) is for oral administration 4 times per day.

Without being bound by any particular theory, it is believed that effecting oral administration at least 3 times per day (e.g., at least 4 times per day) as described in any of the respective embodiments herein, thereby dividing the daily dosage into at least 3 orally administered doses (optionally at least 3 equal doses), provides a relatively steady increase in PTH levels in the body, which is advantageous in the treatment of hypoparathyroidism.

In some embodiments of any one of the embodiments described herein, a composition for each oral administration as described herein comprises at least 100 µg of PTH (e.g., PTH(1-34)). In some embodiments, the composition comprises at least 200 µg of PTH (e.g., PTH(1-34)). In some embodiments, the composition comprises at least 500 µg of PTH (e.g., PTH(1-34)). In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a composition for each oral administration as described herein comprises 10 mg or less of PTH (e.g., PTH(1-34)). In some embodiments, the composition comprises 6 mg (6000 µg) or less of PTH (e.g., PTH(1-34)). In some embodiments, the composition comprises 3 mg (3000 µg) or less of PTH (e.g., PTH(1-34)). In some embodiments, the composition comprises 2000 µg or less of PTH (e.g., PTH(1-34)). In some embodiments, the composition comprises 1000 µg or less of PTH (e.g., PTH(1-34)).

In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a composition for each oral administration as described herein comprises from 100 µg to 10 mg of PTH (e.g., PTH(1-34)). In some embodiments, the composition comprises from 100 µg to 6 mg of PTH (e.g., PTH(1-34)). In some embodiments, the composition comprises from 100 µg to 3 mg of PTH (e.g., PTH(1-34)). In some embodiments, the composition comprises from 750 µg to 3 mg of PTH (e.g., PTH(1-34)). In some embodiments, the composition comprises from 100 to 1500 µg of PTH (e.g., PTH(1-34)). In some embodiments, the composition comprises from 200 to 1000 µg of PTH (e.g., PTH(1-34)). In some embodiments, the composition comprises from 500 to 1000 µg of PTH (e.g., PTH(1-34)). In some embodiments, the composition comprises about 750 µg of PTH (e.g., PTH(1-34)). In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a composition for each oral administration is formulated as a unit dosage form (e.g., according to any of the respective embodiments described herein).

In some embodiments of any one of the embodiments described herein, the composition for oral administration is formulated as an extended-release formulation and/or a multimodal release formulation.

Herein and in the art, the phrase "extended-release formulation" refers to a formulation which releases an active agent gradually (i.e., over an extended period of time) following administration.

Herein, the phrase "multimodal release formulation" refers to a formulation which releases an active agent such that the release profile (release as a function of time following administration) has two or more peaks.

Many techniques for preparing an extended-release formulation are known in the art, which may be used in some embodiments of the invention. In addition, suitable extended-release formulations devised by the inventors are described in detail herein.

Without being bound by any particular theory, it is believed that an extended-release formulation is useful for providing a relatively steady increase in PTH levels in the body, as is advantageous in the treatment of hypoparathyroidism, without necessitating many oral administrations per day.

In some embodiments of any one of the embodiments described herein, oral administration (e.g., oral administration of an extended-release formulation comprising PTH and SNAC) is effected once or twice per day. In some embodiments, oral administration (e.g., oral administration of an extended-release formulation comprising PTH and SNAC) is effected once per day.

Optionally, an extended-release formulation comprises a larger amount of PTH and/or SNAC (as compared to other formulations), in order to facilitate release of therapeutically effective amounts of PTH and SNAC over an extended period of time.

In some embodiments of any one of the embodiments described herein, a composition formulated as an extended-release formulation (e.g., according to any of the respective embodiments described herein) for each oral administration as described herein comprises at least 100 µg of PTH (e.g., PTH(1-34)). In some embodiments, the composition comprises at least 200 µg of PTH (e.g., PTH(1-34)). In some embodiments, the composition comprises at least 500 µg of PTH (e.g., PTH(1-34)). In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a composition formulated as an extended-release formulation (e.g., according to any of the respective embodiments described herein) for each oral administration as described herein comprises 20 mg or less of PTH (e.g., PTH(1-34)). In some embodiments, the composition comprises 10 mg or less of PTH (e.g., PTH(1-34)). In some embodiments, the composition comprises 6 mg (6000 µg) or less of PTH (e.g., PTH(1-34)). In some embodiments, the composition comprises 3 mg (3000 µg) or less of PTH (e.g., PTH(1-34)). In some embodiments, the composition comprises 2000 µg or less of PTH (e.g., PTH(1-34)). In some embodiments, the composition comprises 1000 µg or less of PTH (e.g., PTH(1-34)). In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, a composition formulated as an extended-release formulation (e.g., according to any of the respective embodiments described herein) for each oral administration as described herein comprises from 100 µg to 20 mg of PTH (e.g., PTH(1-34)). In some embodiments, the composition comprises from 200 µg to 10 mg of PTH (e.g., PTH(1-34)). In some embodiments, the composition comprises from 200 µg to 6 mg (6000 µg) of PTH (e.g., PTH(1-34)). In some embodiments, the composition comprises from 200 µg to 3 mg (3000 µg) of PTH (e.g., PTH(1-34)). In some embodiments, the amount of SNAC is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any of the embodiments described herein relating to a composition for administration which is formulated as one or more unit dosage forms, the unit dosage forms is formulated such that the time to full dissolution of the composition unit dosage form is at least 30 minutes at each of pH 2 and pH 6. In some embodiments, the time until full dissolution is at least 60 minutes at each of the aforementioned pH values. In some embodiments, the time until full dissolution is at least 2 hours. In some embodiments, the time until full dissolution is at least 4 hours.

In some embodiments, the time until full dissolution is at least 6 hours. In some embodiments, the time until full dissolution is at least 8 hours. In some embodiments, the time until full dissolution is at least 12 hours.

Herein throughout, dissolution of a composition (or fraction thereof) is indicated by absence of visible composition (or fraction thereof) at the bottom of the fluid, excluding any fraction of the composition (e.g., a relatively insoluble fraction, for example, comprising a hydrophobic polymer described herein) whose dissolution is not being tested. However, visible material suspended in the liquid is not excluded by the terms "soluble" and "dissolution".

Dissolution at pH 2 is optionally determined in simulated gastric fluid without pepsin, under conditions according to USP 23 Apparatus 2 (paddle) (e.g., 800 ml volume, 50 rotations per minute).

Dissolution at pH 6 is optionally determined in an aqueous solution at 37 °C with citrate (optionally 0.1 M) as buffer, and with gentle stirring according to USP 23 Apparatus 2 (paddle) (e.g., 800 ml volume, 50 rotations per minute).

Without being bound by any particular theory, it is believed that slow dissolution under conditions characteristic of the stomach (e.g., about pH 2) as well as under conditions characteristic of much of the intestines (e.g., about pH 6) indicate slow dissolution and gradual, controlled release of a composition (or fraction thereof) in the gastrointestinal tract. It is further believed that a formulation characterized by a lengthy time until dissolution (e.g., as described hereinabove) can serve as an extended-release formulation (e.g., according to any of the respective embodiments described herein).

In some embodiments, the extended-release formulation (e.g., composition unit dosage form) is formulated such that that absorption of the PTH following oral administration of the composition is characterized by a ratio of AUC to Cmax which is at least 3 hours.

In some embodiments, the ratio of AUC to Cmax is at least 4 hours.

In some embodiments, the ratio of AUC to Cmax is at least 5 hours.

In some embodiments, the ratio of AUC to Cmax is at least 6 hours.

In some embodiments, the ratio of AUC to Cmax is at least 8 hours.

In some embodiments, the ratio of AUC to Cmax is at least 10 hours.

In some embodiments, the ratio of AUC to Cmax is at least 12 hours.

As used herein the term "AUC" refers to the area under a curve which represents levels of an administered agent (e.g., PTH) in the blood (e.g., plasma levels) as a function of time following administration, and can be determined by measuring plasma levels of the agent (e.g., PTH) at various time points following administration, as exemplified herein.

As used herein the term "Cmax" refers to the maximal concentration of an administered agent (e.g., PTH) in the blood (e.g., plasma levels), and can be determined by measuring levels of the agent (e.g., PTH) at various time points following administration, as exemplified herein.

As PTH is normally present to some degree in the blood prior to administration, the area under the baseline levels are excluded from the AUC and Cmax (e.g., by subtracting the baseline level from the measured levels at each time point), such that the AUC and Cmax each represent an aspect of the increase above baseline levels which occurs following administration. The baseline can optionally be determined by measuring levels prior to administration and/or by determining (e.g., by curve-fitting) the baseline to which levels decay after administration. Alternatively or additionally, in embodiments wherein the administered PTH species (e.g., teriparatide) is distinct from endogenous PTH, the measurement of PTH may be selective for the administered PTH species (e.g., using the assay described in the Examples section herein).

The ratio of AUC to Cmax (i.e., AUC divided by Cmax) will depend on the nature of the pharmacokinetic profile of the composition, particularly on the shape of the curve which represents levels of the PTH in the blood (e.g., plasma levels) as a function of time following administration. Pharmacokinetic profiles characterized by a sharp increase and decrease within a brief period of time will tend to have a relatively low ratio of AUC to Cmax, whereas pharmacokinetic profiles characterized by a more gradual increase and decrease over a broader period of time will tend to have a relatively high ratio of AUC to Cmax.

Thus, without being bound by any particular theory, it is believed that a ratio of AUC to Cmax which is at least 3 hours, as described herein according to any of the respective embodiments, is associated with a relatively extended release of PTH and absorption of PTH into the blood.

The ratio of AUC to Cmax is optionally calculated based on data from multiple administrations of the composition. In such cases, a ratio of AUC to Cmax is preferably calculated for each administration, and then the ratios calculated for each administration may be averaged.

Similarly, the Cmax is optionally calculated based on data from multiple administrations of the composition. In such cases, a Cmax value is preferably calculated for each administration, and then the Cmax values calculated for each administration may be averaged.

In some embodiments, the extended-release formulation (e.g., composition unit dosage form) is formulated such that that absorption of the PTH following oral administration of the composition is characterized by a Tmax which is at least 30 minutes.

In some embodiments, the Tmax is at least 60 minutes.

In some embodiments, the Tmax is at least 90 minutes.

In some embodiments, the Tmax is at least 2 hours.

In some embodiments, the Tmax is at least 3 hours.

In some embodiments, the Tmax is at least 4 hours.

In some embodiments, the Tmax is at least 5 hours.

In some embodiments, the Tmax is at least 6 hours.

In some embodiments, the Tmax is at least 8 hours.

As used herein the term "Tmax" refers to the duration of time between administration and when maximal concentration of an agent (e.g., PTH) in the blood (e.g., plasma levels) occurs.

In some embodiments, the extended-release formulation (e.g., composition unit dosage form) is formulated such that that absorption of the PTH following oral administration of the composition is characterized by a duration of time of at least 60 minutes between administration and when concentration of PTH in the blood (e.g., plasma levels) decreases below the half-maximal concentration (i.e., Cmax/2) of PTH, after Cmax has been reached and the concentration begins to decline. It is to be appreciated that such a duration of time is by definition longer than Tmax. In some embodiments, such a duration of time is at least 90 minutes. In some embodiments, such a duration of time is at least 2 hours. In some embodiments, such a duration of time is at least 3 hours. In some embodiments, such a duration of time is at least 4 hours. In some embodiments, such a duration of time is at least 5 hours. In some embodiments, such a duration of time is at least 6 hours. In some embodiments, such a duration of time is at least 8 hours. In some embodiments, such a duration of time is at least 10 hours. In some embodiments, such a duration of time is at least 12 hours.

In some embodiments of any one of the embodiments described herein, the composition (e.g., composition unit dosage form) is formulated such that absorption of the PTH following oral administration of the composition is characterized by a Cmax of from 15 pg/ml to 1000 pg/ml.

In some embodiments of any one of the embodiments described herein, the composition (e.g., composition unit dosage form) comprises PTH(1-34) and is formulated such that absorption of the PTH(1-34) following oral administration of the composition is characterized by a Cmax of from 15 pg/ml to 1000 pg/ml. In some embodiments, the Cmax is from 25 to 1000 pg/ml. In some embodiments, the Cmax is from 50 to 1000 pg/ml. In some embodiments, the Cmax is from 100 to 1000 pg/ml. In some embodiments, the Cmax is from 200 to 1000 pg/ml.

In some embodiments of any one of the embodiments described herein, the composition (e.g., composition unit dosage form) comprises PTH(1-34) and is formulated such that absorption of the PTH(1-34) following oral administration of the composition is characterized by a Cmax of from 15 pg/ml to 500 pg/ml. In some embodiments, the Cmax is from 25 to 500 pg/ml. In some embodiments, the Cmax is from 50 to 500 pg/ml. In some embodiments, the Cmax is from 100 to 500 pg/ml. In some embodiments, the Cmax is from 200 to 500 pg/ml.

In some embodiments of any one of the embodiments described herein, the composition (e.g., composition unit dosage form) comprises PTH(1-84) and is formulated such that absorption of the PTH(1-84) following oral administration of the composition is characterized by a Cmax of from 30 pg/ml to 1000 pg/ml. In some embodiments, the Cmax is from 100 to 1000 pg/ml. In some embodiments, the Cmax is from 250 to 1000 pg/ml. In some embodiments, the Cmax is from 500 to 1000 pg/ml.

In some embodiments of any one of the embodiments described herein, the composition (e.g., composition unit dosage form) is formulated such that absorption of the PTH following oral administration of the composition is characterized by a Cmax of from 25 pg/ml to 500 pg/ml.

In some embodiments of any one of the embodiments described herein, the composition (e.g., composition unit dosage form) comprises PTH(1-34) and is formulated such that absorption of the PTH(1-34) following oral administration of the composition is characterized by a Cmax of from 25 pg/ml to 250 pg/ml.

In some embodiments of any one of the embodiments described herein, the composition (e.g., composition unit dosage form) comprises PTH(1-84) and is formulated such that absorption of the PTH(1-84) following oral administration of the composition is characterized by a Cmax of from 50 pg/ml to 500 pg/ml.

In some embodiments of any one of the embodiments described herein, the composition (e.g., composition unit dosage form) is formulated such that absorption of the PTH following oral administration of the composition is characterized by a Cmax of from 100 pg/ml to 500 pg/ml.

In some embodiments of any one of the embodiments described herein, the composition (e.g., composition unit dosage form) comprises PTH(1-34) and is formulated such that absorption of the PTH(1-34) following oral administration of the composition is characterized by a Cmax of from 100 pg/ml to 250 pg/ml.

In some embodiments of any one of the embodiments described herein, the composition (e.g., composition unit dosage form) comprises PTH(1-84) and is formulated such that absorption of the PTH(1-84) following oral administration of the composition is characterized by a Cmax of from 200 pg/ml to 500 pg/ml.

In some embodiments of any one of the embodiments described herein, the composition (e.g., composition unit dosage form) is formulated such that absorption of the PTH following oral administration of the composition is characterized by a Cmax of from 25 pg/ml to 100 pg/ml.

In some embodiments of any one of the embodiments described herein, the composition (e.g., composition unit dosage form) comprises PTH(1-34) and is formulated such that absorption of the PTH(1-34) following oral administration of the composition is characterized by a Cmax of from 25 pg/ml to 50 pg/ml.

In some embodiments of any one of the embodiments described herein, the composition (e.g., composition unit dosage form) comprises PTH(1-84) and is formulated such that absorption of the PTH(1-84) following oral administration of the composition is characterized by a Cmax of from 50 pg/ml to 100 pg/ml.

In some embodiments of any one of the embodiments described herein, any amount (e.g., a Cmax) of any PTH (e.g., including a fragment or homolog according to any of the respective embodiments described herein) is a molar equivalent to the corresponding amount (e.g., a Cmax) for PTH(1-34) and/or PTH(1-84) according to any of the respective embodiments described herein. An amount according to such embodiments may be determined by multiplying the amount of PTH(1-34) and/or PTH(1-84) described herein by the ratio of molecular weight of the PTH to be administered according to such an embodiment to the molecular weight of the PTH(1-34) and/or PTH(1-84).

In some embodiments of any one of the embodiments described herein, a bioavailability of the PTH (e.g., PTH(1-34)) upon oral administration of the composition is in a range of from 0.05 to 50 %. In some embodiments, the bioavailability is in a range of from 0.1 to 15 %. In some embodiments, the bioavailability is in a range of from 0.2 to 5 %. In some embodiments, the bioavailability is in a range of from 0.5 to 3 %.

Without being bound by any particular theory, it is believed that SNAC enhances the bioavailability of the PTH considerably.

In some embodiments of any one of the embodiments described herein, a bioavailability of the PTH (e.g., PTH(1-34)) upon oral administration of the composition is at least 50 % higher than (150 % of the level of) a bioavailability of the PTH (e.g., PTH(1-34)) upon oral administration of an equivalent composition which lacks SNAC (e.g., being identical in all aspects except for the absence of SNAC). In some embodiments, the bioavailability is at least twice (200 % of the level of) the bioavailability upon oral administration of an equivalent composition which lacks SNAC. In some embodiments, the bioavailability is at least four-fold (400 % of the level of) the bioavailability upon oral administration of an equivalent composition which lacks SNAC. In some embodiments, the bioavailability is at least ten-fold (1000 % of the level of) the bioavailability upon oral administration of an equivalent composition which lacks SNAC. In some embodiments, the bioavailability is at least twenty-fold (2000 % of the level of) the bioavailability upon oral administration of an equivalent composition which lacks SNAC. In some embodiments, the bioavailability is at least fifty-fold (5000 % of the level of) the bioavailability upon oral administration of an equivalent composition which lacks SNAC.

Some of the administered agents according to various embodiments described herein comprise polypeptides or proteins, for example, PTH and many of the protease inhibitors described herein. Any of these polypeptides are to be interpreted in line the definition of the term "polypeptide" hereinafter.

Any formulation which provides desired pharmacokinetic parameters according to any of the respective embodiments described herein is suitable for use according to embodiments of the invention in the treatment of the indicated medical conditions, and is encompassed by the terms "pharmaceutical composition", "medicament" and "drug delivery system" recited herein.

Such formulations may include ingredients or combinations of ingredients known to a person skilled in the art as providing the desired pharmacokinetic parameters according to any of the respective embodiments described herein.

Any of the compositions and unit dosage forms described herein may optionally consist essentially of the ingredients described herein (e.g., PTH, SNAC, and optionally at least one protease inhibitor), or alternatively, the composition further comprises suitable pharmaceutically acceptable carriers or excipients.

Hereinafter, the phrases "physiologically acceptable carrier" and "pharmaceutically acceptable carrier", which may be interchangeably used, refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

The term "unit dosage form", as used herein, describes physically discrete units, each unit containing a predetermined quantity of one or more active ingredient(s) calculated to produce the desired therapeutic effect, in association with at least one pharmaceutically acceptable carrier, diluent, excipient, or combination thereof.

In some embodiments of any one of the embodiments described herein, the composition is formulated as a solid composition. In some embodiments, the composition is formulated as a tablet.

In some embodiments of any one of the embodiments described herein, the composition consists primarily of the combination of PTH, SNAC, and optional at least one protease inhibitor described herein, that is, at least 50 weight percents of the composition consists of ingredients selected from the group consisting of a PTH, SNAC and (optional) at least one protease inhibitor. In some embodiments, at least 60 weight percents of the composition consists of a PTH, SNAC and (optional) at least one protease inhibitor. In some embodiments, at least 70 weight percents of the composition consists of a PTH, SNAC and (optional) at least one protease inhibitor.

In some embodiments, at least 80 weight percents of the composition consists of a PTH, SNAC and (optional) at least one protease inhibitor. In some embodiments, at least 90 weight percents of the composition consists of a PTH, SNAC and (optional) at least one protease inhibitor. In some embodiments, at least 95 weight percents of the composition consists of a PTH, SNAC and (optional) at least one protease inhibitor. In some embodiments, at least 98 weight percents of the composition consists of a PTH, SNAC and (optional) at least one protease inhibitor.

In some embodiments, the composition is formulated as a tablet.

Techniques for formulation and administration of drugs may be found in "Remington's Pharmaceutical Sciences," Mack Publishing Co., Easton, PA, latest edition .

Pharmaceutical compositions of some embodiments of the invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with some embodiments of the invention thus may be formulated in conventional manner using one or more physiologically acceptable carriers comprising excipients and auxiliaries, which facilitate processing of the active ingredients into preparations which, can be used pharmaceutically.

The pharmaceutical composition can be formulated readily by combining the active compounds with pharmaceutically acceptable carriers well known in the art as being suitable for oral administration. Such carriers optionally facilitate formulation of the pharmaceutical composition as tablets, pills, dragees, capsules, liquids, gels, syrups, slurries, suspensions, and the like, for oral ingestion by a patient.

Pharmacological preparations for oral use can be made using a solid excipient, optionally grinding the resulting mixture, and processing the mixture of granules, after adding suitable auxiliaries if desired, to obtain tablets or dragee cores.

Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate; and/or lubricants such as talc or magnesium stearate.

In some embodiments of any one of the embodiments described herein, the composition (e.g., formulated as a tablet) further comprises a lubricant. In some embodiments, the lubricant is included in a concentration of 5 weight percents or less, optionally 2 weight percents or less, and optionally about 1 weight percent. In some embodiments, the composition (e.g., formulated as a tablet) consists essentially of the PTH (as described herein), SNAC, lubricant and optionally at least one protease inhibitor (as described herein). In some embodiments, the lubricant is magnesium stearate.

Dragee cores are optionally provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added.

In some embodiments of any of the embodiments described herein, the composition comprising PTH and SNAC (according to any of the respective embodiments described herein) is coated by an enteric coating. In some embodiments, the enteric coating increases bioavailability of the PTH by reducing inactivation of SNAC and/or PTH which is induced by exposure to gastric conditions.

Herein, the phrase "enteric coating" refers to a coating which dissolves under conditions in the intestines (e.g., in an aqueous environment having a pH of at least 5.5 and/or in the presence of colonic bacteria), but does not dissolve under conditions in the stomach (e.g., in an aqueous environment having a pH in a range of from 1 to 3.5). An enteric coating may optionally dissolve in the duodenum, optionally in the jejunum, optionally in the ileum, and optionally in the colon, thereby exposing the core.

In some embodiments of any of the embodiments described herein relating to an enteric coating, the enteric coating comprises at least one enteric polymer. In some such embodiments, a concentration of enteric polymer in the enteric coating is at least 20 weight percents. In some such embodiments, a concentration of the enteric polymer(s) in the enteric coating is at least 30 weight percents. In some such embodiments, a concentration of the enteric polymer(s) in the enteric coating is at least 40 weight percents. In some such embodiments, a concentration of the enteric polymer(s) in the enteric coating is at least 50 weight percents. In some such embodiments, a concentration of the enteric polymer(s) in the enteric coating is at least 60 weight percents. In some such embodiments, a concentration of the enteric polymer(s) in the enteric coating is at least 70 weight percents. In some such embodiments, a concentration of the enteric polymer(s) in the enteric coating is at least 80 weight percents. In some such embodiments, a concentration of the enteric polymer(s) in the enteric coating is at least 90 weight percents. In some such embodiments, the enteric coating consists essentially of the enteric polymer(s).

As used herein, the term "enteric polymer" refers to a solid polymer or mixture of polymers which is soluble in an aqueous solution at a pH in a range of from 5.5 to 8 (i.e., soluble in at least a portion of the aforementioned pH range), but not soluble in an aqueous solution at any pH in a range of from 1 to 3.5, and preferably insoluble at any pH in a range of from 1 to 5.5. When an enteric polymer is a mixture of polymers, the mixture is considered herein to be soluble under any given conditions if at least a portion of the polymers in the mixture are soluble under such conditions, provided that dissolution of the soluble polymer(s) results in full disintegration of the mixture (optionally disintegration to particles of no more than 1 mm in diameter, and optionally no more than 0.1 mm in diameter).

The pH dependency of solubility of an enteric polymer allows the enteric polymer to be in a form of a solid coating in the stomach, as well as under dry conditions (e.g., prior to oral administration), while becoming soluble in at least a portion of the intestines.

Many enteric polymers are known in the art, and the skilled person will be readily capable of selecting and preparing a suitable enteric polymer for dissolving at a pre-determined pH and/or in a pre-determined region of the intestines.

Examples of enteric polymers include, without limitation, copolymers of one or more hydrophobic monomers and one or more anionic monomers (e.g., monomers containing a carboxylic acid and/or carboxylate salt group), optionally with about a 1:1 ratio of hydrophobic monomers to anionic monomers). Such copolymers are anionic, and consequently water-soluble, at a pH of about 7, but relatively non-ionic and hydrophobic, and consequently water-insoluble, at a pH which is sufficiently to result in protonation of almost all of the anionic groups.

Examples of such copolymers used in the art (e.g., commercially available as Eudragit^{®} products) include, without limitation, copolymers wherein anionic monomers are acrylic acid and/or methacrylic acid monomers, and hydrophobic monomers are esters (e.g., alkyl esters) of acrylic acid and/or methacrylic acid monomers, for example, ethyl acrylate, methyl acrylate, ethyl methacrylate and/or methyl methacrylate monomers. For example, poly(methacylic acid-co-ethyl acrylate) (with about a 1:1 ratio of methacrylic acid to ethyl acrylate) is commercially available, e.g., as Eudragit^{®} L100-55.

Further examples of enteric polymers include, without limitation, polyvinyl acetate phthalate, cellulose acetate succinate, cellulose acetate phthalate, hydroxypropyl methyl cellulose phthalate, hydroxypropyl methyl cellulose acetate succinate, and cellulose acetate trimellitate.

In some embodiments of any one of the embodiments described herein relating to an enteric polymer, the enteric polymer is soluble in an aqueous solution at pH 5.5.

In some such embodiments, dissolution of the enteric polymer commences soon after the enteric-coated composition reaches the intestines, for example, in the duodenum.

In some embodiments of any one of the embodiments described herein relating to an enteric coating, the enteric coating is soluble in an aqueous solution at pH 5.5. In some such embodiments, dissolution of the enteric coating commences soon after the enteric-coated composition reaches the intestines, for example, in the duodenum.

In some embodiments of any one of the embodiments described herein relating to an enteric polymer, the enteric polymer is not soluble in an aqueous solution at pH 5.5, and is soluble in an aqueous solution at pH 6.0. In some such embodiments, dissolution of the enteric polymer commences relatively soon after the enteric-coated composition reaches the intestines, for example, in the duodenum.

In some embodiments of any one of the embodiments described herein relating to an enteric coating, the enteric coating is not soluble in an aqueous solution at pH 5.5, and is soluble in an aqueous solution at pH 6.0. In some such embodiments, dissolution of the enteric coating commences relatively soon after the enteric-coated composition reaches the intestines, for example, in the duodenum.

In some embodiments of any one of the embodiments described herein relating to an enteric polymer, the enteric polymer is not soluble in an aqueous solution at pH 5.5 or 6.0, and is soluble in an aqueous solution at pH 6.5. In some such embodiments, dissolution of the enteric polymer commences in the small intestines (e.g., in the jejunum), although optionally not in the duodenum.

In some embodiments of any one of the embodiments described herein relating to an enteric coating, the enteric coating is not soluble in an aqueous solution at pH 5.5 or 6.0, and is soluble in an aqueous solution at pH 6.5. In some such embodiments, dissolution of the enteric coating commences in the small intestines (e.g., in the jejunum), although optionally not in the duodenum.

In some embodiments of any one of the embodiments described herein relating to an enteric polymer, the enteric polymer is not soluble in an aqueous solution at either pH 5.5, 6.0 or 6.5, and is soluble in an aqueous solution at pH 7.0 and/or at pH 7.5. In some such embodiments, dissolution of the enteric polymer commences in the ileum or colon, and optionally not in the duodenum or jejunum.

In some embodiments of any one of the embodiments described herein relating to an enteric coating, the enteric coating is not soluble in an aqueous solution at either pH 5.5, 6.0 or 6.5, and is soluble in an aqueous solution at pH 7.0 and/or at pH 7.5. In some such embodiments, dissolution of the enteric coating commences in the ileum or colon, and optionally not in the duodenum or jejunum.

It is to be appreciated that in some embodiments when dissolution of an enteric polymer and/or enteric coating commences at any given pH and/or location in the gastrointestinal tract (e.g., as described herein), a significant amount of time may pass until a composition coated by the coating is exposed and/or the coating is disintegrated and/or completely dissolved, as the dissolution of enteric polymer and/or enteric coating is not necessarily a very rapid process. The time until the core is exposed and/or the coating is disintegrated and/or completely dissolved may optionally be controlled, for example, in accordance with the thickness of the enteric coating, wherein thicker enteric coatings are associated with longer dissolution times.

Pharmaceutical compositions suitable for use in context of some embodiments of the invention include compositions wherein the PTH is contained in an amount effective to achieve the intended purpose. More specifically, the composition preferably comprises a therapeutically effective amount of PTH, that is, an amount of PTH effective to prevent, alleviate or ameliorate symptoms of hypoparathyroidism. Furthermore, an amount of SNAC is preferably effective for enhancing absorption of the PTH (e.g., in a manner described herein); and an amount of protease inhibitor is preferably effective for inhibiting degradation of the PTH by a protease.

Determination of a therapeutically effective amount is well within the capability of those skilled in the art, especially in light of the detailed disclosure provided herein.

For any preparation used in the methods of the invention, the therapeutically effective amount or dose can be estimated initially from *in vitro* and cell culture assays. For example, a dose can be formulated in animal models to achieve a desired concentration or titer. Such information can be used to more accurately determine useful doses in humans.

Toxicity and therapeutic efficacy of the PTH described herein can be determined by standard pharmaceutical procedures *in vitro,* in cell cultures or experimental animals. The data obtained from these *in vitro* and cell culture assays and animal studies can be used in formulating a range of dosage for use in human. The dosage may vary depending upon the dosage form employed and the route of administration utilized. The exact formulation and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to provide levels (e.g., plasma levels) of PTH sufficient to induce or suppress a biological effect (minimal effective concentration, MEC). The MEC will vary for each preparation, but can be estimated from *in vitro* data. Dosages necessary to achieve the MEC will depend on individual characteristics. Detection assays for PTH are known in the art and can be used to determine plasma concentrations of PTH.

Depending on the severity and responsiveness of the condition to be treated, dosing can be of a single or a plurality of administrations, with course of treatment lasting from several hours to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of some embodiments of the invention may, if desired, be presented in a pack or dispenser device, such as an FDA approved kit, which may contain one or more unit dosage forms containing the active ingredient(s). The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention may also be prepared (e.g., as described herein), placed in an appropriate container, and labeled for treatment of hypoparathyroidism, as is further detailed herein.

In some embodiments of any one of the embodiments described herein, treatment according to any of the aspects described herein is effected by orally administering the composition on a relatively empty stomach and small intestines.

In some embodiments of any one of the embodiments described herein, oral administration of the composition is effected at least 2 hours after the most recent food intake. In some embodiments, oral administration of the composition is effected at least 4 hours after the most recent food intake. In some embodiments, oral administration of the composition is effected at least 6 hours after the most recent food intake. In some embodiments, oral administration of the composition is effected at least 8 hours after the most recent food intake. In some embodiments, oral administration of the composition is effected at least 10 hours after the most recent food intake. According to the claimed invention, oral administration of the composition is effected at least 4 hours after the most recent food intake.

In some embodiments of any one of the embodiments described herein, oral administration of the composition is effected at least 2 hours after the most recent intake of food or drink. In some embodiments, oral administration of the composition is effected at least 4 hours after the most recent intake of food or drink. In some embodiments, oral administration of the composition is effected at least 6 hours after the most recent intake of food or drink. In some embodiments, oral administration of the composition is effected at least 8 hours after the most recent intake of food or drink. In some embodiments, oral administration of the composition is effected at least 10 hours after the most recent intake of food or drink.

According to the claimed invention, oral administration of the composition is effected at least 4 hours after the most recent food intake. In some embodiments of any one of the embodiments described herein, oral administration of the composition is effected in the morning prior to eating. In some embodiments, oral administration of the composition is effected in the morning prior to eating or drinking. Such administration of the unit dosage form and/or drug delivery device in the morning (e.g., after sleeping) may optionally be the most convenient way for a subject to ensure that a considerable period of time has passed between oral administration and the most recent intake of food (and optionally drink).

In some embodiments of any one of the embodiments described herein, oral administration of the composition is effected at least 10 minutes prior to eating (e.g., a subject should abstain from eating for at least 10 minutes after administration). In some embodiments, oral administration of the composition is effected at least 20 minutes prior to eating. In some embodiments, oral administration of the composition is effected at least 30 minutes prior to eating. In some embodiments, oral administration of the composition is effected at least 60 minutes (1 hour) prior to eating. In some embodiments, oral administration of the composition is effected at least 2 hours prior to eating. In some embodiments, oral administration of the composition is effected at least 3 hours prior to eating. In some embodiments oral administration of the composition is effected at least 4 hours prior to eating.

In some embodiments of any one of the embodiments described herein, oral administration of the composition is effected at least 10 minutes prior to eating or drinking (e.g., a subject should abstain from eating or drinking for at least 10 minutes after administration). In some embodiments, oral administration of the composition is effected at least 20 minutes prior to eating or drinking. In some embodiments, oral administration of the composition is effected at least 30 minutes prior to eating or drinking. In some embodiments, oral administration of the composition is effected at least 60 minutes (1 hour) prior to eating or drinking. In some embodiments, oral administration of the composition is effected at least 2 hours prior to eating or drinking. In some embodiments, oral administration of the composition is effected at least 3 hours prior to eating or drinking. In some embodiments, oral administration of the composition is effected at least 4 hours prior to eating or drinking.

Without being bound by any particular theory, it is believed that food (and optionally drink) in the stomach and small intestines may interact with the SNAC and/or PTH in a manner which is detrimental to absorption of the PTH in an efficient and predictable manner. It is further believed that oral administration of a composition as described herein on an empty stomach (e.g., in the morning) allows the release of PTH and SNAC at various locations in the intestines, while food ingested after the oral administration (e.g., during the day after administration of the composition in the morning) generally remains "behind" the PTH and SNAC in the gastrointestinal tract, thereby reducing interactions between ingested food and the SNAC and/or PTH.

As the composition passes through the gastrointestinal tract after oral administration, the PTH and/or SNAC may optionally be released in a controlled manner (e.g., extended release and/or multi-modal release according to any of the respective embodiments described herein), thereby providing control over the pharmacokinetic profile of the PTH.

Without being bound by any particular theory, it is believed that using a single oral administration to achieve absorption during an extended period of time is advantageous for use of PTH with SNAC in the treatment of hypoparathyroidism, as compared with the use of multiple oral administrations of PTH with SNAC, for which it is not realistic to perform all such administrations on a relatively empty stomach (e.g., as described herein).

### Protease inhibitor:

In some embodiments of any of the embodiments described herein, the composition further comprises at least one protease inhibitor.

According to the claimed invention, the composition comprises at least one protease inhibitor.

Herein throughout, the term "protease inhibitor" refers to a compound which reduces a proteolytic activity of a protease, for example, a proteolytic activity which inactivates a PTH described herein. The term "protease inhibitor" encompasses, for example, both large molecules (e.g., proteins) and small molecules, as well as both naturally occurring compounds and synthetic compounds.

In some embodiments of any of the embodiments described herein, the at least one protease inhibitor comprises at least one trypsin inhibitor. In some embodiments, the at least one protease inhibitor consists essentially of one or more trypsin inhibitor(s).

Examples of trypsin inhibitors which may be utilized in any one of the embodiments described herein include, without limitation, lima bean trypsin inhibitor, aprotinin, soybean trypsin inhibitor, ovomucoid trypsin inhibitor and any combination thereof. In some embodiments, the at least one trypsin inhibitor comprises soybean trypsin inhibitor (SBTI). In some embodiments, the at least one trypsin inhibitor (an optionally the at least one protease inhibitor) consists essentially of SBTI.

In some embodiments of any of the embodiments described herein, the at least one protease inhibitor comprises at least one serpin. In some embodiments, the at least one protease inhibitor consists essentially of one or more serpin(s).

Examples of serpins which may be utilized in any one of the embodiments described herein, include, without limitation, alpha 1-antitrypsin, antitrypsin-related protein, alpha 1-antichymotrypsin, kallistatin, protein C inhibitor, cortisol binding globulin, thyroxine-binding globulin, angiotensinogen, centerin, protein Z-related protease inhibitor, vaspin, monocyte/neutrophil elastase inhibitor, plasminogen activator inhibitor-2, squamous cell carcinoma antigen-1 (SCCA-1), squamous cell carcinoma antigen-2 (SCCA-2), maspin, proteinase inhibitor 6 (PI-6), megsin, serpin B8 (PI-8), serpin B9 (PI-9), bomapin, yukopin, hurpin/headpin, antithrombin, heparin cofactor II, plasminogen activator inhibitor 1, glia-derived nexin, pigment epithelium derived factor, alpha 2-antiplasmin, complement 1-inhibitor, 47 kDa heat shock protein (HSP47), neuroserpin and pancpin.

In some embodiments of any of the embodiments described herein, the at least one protease inhibitor comprises at least one cysteine protease inhibitor. In some embodiments, the at least one protease inhibitor consists essentially of one or more cysteine protease inhibitor(s).

Examples of cysteine protease inhibitors which may be utilized in any one of the embodiments described herein include, without limitation, type 1 cystatins, type 2 cystatins, human cystatins C, D, S, SN, and SA, cystatin E/M, cystatin F, and type 3 cystatins (including kininogens).

In some embodiments of any of the embodiments described herein, the at least one protease inhibitor comprises at least one threonine protease inhibitor. In some embodiments, the at least one protease inhibitor consists essentially of one or more threonine protease inhibitor(s).

Examples of threonine protease inhibitors which may be utilized in any one of the embodiments described herein include, without limitation, bortezomib, MLN-519, ER-807446 and TMC-95A.

In some embodiments of any of the embodiments described herein, the at least one protease inhibitor comprises at least one aspartic protease inhibitor. In some embodiments, the at least one protease inhibitor consists essentially of one or more aspartic protease inhibitor(s).

Examples of aspartic protease inhibitors which may be utilized in any one of the embodiments described herein, include, without limitation, α₂-macroglobulin, pepstatin A, aspartic protease inhibitor 11, aspartic protease inhibitor 1, aspartic protease inhibitor 2, aspartic protease inhibitor 3, aspartic protease inhibitor 4, aspartic protease inhibitor 5, aspartic protease inhibitor 6, aspartic protease inhibitor 7, aspartic protease inhibitor 8, aspartic protease inhibitor 9, pepsin inhibitor Dit33, and protease A inhibitor 3.

In some embodiments of any of the embodiments described herein, the at least one protease inhibitor comprises at least one metalloprotease inhibitor. In some embodiments, the at least one protease inhibitor consists essentially of one or more metalloprotease inhibitor(s).

Examples of metalloprotease inhibitors which may be utilized in any one of the embodiments described herein, include, without limitation, angiotensin-1-converting enzyme inhibitory peptide, antihemorrhagic factor BJ46a, beta-casein, proteinase inhibitor CeKI, venom metalloproteinase inhibitor DM43, carboxypeptidase A inhibitor, smpI, IMPI, alkaline proteinase, latexin, carboxypeptidase inhibitor, antihemorrhagic factor HSF, testican-3, SPOCK3, TIMP1, metalloproteinase inhibitor 1, metalloproteinase inhibitor 2, TIMP2, metalloproteinase inhibitor 3, TIMP3, metalloproteinase inhibitor 4, TIMP4, putative metalloproteinase inhibitor tag-225, tissue inhibitor of metalloprotease, WAP, kazal inhibitor, immunoglobulin, and kunitz and NTR domain-containing protein 1.

Examples of protease inhibitors which may be utilized in any one of the embodiments described herein also include, without limitation, AEBSF-HCl, ε-aminocaproic acid, α1-antichymotypsin, antipain, antithrombin III, α1-antitrypsin, APMSF (4-amidinophenyl-methane sulfonyl-fluoride), sprotinin, benzamidine, chymostatin, DFP (diisopropylfluoro-phosphate), leupeptin, 4-(2-Aminoethyl)-benzenesulfonyl fluoride hydrochloride, PMSF (phenylmethyl sulfonyl fluoride), TLCK (1-chloro-3-tosylamido-7-amino-2-heptanone), TPCK (1-chloro-3-tosylamido-4-phenyl-2-butanone), pentamidine isothionate, pepstatin, guanidium, α2-macroglobulin, a chelating agent of zinc, and iodoacetate.

In some embodiments of any one of the embodiments described herein, the amount of a protease inhibitor in a composition for administration as described herein is at least about 0.1 mg. In some embodiments, the amount of a protease inhibitor in a composition for administration as described herein is at least about 0.2 mg. In some embodiments, the amount of a protease inhibitor in a composition for administration as described herein is at least about 0.3 mg. In some embodiments, the amount of a protease inhibitor in a composition for administration as described herein is at least about 0.4 mg. In some embodiments, the amount of a protease inhibitor in a composition for administration as described herein is at least about 0.6 mg. In some embodiments, the amount of a protease inhibitor in a composition for administration as described herein is at least about 0.8 mg. In some embodiments, the amount of a protease inhibitor in a composition for administration as described herein is at least about 1 mg. In some embodiments, the amount of a protease inhibitor in a composition for administration as described herein is at least about 1.5 mg. In some embodiments, the amount of a protease inhibitor in a composition for administration as described herein is at least about 2 mg. In some embodiments, the amount of a protease inhibitor in a composition for administration as described herein is at least about 2.5 mg. In some embodiments, the amount of a protease inhibitor in a composition for administration as described herein is at least about 3 mg. In some embodiments, the amount of a protease inhibitor in a composition for administration as described herein is at least about 5 mg. In some embodiments, the amount of a protease inhibitor in a composition for administration as described herein is at least about 7 mg. In some embodiments, the amount of a protease inhibitor in a composition for administration as described herein is at least about 10 mg. In some embodiments, the amount of a protease inhibitor in a composition for administration as described herein is at least about 12 mg. In some embodiments, the amount of a protease inhibitor in a composition for administration as described herein is at least about 15 mg. In some embodiments, the amount of a protease inhibitor in a composition for administration as described herein is at least about 20 mg. In some embodiments, the amount of a protease inhibitor in a composition for administration as described herein is at least about 30 mg. In some embodiments, the amount of a protease inhibitor in a composition for administration as described herein is at least about 50 mg. In some embodiments, the amount of a protease inhibitor in a composition for administration as described herein is at least about 70 mg. In some embodiments, the amount of a protease inhibitor in a composition for administration as described herein is at least about 100 mg.

In some embodiments of any one of the embodiments described herein, the amount of a protease inhibitor in a composition for administration as described herein is in a range of from 0.1 to 1 mg. In some embodiments, the amount of a protease inhibitor in a composition for administration as described herein is in a range of from 0.2 to 1 mg. In some embodiments, the amount of a protease inhibitor in a composition for administration as described herein is in a range of from 0.3 to 1 mg.

In some embodiments, the amount of a protease inhibitor in a composition for administration as described herein is in a range of from 0.5 to 1 mg.

In some embodiments of any one of the embodiments described herein, the amount of a protease inhibitor in a composition for administration as described herein is in a range of from 0.1 to 2 mg. In some embodiments, the amount of a protease inhibitor in a composition for administration as described herein is in a range of from 0.2 to 2 mg. In some embodiments, the amount of a protease inhibitor in a composition for administration as described herein is in a range of from 0.3 to 2 mg.

In some embodiments, the amount of a protease inhibitor in a composition for administration as described herein is in a range of from 0.5 to 2 mg. In some embodiments, the amount of a protease inhibitor in a composition for administration as described herein is in a range of from 1 to 2 mg.

In some embodiments of any one of the embodiments described herein, the amount of a protease inhibitor in a composition for administration as described herein is in a range of from 1 to 10 mg. In some embodiments, the amount of a protease inhibitor in a composition for administration as described herein is in a range of from 2 to 10 mg. In some embodiments, the amount of a protease inhibitor in a composition for administration as described herein is in a range of from 3 to 10 mg. In some embodiments, the amount of a protease inhibitor in a composition for administration as described herein is in a range of from 5 to 10 mg.

In some embodiments of any one of the embodiments described herein, the amount of a protease inhibitor in a composition for administration as described herein is in a range of from 1 to 20 mg. In some embodiments, the amount of a protease inhibitor in a composition for administration as described herein is in a range of from 2 to 20 mg. In some embodiments, the amount of a protease inhibitor in a composition for administration as described herein is in a range of from 3 to 20 mg. In some embodiments, the amount of a protease inhibitor in a composition for administration as described herein is in a range of from 5 to 20 mg. In some embodiments, the amount of a protease inhibitor in a composition for administration as described herein is in a range of from 10 to 20 mg.

In some embodiments of any one of the embodiments described herein, the amount of a protease inhibitor in a composition for administration as described herein is in a range of from 10 to 100 mg. In some embodiments, the amount of a protease inhibitor in a composition for administration as described herein is in a range of from 20 to 100 mg. In some embodiments, the amount of a protease inhibitor in a composition for administration as described herein is in a range of from 30 to 100 mg.

In some embodiments, the amount of a protease inhibitor in a composition for administration as described herein is in a range of from 50 to 100 mg.

In some embodiments of any one of the embodiments described herein, the amount of a protease inhibitor in a composition for administration as described herein is in a range of from 10 to 200 mg. In some embodiments, the amount of a protease inhibitor in a composition for administration as described herein is in a range of from 20 to 200 mg. In some embodiments, the amount of a protease inhibitor in a composition for administration as described herein is in a range of from 30 to 200 mg.

In some embodiments, the amount of a protease inhibitor in a composition for administration as described herein is in a range of from 50 to 200 mg. In some embodiments, the amount of a protease inhibitor in a composition for administration as described herein is in a range of from 100 to 200 mg.

In some embodiments of any one of the embodiments described herein, the amount of a protease inhibitor in a composition for administration as described herein is at least about 10 kallikrein inactivator units (k.i.u.). In some embodiments, the amount of a protease inhibitor in a composition for administration as described herein is at least about 12 k.i.u. In some embodiments, the amount of a protease inhibitor in a composition for administration as described herein is at least about 15 k.i.u. In some embodiments, the amount of a protease inhibitor in a composition for administration as described herein is at least about 20 k.i.u. In some embodiments, the amount of a protease inhibitor in a composition for administration as described herein is at least about 30 k.i.u. In some embodiments, the amount of a protease inhibitor in a composition for administration as described herein is at least about 40 k.i.u. In some embodiments, the amount of a protease inhibitor in a composition for administration as described herein is at least about 50 k.i.u. In some embodiments, the amount of a protease inhibitor in a composition for administration as described herein is at least about 70 k.i.u. In some embodiments, the amount of a protease inhibitor in a composition for administration as described herein is at least about 100 k.i.u. In some embodiments, the amount of a protease inhibitor in a composition for administration as described herein is at least about 150 k.i.u. In some embodiments, the amount of a protease inhibitor in a composition for administration as described herein is at least about 200 k.i.u. In some embodiments, the amount of a protease inhibitor in a composition for administration as described herein is at least about 300 k.i.u. In some embodiments, the amount of a protease inhibitor in a composition for administration as described herein is at least about 500 k.i.u. In some embodiments, the amount of a protease inhibitor in a composition for administration as described herein is at least about 700 k.i.u. In some embodiments, the amount of a protease inhibitor in a composition for administration as described herein is at least about 1000 k.i.u. In some embodiments, the amount of a protease inhibitor in a composition for administration as described herein is at least about 1500 k.i.u. In some embodiments, the amount of a protease inhibitor in a composition for administration as described herein is at least about 3000 k.i.u. In some embodiments, the amount of a protease inhibitor in a composition for administration as described herein is at least about 4000 k.i.u. In some embodiments, the amount of a protease inhibitor in a composition for administration as described herein is at least about 5000 k.i.u.

Herein and in the art, a "kallikrein inactivating unit" (k.i.u) refers to an amount of protease inhibitor that has the ability to inhibit 2 units of kallikrein by 50 % (e.g., in aqueous solution at an optimal pH and solution volume for activity of the protease inhibitor).

In some embodiments of any one of the embodiments described herein, a weight ratio of protease inhibitor to PTH (e.g., PTH(1-34)) is in a range of from 1:1 to 5:1 (protease inhibitor: PTH). In some embodiments, a weight ratio of protease inhibitor PTH (e.g., PTH(1-34)) is in a range of from 5:1 to 10:1. In some embodiments, a weight ratio of protease inhibitor to PTH (e.g., PTH(1-34)) is in a range of from 10:1 to 20:1. In some embodiments, a weight ratio of protease inhibitor to PTH (e.g., PTH(1-34)) is in a range of from 20:1 to 30:1. In some embodiments, a weight ratio of protease inhibitor to PTH (e.g., PTH(1-34)) is in a range of from 30:1 to 40:1. In some embodiments, a weight ratio of protease inhibitor to PTH (e.g., PTH(1-34)) is in a range of from 40:1 to 50:1. In some embodiments, a weight ratio of protease inhibitor to PTH (e.g., PTH(1-34)) is in a range of from 50:1 to 75:1. In some embodiments, a weight ratio of protease inhibitor to PTH (e.g., PTH(1-34)) is in a range of from 75:1 to 100:1. In some embodiments, a weight ratio of protease inhibitor to PTH (e.g., PTH(1-34)) is in a range of from 100:1 to 200:1. In some embodiments, a weight ratio of protease inhibitor to PTH (e.g., PTH(1-34)) is in a range of from 200:1 to 300:1. In some embodiments, a weight ratio of protease inhibitor to PTH (e.g., PTH(1-34)) is in a range of from 300:1 to 400:1. In some embodiments, a weight ratio of protease inhibitor to PTH (e.g., PTH(1-34)) is in a range of from 400:1 to 500:1. In some embodiments, the protease inhibitor is soybean trypsin inhibitor.

### SNAC:

In some embodiments of any one of the embodiments described herein, the SNAC may optionally be replaced with a similar compound, such as SNAD (sodium 10-N-(2-hydroxybenzoyl)aminodecanoic acid). As shown below, the structure of SNAD differs from that of SNAC only in the length of the fatty acid moiety.

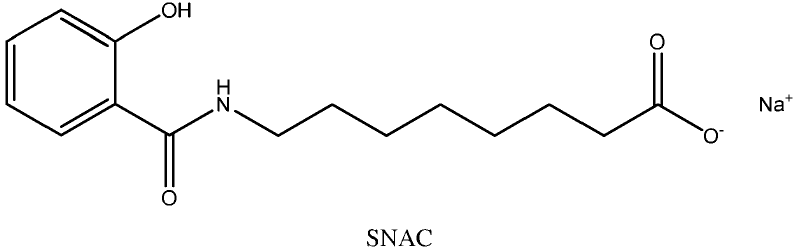

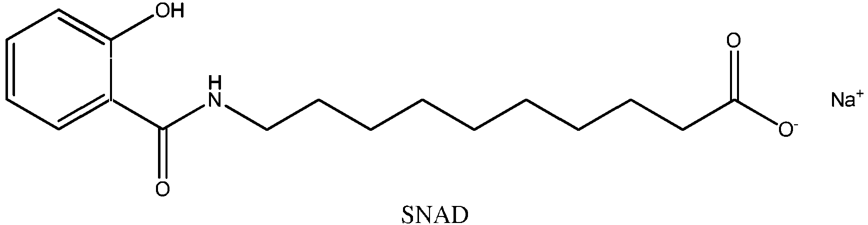

In some embodiments of any one of the embodiments described herein, the SNAC may optionally be replaced with a similar compound, wherein the caprylic acid moiety of SNAC is replaced by another fatty acid moiety at least 6 carbon atoms in length, for example, from 6 to 20 carbon atoms in length, optionally from 6 to 18 carbon atoms in length, optionally from 6 to 16 carbon atoms in length, optionally from 6 to 14 carbon atoms in length, optionally from 6 to 12 carbon atoms in length and optionally from 6 to 10 carbon atoms in length. The fatty acid moiety may be saturated (e.g., as are caprylic acid in SNAC and decanoic acid in SNAD) or unsaturated (i.e., comprising at least one unsaturated carbon-carbon bond).

According to the claimed invention, the composition comprises SNAC.

In some embodiments of any one of the embodiments described herein, a concentration of SNAC in a composition described herein is in a range of from 2.5 to 99.4 weight percents. In some of the aforementioned embodiments, the concentration of SNAC is in a range of from 2.5 to 10 weight percents. In some of the aforementioned embodiments, the concentration of SNAC is in a range of from 8 to 15 weight percents. In some of the aforementioned embodiments, the concentration of SNAC is in a range of from 10 to 20 weight percents. In some of the aforementioned embodiments, the concentration of SNAC is in a range of from 15 to 30 weight percents. In some of the aforementioned embodiments, the concentration of SNAC is in a range of from 20 to 40 weight percents. In some of the aforementioned embodiments, the concentration of SNAC is in a range of from 30 to 50 weight percents. In some of the aforementioned embodiments, the concentration of SNAC is in a range of from 40 to 60 weight percents. In some of the aforementioned embodiments, the concentration of SNAC is in a range of from 50 to 70 weight percents. In some of the aforementioned embodiments, the concentration of SNAC is in a range of from 2.5 to 10 weight percents. In some of the aforementioned embodiments, the concentration of SNAC is in a range of from 2.5 to 10 weight percents. In some of the aforementioned embodiments, the concentration of SNAC is in a range of from 70 to 99.4 weight percents.

In some embodiments of any one of the embodiments described herein, a weight ratio of SNAC to the PTH (e.g., PTH(1-34)) is in a range of from 5:1 to 10:1 (SNAC: PTH). In some embodiments, the ratio is about 7.5:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 10:1 to 20:1 (SNAC: PTH). In some embodiments, the ratio is about 15:1. In some of the aforementioned embodiments, the composition further comprises a protease inhibitor.

In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 20:1 to 30:1 (SNAC: PTH). In some embodiments, the ratio is about 25:1. In some of the aforementioned embodiments, the composition further comprises a protease inhibitor.

In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 30:1 to 50:1 (SNAC: PTH). In some embodiments, the ratio is about 40:1. In some of the aforementioned embodiments, the composition further comprises a protease inhibitor.

In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 50:1 to 100:1 (SNAC: PTH). In some embodiments, the ratio is about 75:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 100:1 to 200:1 (SNAC: PTH). In some embodiments, the ratio is about 150:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 200:1 to 300:1 (SNAC: PTH). In some embodiments, the ratio is about 250:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 300:1 to 500:1 (SNAC: PTH). In some embodiments, the ratio is about 400:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100: 1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, a weight ratio of SNAC to PTH (e.g., PTH(1-34)) is in a range of from 500:1 to 1000:1 (SNAC: PTH). In some embodiments, the ratio is about 750:1. In some embodiments, the composition further comprises a protease inhibitor. In some of the aforementioned embodiments wherein the composition comprises a protease inhibitor, a weight ratio of protease inhibitor to PTH (e.g., PTH(1-34)) is in a range of from 1:1 to 5:1 (protease inhibitor: PTH), optionally about 3:1. In some embodiments, a weight ratio of protease inhibitor to PTH (e.g., PTH(1-34)) is in a range of from 5:1 to 10:1, optionally about 7.5:1. In some embodiments, a weight ratio of protease inhibitor to PTH (e.g., PTH(1-34)) is in a range of from 10:1 to 20:1, optionally about 15:1. In some embodiments, a weight ratio of protease inhibitor to PTH (e.g., PTH(1-34)) is in a range of from 20:1 to 30:1, optionally about 25:1. In some embodiments, a weight ratio of protease inhibitor to PTH (e.g., PTH(1-34)) is in a range of from 30:1 to 40:1, optionally about 35:1. In some embodiments, a weight ratio of protease inhibitor to PTH (e.g., PTH(1-34)) is in a range of from 40:1 to 50:1, optionally about 45:1. In some embodiments, a weight ratio of protease inhibitor to PTH (e.g., PTH(1-34)) is in a range of from 50:1 to 75:1, optionally about 62.5:1. In some embodiments, a weight ratio of protease inhibitor to PTH (e.g., PTH(1-34)) is in a range of from 75:1 to 100:1, optionally about 87.5:1. In some embodiments, a weight ratio of protease inhibitor to PTH (e.g., PTH(1-34)) is in a range of from 100:1 to 200:1, optionally about 150:1. In some embodiments, a weight ratio of protease inhibitor to PTH (e.g., PTH(1-34)) is in a range of from 200:1 to 300:1, optionally about 250:1. In some embodiments, a weight ratio of protease inhibitor to PTH (e.g., PTH(1-34)) is in a range of from 300:1 to 400:1, optionally about 350:1. In some embodiments, a weight ratio of protease inhibitor to PTH (e.g., PTH(1-34)) is in a range of from 400:1 to 500:1, optionally about 450:1. In some embodiments, the protease inhibitor is soybean trypsin inhibitor.

In some embodiments of any one of the embodiments described herein, the amount of SNAC in a composition for administration as described herein is at least about 0.1 mg. In some embodiments, the amount of SNAC in a composition for administration as described herein is at least about 0.2 mg. In some embodiments, the amount of SNAC in a composition for administration as described herein is at least about 0.3 mg. In some embodiments, the amount of SNAC in a composition for administration as described herein is at least about 0.4 mg. In some embodiments, the amount of SNAC in a composition for administration as described herein is at least about 0.6 mg. In some embodiments, the amount of SNAC in a composition for administration as described herein is at least about 0.8 mg. In some embodiments, the amount of SNAC in a composition for administration as described herein is at least about 1 mg. In some embodiments, the amount of SNAC in a composition for administration as described herein is at least about 1.5 mg. In some embodiments, the amount of SNAC in a composition for administration as described herein is at least about 2 mg. In some embodiments, the amount of SNAC in a composition for administration as described herein is at least about 2.5 mg. In some embodiments, the amount of SNAC in a composition for administration as described herein is at least about 3 mg. In some embodiments, the amount of SNAC in a composition for administration as described herein is at least about 5 mg. In some embodiments, the amount of SNAC in a composition for administration as described herein is at least about 7 mg. In some embodiments, the amount of SNAC in a composition for administration as described herein is at least about 10 mg. In some embodiments, the amount of SNAC in a composition for administration as described herein is at least about 12 mg. In some embodiments, the amount of SNAC in a composition for administration as described herein is at least about 15 mg. In some embodiments, the amount of SNAC in a composition for administration as described herein is at least about 20 mg. In some embodiments, the amount of SNAC in a composition for administration as described herein is at least about 30 mg. In some embodiments, the amount of SNAC in a composition for administration as described herein is at least about 50 mg. In some embodiments, the amount of SNAC in a composition for administration as described herein is at least about 70 mg. In some embodiments, the amount of SNAC in a composition for administration as described herein is at least about 100 mg. In some embodiments, the amount of PTH (e.g., PTH(1-34)) is in accordance with any one of the ratios of SNAC to PTH (e.g., PTH(1-34)) described herein. In some embodiments, the composition further comprises at least one protease inhibitor in an amount which is in accordance with any one of the ratios of protease inhibitor to PTH (e.g., PTH(1-34)) described herein.

In some embodiments of any one of the embodiments described herein, the amount of SNAC in a composition for administration as described herein is in a range of from 0.1 to 1 mg. In some embodiments, the amount of SNAC in a composition for administration as described herein is in a range of from 0.2 to 1 mg. In some embodiments, the amount of SNAC in a composition for administration as described herein is in a range of from 0.3 to 1 mg. In some embodiments, the amount of SNAC in a composition for administration as described herein is in a range of from 0.5 to 1 mg.

In some embodiments of any one of the embodiments described herein, the amount of SNAC in a composition for administration as described herein is in a range of from 0.1 to 2 mg. In some embodiments, the amount of SNAC in a composition for administration as described herein is in a range of from 0.2 to 2 mg. In some embodiments, the amount of SNAC in a composition for administration as described herein is in a range of from 0.3 to 2 mg. In some embodiments, the amount of SNAC in a composition for administration as described herein is in a range of from 0.5 to 2 mg. In some embodiments, the amount of SNAC in a composition for administration as described herein is in a range of from 1 to 2 mg.

In some embodiments of any one of the embodiments described herein, the amount of SNAC in a composition for administration as described herein is in a range of from 1 to 10 mg. In some embodiments, the amount of SNAC in a composition for administration as described herein is in a range of from 2 to 10 mg. In some embodiments, the amount of SNAC in a composition for administration as described herein is in a range of from 3 to 10 mg. In some embodiments, the amount of SNAC in a composition for administration as described herein is in a range of from 5 to 10 mg.

In some embodiments of any one of the embodiments described herein, the amount of SNAC in a composition for administration as described herein is in a range of from 1 to 20 mg. In some embodiments, the amount of SNAC in a composition for administration as described herein is in a range of from 2 to 20 mg. In some embodiments, the amount of SNAC in a composition for administration as described herein is in a range of from 3 to 20 mg. In some embodiments, the amount of SNAC in a composition for administration as described herein is in a range of from 5 to 20 mg. In some embodiments, the amount of SNAC in a composition for administration as described herein is in a range of from 10 to 20 mg.

In some embodiments of any one of the embodiments described herein, the amount of SNAC in a composition for administration as described herein is in a range of from 10 to 100 mg. In some embodiments, the amount of SNAC in a composition for administration as described herein is in a range of from 20 to 100 mg. In some embodiments, the amount of SNAC in a composition for administration as described herein is in a range of from 30 to 100 mg. In some embodiments, the amount of SNAC in a composition for administration as described herein is in a range of from 50 to 100 mg.

In some embodiments of any one of the embodiments described herein, the amount of SNAC in a composition for administration as described herein is in a range of from 10 to 200 mg. In some embodiments, the amount of SNAC in a composition for administration as described herein is in a range of from 20 to 200 mg. In some embodiments, the amount of SNAC in a composition for administration as described herein is in a range of from 30 to 200 mg. In some embodiments, the amount of SNAC in a composition for administration as described herein is in a range of from 50 to 200 mg. In some embodiments, the amount of SNAC in a composition for administration as described herein is in a range of from 100 to 200 mg.

In some embodiments of any one of the embodiments described herein, the amount of SNAC in a composition for administration as described herein is in a range of from 10 to 500 mg. In some embodiments, the amount of SNAC in a composition for administration as described herein is in a range of from 20 to 500 mg. In some embodiments, the amount of SNAC in a composition for administration as described herein is in a range of from 30 to 500 mg. In some embodiments, the amount of SNAC in a composition for administration as described herein is in a range of from 50 to 500 mg. In some embodiments, the amount of SNAC in a composition for administration as described herein is in a range of from 100 to 500 mg. In some embodiments, the amount of SNAC in a composition for administration as described herein is in a range of from 200 to 500 mg.

In some embodiments of any one of the embodiments described herein, the amount of SNAC in a composition for administration as described herein is in a range of from 10 to 1000 mg. In some embodiments, the amount of SNAC in a composition for administration as described herein is in a range of from 20 to 1000 mg. In some embodiments, the amount of SNAC in a composition for administration as described herein is in a range of from 30 to 1000 mg. In some embodiments, the amount of SNAC in a composition for administration as described herein is in a range of from 50 to 1000 mg. In some embodiments, the amount of SNAC in a composition for administration as described herein is in a range of from 100 to 1000 mg. In some embodiments, the amount of SNAC in a composition for administration as described herein is in a range of from 200 to 1000 mg. In some embodiments, the amount of SNAC in a composition for administration as described herein is in a range of from 500 to 1000 mg.

In some embodiments of any one of the embodiments described herein, the amount of SNAC in a composition for administration as described herein is in a range of from 10 to 1000 mg. In some embodiments, the amount of SNAC in a composition for administration as described herein is in a range of from 20 to 1000 mg. In some embodiments, the amount of SNAC in a composition for administration as described herein is in a range of from 30 to 1000 mg. In some embodiments, the amount of SNAC in a composition for administration as described herein is in a range of from 50 to 1000 mg. In some embodiments, the amount of SNAC in a composition for administration as described herein is in a range of from 100 to 1000 mg. In some embodiments, the amount of SNAC in a composition for administration as described herein is in a range of from 200 to 1000 mg. In some embodiments, the amount of SNAC in a composition for administration as described herein is in a range of from 500 to 1000 mg.

In some embodiments of any one of the embodiments described herein, the amount of SNAC in a composition for administration as described herein is in a range of from 10 to 2000 mg. In some embodiments, the amount of SNAC in a composition for administration as described herein is in a range of from 20 to 2000 mg. In some embodiments, the amount of SNAC in a composition for administration as described herein is in a range of from 30 to 2000 mg. In some embodiments, the amount of SNAC in a composition for administration as described herein is in a range of from 50 to 2000 mg. In some embodiments, the amount of SNAC in a composition for administration as described herein is in a range of from 100 to 2000 mg. In some embodiments, the amount of SNAC in a composition for administration as described herein is in a range of from 200 to 2000 mg. In some embodiments, the amount of SNAC in a composition for administration as described herein is in a range of from 500 to 2000 mg. In some embodiments, the amount of SNAC in a composition for administration as described herein is in a range of from 1000 to 2000 mg.

In some embodiments of any one of the embodiments described herein, at least 50 weight percents of the composition consists of SNAC. In some embodiments, at least 60 weight percents of the composition consists of SNAC. In some embodiments, at least 70 weight percents of the composition consists of SNAC. In some embodiments, at least 80 weight percents of the composition consists of SNAC. In some embodiments, at least 90 weight percents of the composition consists of SNAC.

The following describes exemplary compositions suitable for use in the context of the present embodiments.

### Composition comprising hydrophobic substance:

In some embodiments according to any of the aspects of the embodiments described herein, the composition (optionally in a form of a composition unit dosage form, according to any of the respective embodiments described herein) comprises, in addition to PTH and SNAC and optionally a protease inhibitor (in accordance with any of the respective embodiments described herein), at least one hydrophobic substance.

In some such embodiments, the composition comprising a hydrophobic substance is an extended-release formulation (e.g., according to any of the respective embodiments described herein).

As exemplified in the Examples section herein, inclusion of a hydrophobic substance can lengthen the time until disintegration and/or dissolution of a composition, and may thereby slow the rate of release of the PTH and/or SNAC therein.

Examples of suitable hydrophobic substances which may be incorporated into a composition described herein include, without limitation, hydrophobic polymers and waxes.

Herein throughout, the phrase "hydrophobic polymer" refers to a polymer which is neither water-soluble (as defined herein) nor water-swellable.

In some embodiments of any of the embodiments described herein relating to a hydrophobic polymer, the hydrophobic polymer has a solubility of less than 0.3 gram per liter in water at a pH of 7. In some embodiments, the solubility is less than 0.1 gram per liter. In some embodiments, the solubility is less than 0.03 gram per liter. In some embodiments, the solubility is less than 0.01 gram per liter.

Herein, the term "water-swellable" refers to an ability of a solid substance to absorb at least 20 weight percents water (weight of water per weight of solid) upon contact with water at a pH of 7 at 37 °C.

In some embodiments of any of the embodiments described herein relating to a hydrophobic polymer, the hydrophobic polymer absorbs less than 10 weight percents water (weight of water per weight of solid) upon contact with water at a pH of 7. In some embodiments, the hydrophobic polymer absorbs less than 5 weight percents water. In some embodiments, the hydrophobic polymer absorbs less than 2 weight percents water. In some embodiments, the hydrophobic polymer absorbs less than 1 weight percents water.

Examples of hydrophobic polymers which may be used in some embodiments of any of the embodiments described herein relating to a hydrophobic polymer include, without limitation, ethyl cellulose and other hydrophobic cellulose ethers, polyvinyl acetate, polyethylene, poly(methyl methacrylate), poly(ethyl methacrylate), poly(methyl acrylate), poly(ethyl acrylate) and copolymers thereof (e.g., poly(ethylene-co-vinyl acetate), poly(ethyl acrylate-co-methyl methacrylate)). Ethyl cellulose is an exemplary hydrophobic polymer.

Herein, the term "wax" refers to a substance consisting primarily of (i.e., more than 50 weight percents) at least one hydrocarbon having at least 20 carbon atoms and/or at least one carboxylate ester, the substance being solid at 25 °C (e.g., the substance having a melting point at a temperature above 25 °C).

Examples of waxes suitable for use in embodiments of the invention include, without limitation, beeswax (also referred to in the art as "E901"); candelilla wax (also referred to in the art as "E902"); carnauba wax (also referred to in the art as "E903"); petroleum wax, including microcrystalline wax and paraffin wax (also referred to in the art as "E905c"); E907; rice bran wax (also referred to in the art as "E908"); spermaceti wax (also referred to in the art as "E909"); E910; methyl esters of fatty acids (also referred to in the art as "E911"); montanic acid esters (also referred to in the art as "E912"); lanolin (also referred to in the art as "E913"); oxidized polyethylene wax (also referred to in the art as "E914"); montan wax; and ouricury wax.

Herein, the term "carboxylate ester" refers to a compound having the formula R'-O-C(=O)-R", wherein R' and R" are each independently selected from the group consisting of alkyl, cycloalkyl, aryl, heteroalicyclic and heteroaryl (wherein heteroalicyclic and heteroaryl are bonded via a carbon atom), each being substituted or non-substituted.

In some embodiments of any of the embodiments described herein, R' and R" are each a hydrocarbon, for example, alkyl, cycloalkyl or aryl, each being substituted by a hydrocarbon moiety (e.g., non-substituted alkyl, cycloalkyl or aryl) or non-substituted.

In some embodiments of any of the embodiments described herein, R" comprises at least 3 carbon atoms (such that the carboxylate moiety comprises at least 4 carbon atoms). In some embodiments, R" comprises at least 5 carbon atoms. In some embodiments, R" comprises at least 7 carbon atoms. In some embodiments, R" comprises at least 9 carbon atoms. In some embodiments, R" comprises at least 11 carbon atoms. In some embodiments, R" comprises at least 13 carbon atoms. In some embodiments, R" comprises at least 15 carbon atoms. In some embodiments, R" comprises at least 17 carbon atoms. In some embodiments, R" comprises at least 19 carbon atoms.

In some embodiments of any of the embodiments described herein, R' comprises at least 4 carbon atoms (such that the alcohol moiety comprises at least 4 carbon atoms). In some embodiments, R' comprises at least 6 carbon atoms. In some embodiments, R' comprises at least 8 carbon atoms. In some embodiments, R' comprises at least 10 carbon atoms. In some embodiments, R' comprises at least 12 carbon atoms.

In some embodiments of any of the embodiments described herein, R' and R" each comprise at least 3 carbon atoms. In some embodiments, R' and R" each comprise at least 5 carbon atoms. In some embodiments, R' and R" each comprise at least 7 carbon atoms. In some embodiments, R' and R" each comprise at least 9 carbon atoms. In some embodiments, R' and R" each comprise at least 11 carbon atoms.

In some embodiments of any of the embodiments described herein, at least 50 weight percents of the wax consists of a carboxylate ester or a mixture of carboxylate esters (e.g., according to any of the embodiments described herein relating to a carboxylate ester). In some embodiments, at least 60 weight percents of the wax is carboxylate ester(s). In some embodiments, at least 70 weight percents of the wax is carboxylate ester(s). In some embodiments, at least 80 weight percents of the wax is carboxylate ester(s). In some embodiments, at least 90 weight percents of the wax is carboxylate ester(s). In some embodiments, the wax consists essentially of one or more carboxylate ester.

In some embodiments of any of the embodiments described herein, at least 50 weight percents of the wax consists of a hydrocarbon or a mixture of hydrocarbons having at least 20 carbon atoms (e.g., according to any of the respective embodiments described herein). In some embodiments, at least 60 weight percents of the wax is hydrocarbon(s). In some embodiments, at least 70 weight percents of the wax is hydrocarbon (s). In some embodiments, at least 80 weight percents of the wax is hydrocarbon(s). In some embodiments, at least 90 weight percents of the wax is hydrocarbon(s). In some embodiments, the wax consists essentially of one or more hydrocarbons having at least 20 carbon atoms.

Without being bound by any particular theory, it is believed that non-polar substances such as hydrophobic polymers and waxes described herein tend to be relatively inert due, for example, to a deficiency in capability to form non-covalent bonds such as hydrogen bonds and ionic bonds (this deficiency typically being associated with hydrophobicity).

In some embodiments of any of the embodiments described herein, the composition is formulated such that the hydrophobic substance delays release of PTH and SNAC, as compared with release from a similar composition without the hydrophobic substance.

In some embodiments of any of the embodiments described herein, the composition (e.g., unit dosage form) is formulated such that the hydrophobic substance is distributed substantially throughout the composition. In some embodiments, the hydrophobic substance is formulated as a matrix in which the PTH and/or SNAC is embedded and/or enclosed. In some embodiments, the hydrophobic substance is distributed homogeneously throughout the composition.

In some embodiments of any of the embodiments described herein, the composition is formulated as a tablet (e.g., using standard tableting methodology).

In some embodiments of any of the embodiments described herein, the composition (e.g., unit dosage form) comprises a mixture of the hydrophobic substance with the PTH and/or SNAC, and optionally with additional ingredients. In some embodiments, the mixture is substantially homogeneous. In some embodiments, the mixture is a substantially homogeneous mixture of the PTH, SNAC and hydrophobic substance according to any of the respective embodiments described herein, and any optional additional ingredient present (if any).

In some embodiments of any of the embodiments described herein, the mixture of the hydrophobic substance with the PTH and/or SNAC (and optionally with additional ingredients), according to any of the respective embodiments described herein, is at least 50 weight percents of the composition (e.g., unit dosage form). In some embodiments, the aforementioned mixture is at least 60 weight percents of the composition. In some embodiments, the aforementioned mixture is at least 70 weight percents of the composition. In some embodiments, the aforementioned mixture is at least 80 weight percents of the composition. In some embodiments, the aforementioned mixture is at least 90 weight percents of the composition. In some embodiments, the aforementioned mixture is at least 95 weight percents of the composition. In some embodiments, the aforementioned mixture is at least 98 weight percents of the composition. In some embodiments, the aforementioned mixture is substantially 100 weight percents of the composition.

In some embodiments of any of the embodiments described herein, at least 50 weight percents of the composition (e.g., unit dosage form) consists of the hydrophobic substance, SNAC and PTH. In some embodiments, at least 60 weight percents of the composition consists of the hydrophobic substance, SNAC and PTH. In some embodiments, at least 70 weight percents of the composition consists of the hydrophobic substance, SNAC and PTH. In some embodiments, at least 80 weight percents of the composition consists of the hydrophobic substance, SNAC and PTH.

In some embodiments, at least 90 weight percents of the composition consists of the hydrophobic substance, SNAC and PTH. In some embodiments, at least 95 weight percents of the composition consists of the hydrophobic substance, SNAC and PTH. In some embodiments, at least 98 weight percents of the composition consists of the hydrophobic substance, SNAC and PTH. In some embodiments, the composition consists essentially of the hydrophobic substance, SNAC and PTH.

In some embodiments of any of the embodiments described herein, a concentration of hydrophobic substance in the composition (e.g., unit dosage form) is in a range of from 5 to 95 weight percents. In some embodiments, a concentration of hydrophobic substance in the composition is in a range of from 10 to 90 weight percents. In some embodiments, a concentration of hydrophobic substance in the composition is in a range of from 15 to 85 weight percents. In some embodiments, a concentration of hydrophobic substance in the composition is in a range of from 20 to 80 weight percents. In some embodiments, a concentration of hydrophobic substance in the composition is in a range of from 25 to 70 weight percents.

In some embodiments of any of the embodiments described herein, a concentration of hydrophobic substance in the composition (e.g., unit dosage form) is in a range of from 5 to 50 weight percents. In some embodiments, a concentration of hydrophobic substance in the composition is in a range of from 10 to 50 weight percents. In some embodiments, a concentration of hydrophobic substance in the composition is in a range of from 15 to 50 weight percents. In some embodiments, a concentration of hydrophobic substance in the composition is in a range of from 20 to 50 weight percents. In some embodiments, a concentration of hydrophobic substance in the composition is in a range of from 20 to 40 weight percents.

In some embodiments of any of the embodiments described herein, a concentration of hydrophobic substance in the composition (e.g., unit dosage form) is in a range of from 50 to 95 weight percents. In some embodiments, a concentration of hydrophobic substance in the composition is in a range of from 50 to 90 weight percents. In some embodiments, a concentration of hydrophobic substance in the composition is in a range of from 50 to 80 weight percents. In some embodiments, a concentration of hydrophobic substance in the composition is in a range of from 55 to 70 weight percents.

In some embodiments of any of the embodiments described herein relating to a hydrophobic substance, the hydrophobic substance is characterized by a water-solubility at pH 6 which no more than 20 % of a water-solubility at pH 6 of each of the PTH and the SNAC. In some embodiments, the hydrophobic substance is characterized by a water-solubility at pH 6 which no more than 10 % of a water-solubility at pH 6 of each of the PTH and the SNAC. In some embodiments, the hydrophobic substance is characterized by a water-solubility at pH 6 which no more than 5 % of a water-solubility at pH 6 of each of the PTH and the SNAC. In some embodiments, the hydrophobic substance is characterized by a water-solubility at pH 6 which no more than 2 % of a water-solubility at pH 6 of each of the PTH and the SNAC. In some embodiments, the hydrophobic substance is characterized by a water-solubility at pH 6 which no more than 1 % of a water-solubility at pH 6 of each of the PTH and the SNAC. In some embodiments, the hydrophobic substance is characterized by a water-solubility at pH 6 which no more than 0.5 % of a water-solubility at pH 6 of each of the PTH and the SNAC. In some embodiments, the hydrophobic substance is characterized by a water-solubility at pH 6 which no more than 0.2 % of a water-solubility at pH 6 of each of the PTH and the SNAC. In some embodiments, the hydrophobic substance is characterized by a water-solubility at pH 6 which no more than 0.1 % of a water-solubility at pH 6 of each of the PTH and the SNAC.

Without being bound by any particular theory, a hydrophobic substance which is considerably less water-soluble (e.g., no more than 20 % as water-soluble, as described hereinabove) than the PTH and the SNAC under conditions characteristic of the intestines (e.g., a pH of about 6) will result in dissolution of the PTH and SNAC occurring primarily under conditions in which the hydrophobic substance remains largely or entirely intact, thereby limiting the rate of release of dissolved PTH and SNAC. It is further believed that the largely or entirely intact hydrophobic substance can limit release of dissolved PTH and SNAC by reducing the rate of release of dissolved PTH and SNAC (e.g., by impeding diffusion of dissolved PTH and SNAC) and/or by reducing the rate of dissolution of PTH and SNAC (e.g., by resulting in local concentrations of PTH and/or SNAC which are at or near saturation).

### Coated particles:

In some embodiments according to any of the aspects of the embodiments described herein, the composition comprising PTH and SNAC (in accordance with any of the respective embodiments described herein) forms a part of a drug delivery system comprising at least one solid particle. The solid particle comprises: a core comprising the composition which comprises PTH and SNAC; and a coating selected from the group consisting of a water-insoluble water-permeable coating and an enteric coating (as defined herein). In some embodiments, the coating covers the entire surface of the core. In some such embodiments, the drug delivery system comprising at least one solid particle is an extended-release formulation (e.g., according to any of the respective embodiments described herein).

Herein, a "drug delivery system" is encompassed by the phrase "unit dosage form", according to any of the respective embodiments described herein.

Herein, the phrase "water-insoluble water-permeable coating" refers to a coating comprising a water-insoluble substance, and which coating maintains integrity (thereby continuing to coat the core) upon contact with 1 liter of water at pH 7 at 37 °C (for at least 24 hours), yet does not prevent permeation of water through the coating (thereby allowing contact of the core with water, even when the core is entirely covered by the coating). Preferably, permeation of water through the coating further allows dissolved molecules of PTH and SNAC to exit the particle, even when the core is entirely covered by the coating.

It is to be appreciated that the water-insoluble water-permeable coating may optionally include a water-soluble fraction, provided that the water-soluble fraction does not cause the coating to lose integrity upon contact with water under the abovementioned conditions (e.g., wherein the water-soluble fraction is dispersed in a water-insoluble fraction).

In some embodiments of any of the embodiments described herein, the water-insoluble water-permeable coating comprises at least one water-insoluble compound. In some embodiments, the water-insoluble compound(s) for a continuous bulk of the water-insoluble water-permeable coating, which optionally comprises a water-soluble fraction dispersed therein.

In some embodiments of any of the embodiments described herein, the water-insoluble water-permeable coating comprises at least one hydrophobic substance according to any of the respective embodiments described herein, for example, at least one hydrophobic polymer (according to any of the embodiments described herein relating to a hydrophobic polymer) and/or at least one wax (according to any of the embodiments described herein relating to a wax).

In some embodiments of any of the embodiments described herein, the water-insoluble water-permeable coating comprises at least one water-soluble compound (as defined herein) in combination with at least one water-insoluble compound (as defined herein). In some such embodiments, the at least one water-insoluble compound is at least one hydrophobic polymer and/or wax (e.g., as described herein according to any of the respective embodiments).

Without being bound by any particular theory, it is believed that a water-soluble compound in a water-insoluble water-permeable coating can dissolve in an aqueous environment (e.g., in the gastrointestinal tract), thereby leaving voids in a water-insoluble substance remaining in the coating, and that such voids facilitate permeation of the coating by water.

It is further believed that a water-soluble compound can be effectively utilized provide water-permeability to a coating with little or no interaction with a compound in the core (e.g., SNAC) which may otherwise be incompatible with the water-soluble compound, as the aforementioned provision or water-permeability may be effected using relatively small quantities of the water-soluble compound. Furthermore, the water-soluble compound may be released relatively rapidly from the drug delivery device upon contact of the coating with water, thereby further limiting interaction between the water-soluble compound and the core contents.

In some embodiments of any of the embodiments described herein, at least 50 weight percents of the water-insoluble water-permeable coating consists of the combination of at least one water-soluble compound (as described herein in any of the respective embodiments) with at least one hydrophobic polymer (as described herein in any of the respective embodiments) and/or at least one wax (as described herein in any of the respective embodiments). In some embodiments, at least 60 weight percents of the coating consists of the water-soluble compound(s) with the hydrophobic polymer(s) and/or wax(es). In some embodiments, at least 70 weight percents of the coating consists of the water-soluble compound(s) with the hydrophobic polymer(s) and/or wax(es). In some embodiments, at least 80 weight percents of the coating consists of the water-soluble compound(s) with the hydrophobic polymer(s) and/or wax(es). In some embodiments, at least 90 weight percents of the coating consists of the water-soluble compound(s) with the hydrophobic polymer(s) and/or wax(es). In some embodiments, the coating consists essentially of the water-soluble compound(s) with the hydrophobic polymer(s) and/or wax(es).

In some embodiments of any of the embodiments described herein, the water-soluble compound is a water-soluble polymer. Examples of suitable water-soluble polymers include, without limitation, poly(ethylene glycol) and any enteric polymer (e.g., as described herein) which is water-soluble, as defined herein. It is to be appreciated that water-solubility is defined herein with respect to a pH of 7, at which pH enteric polymers are typically water-soluble.

In some embodiments of any of the embodiments described herein, less than 20 weight percents of the water-insoluble water-permeable coating consists of the water-soluble compound(s). In some embodiments, less than 10 weight percents of the coating consists of the water-soluble compound(s). In some embodiments, less than 5 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, at least 0.1 weight percent of the water-insoluble water-permeable coating consists of the water-soluble compound(s). In some embodiments, from 0.1 to 20 weight percents of the coating consists of the water-soluble compound(s). In some embodiments, from 0.1 to 10 weight percents of the coating consists of the water-soluble compound(s). In some embodiments, from 0.1 to 5 weight percents of the coating consists of the water-soluble compound(s). In some embodiments, from 0.1 to 2 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, at least 0.2 weight percent of the water-insoluble water-permeable coating consists of the water-soluble compound(s). In some embodiments, from 0.2 to 20 weight percents of the coating consists of the water-soluble compound(s). In some embodiments, from 0.2 to 10 weight percents of the coating consists of the water-soluble compound(s). In some embodiments, from 0.2 to 5 weight percents of the coating consists of the water-soluble compound(s). In some embodiments, from 0.2 to 2 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, at least 0.5 weight percent of the water-insoluble water-permeable coating consists of the water-soluble compound(s). In some embodiments, from 0.5 to 20 weight percents of the coating consists of the water-soluble compound(s). In some embodiments, from 0.5 to 10 weight percents of the coating consists of the water-soluble compound(s). In some embodiments, from 0.5 to 5 weight percents of the coating consists of the water-soluble compound(s). In some embodiments, from 0.5 to 2 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, at least 1 weight percent of the water-insoluble water-permeable coating consists of the water-soluble compound(s). In some embodiments, from 1 to 20 weight percents of the coating consists of the water-soluble compound(s). In some embodiments, from 1 to 10 weight percents of the coating consists of the water-soluble compound(s). In some embodiments, from 1 to 5 weight percents of the coating consists of the water-soluble compound(s). In some embodiments, from 1 to 2 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, at least 2 weight percents of the water-insoluble water-permeable coating consists of the water-soluble compound(s). In some embodiments, from 2 to 20 weight percents of the coating consists of the water-soluble compound(s). In some embodiments, from 2 to 10 weight percents of the coating consists of the water-soluble compound(s). In some embodiments, from 2 to 5 weight percents of the coating consists of the water-soluble compound(s). In some of the aforementioned embodiments, the balance of the coating consists essentially of hydrophobic polymer(s) and/or wax(es), according to any of the respective embodiments described herein.

In some embodiments of any of the embodiments described herein, the drug delivery system comprises only one solid particle as described herein. In some such embodiments, the drug delivery system consists of one solid particle as described herein.

In some embodiments of any of the embodiments described herein relating to a system comprising only one solid particle, the coating of the particle is a water-insoluble water-permeable coating according to any of the respective embodiments described herein.

In some embodiments of any of the embodiments described herein relating to a system comprising only one solid particle, the core is formulated as a tablet (e.g., according to any tablet-forming methodology known in the art), such that the drug delivery system is a coated tablet.

In some embodiments of any of the embodiments described herein, the drug delivery system is formulated such that the time to full dissolution of the PTH and SNAC in the solid particle(s) is at least 30 minutes at each of pH 2 and pH 6. In some embodiments, the time until full dissolution is at least 60 minutes (1 hour). In some embodiments, the time until full dissolution is at least 2 hours. In some embodiments, the time until full dissolution is at least 4 hours. In some embodiments, the time until full dissolution is at least 6 hours. In some embodiments, the time until full dissolution is at least 8 hours. In some embodiments, the time until full dissolution is at least 12 hours.

In some embodiments of any of the embodiments described herein relating to particles comprising an enteric coating, the enteric coating comprises an enteric polymer (as defined herein), for example, according to any of the respective embodiments described herein relating to an enteric polymer.

The location in the gastrointestinal tract at which dissolution of the enteric coating commences can be controlled according to the pH dependence of the enteric coating and/or enteric polymer, as described herein according to any of the respective embodiments.

When dissolution of an enteric polymer and/or enteric coating commences at any given pH and/or location in the gastrointestinal tract (e.g., as described herein), a significant amount of time may pass until the core is exposed and/or the coating is disintegrated and/or completely dissolved, as the dissolution of enteric polymer and/or enteric coating is not necessarily a very rapid process. The time until the core is exposed and/or the coating is disintegrated and/or completely dissolved may optionally be controlled, for example, in accordance with the thickness of the enteric coating, wherein thicker enteric coatings are associated with longer dissolution times.

In some embodiments of any one of the embodiments described herein, complete dissolution of the enteric coating is effected at least 10 minutes after being subjected to an aqueous solution at a pH in which the enteric coating is soluble (e.g., pH 5.5, 6.0, 6.5 or 7.0, as described herein). In some embodiments, complete dissolution of the enteric coating is effected at least 30 minutes after being subjected to such an aqueous solution. In some embodiments, complete dissolution of the enteric coating is effected at least 60 minutes after being subjected to such an aqueous solution. In some embodiments, complete dissolution of the enteric coating is effected at least 1200 minutes after being subjected to such an aqueous solution.

In some embodiments of any one of the embodiments described herein, the drug delivery system comprises a plurality of solid particles which are held together, for example, enclosed within a capsule and/or attached by a binding material. In some such embodiments, the capsule (e.g., gelatin capsule) and/or binding material is selected so as to degrade under conditions in the stomach (e.g., due to the low pH and/or susceptibility to enzymatic proteolysis in the stomach), thereby releasing the particles, and allowing the particles to travel individually through the gastrointestinal tract.

The solid particles may optionally be in any of various forms, including tablets, granules and microspheres. Coated tablets are generally a suitable form of solid coated particle in embodiments of a drug delivery system comprising a small number (e.g., 1-4) of solid particles, whereas coated microspheres are generally a suitable form of solid coated particle in embodiments of a drug delivery system comprising a large number (e.g., at least 10, 100, 1,000) of solid particles.

Tablets, microspheres and additional particle types can be prepared using known methodologies, for example, spheronization for preparing microspheres, and pressing to prepare tablets. In addition, numerous coating techniques will be known to the skilled person, including, without limitation, spray coating, dip coating.

In some embodiments of any of the embodiments described herein, the drug delivery system comprises a plurality of solid particles (solid particles as described herein according to any of the respective embodiments).

In some embodiments of any of the embodiments described herein, the drug delivery system comprises a plurality of populations of a solid particle (a solid particle as described herein according to any of the respective embodiments), wherein each of the populations is characterized by a different release profile of PTH and/or SNAC. In some such embodiments, the solid particles comprise an enteric coating, according to any of the respective embodiments described herein.

Without being bound by any particular theory, it is believed that oral administration, in a single drug delivery system, of populations with different release profiles can mimic the effect of administration of different doses of PTH (with SNAC).

Herein, the term "population" encompasses an individual solid particle as well as a plurality of solid particles, for example, at least 2 solid particles, at least 5 solid particles, at least 10 solid particles, at least 20 solid particles, at least 50 solid particles, at least 100 solid particles, at least 200 solid particles, at least 500 solid particles, at least 1,000 solid particles, or at least 10,000 solid particles. Thus, for example, a plurality of populations of a solid particle may optionally consist of two different solid particles, each solid particle representing a population. Alternatively, some or all of the populations may comprise a plurality of particles.

In some embodiments of any of the embodiments described herein relating to a population comprising more than one solid particle, the solid particles of an individual population are substantially similar, for example, being prepared by the same technique.

In some embodiments of any of the embodiments described herein, the drug delivery system comprises 2 populations of a solid particle.

In some embodiments of any of the embodiments described herein, the drug delivery system comprises at least 3 populations of a solid particle. In some embodiments, the drug delivery system comprises exactly 3 populations of a solid particle.

In some embodiments of any of the embodiments described herein, the drug delivery system comprises at least 4 populations of a solid particle. In some embodiments, the drug delivery system comprises exactly 4 populations of a solid particle.

In some embodiments of any of the embodiments described herein, at least one population of a solid particle comprises from 1 to 3 solid particles. In some embodiments, at least one population of a solid particle comprises 1 or 2 solid particles. In some embodiments, at least one population of a solid particle comprises 1 solid particle.

In some embodiments of any of the embodiments described herein, each population of a solid particle consists of from 1 to 3 solid particles. In some embodiments, each population of a solid particle consists of 1 or 2 solid particles. In some embodiments, each population of a solid particle consists of 1 solid particle.

In some embodiments of any of the embodiments described herein, at least one population of a solid particle comprises at least 4 solid particles. In some embodiments, at least one population of a solid particle comprises at least 10 solid particles. In some embodiments, at least one population of a solid particle comprises at least 30 solid particles. In some embodiments, at least one population of a solid particle comprises at least 100 solid particles.

In some embodiments of any of the embodiments described herein, each population of a solid particle comprises at least 4 solid particles. In some embodiments, each population of a solid particle comprises at least 10 solid particles.

In some embodiments, each population of a solid particle comprises at least 30 solid particles. In some embodiments, each population of a solid particle comprises at least 100 solid particles.

In some embodiments of any of the embodiments described herein, each of the populations of a solid particle is characterized by a different structure of solid particle.

It is important to appreciate that the solid particles in a population do not necessarily have identical structures, but rather, variables defining the structure (e.g., coating thickness, particle diameter and/or concentration of a component in the coating and/or core) may optionally fit a statistical distribution (including, without limitation, a normal distribution and/or a Poisson distribution), wherein a "structure" of solid particle which characterizes a population refers to a structure having the highest probability, that is, the mode (e.g., as defined by the mode of the variable(s)). Thus, for example, in the case of a first population in which the coating thickness of the particles is represented by a distribution with a mode of 0.1 mm and a standard deviation of 0.05 mm, and a second population in which the coating thickness is represented by a distribution with a mode of 0.5 mm and a standard deviation of 0.2 mm, the populations are characterized by different structures (a structure having a coating thickness of 0.1 mm and a structure having a coating thickness of 0.5 mm), even though the distributions of the populations may optionally overlap.

In some embodiments of any of the embodiments described herein, the structure of the solid particles in the drug delivery system (including the particles in all populations) is multimodal (i.e., having two or more peaks), and each mode is associated with a different population of solid particle.

In some embodiments of any of the embodiments described herein, the populations are characterized by a different coating. In some such embodiments, the populations are characterized by a different enteric coating. The coatings (e.g., enteric coatings) may optionally be different in any aspect, for example, composition of the coating (e.g., concentration and/or species of enteric polymer) and/or dimensions (e.g., thickness) of the coating.

In some embodiments, the populations are characterized by a different thickness of enteric coating. In some embodiments, the enteric coating in each population characterized by a different coating thickness is composed of the same ingredients (e.g., such that the enteric coatings of different populations differ only in their thickness).

Without being bound by any particular theory, it is believed that the properties of the coating in general, and the coating thickness in particular, are especially suitable for controlling the release profile of PTH (and SNAC) from particles, for example, by using relatively thin coatings in one population to obtain relatively rapid release from said population, and also by using relatively thick coatings in another population to obtain relatively slow release from said population.

In some embodiments of any of the embodiments described herein, the drug delivery system is formulated such that a rate of release of the PTH (i.e., an amount of PTH released per unit time) as a function of time at pH 6 is characterized by at least two peaks (i.e., rate of release as a function of time is a multimodal function). In such embodiments, each peak may optionally be considered as effectively representing a separate dose of PTH (with SNAC), the drug delivery system releasing at least two doses at different times.

In some embodiments of any of the embodiments described herein, the drug delivery system is formulated such that a rate of release of PTH (i.e., an amount of PTH released per unit time) as a function of time at pH 7 is characterized by at least two peaks (i.e., rate of release as a function of time is a multimodal function).

The rate of release at pH 6 and/or pH 7 is optionally determined by placing a drug delivery system in an aqueous solution (optionally 1 liter) at 37 °C with citrate (optionally 0.1 M) as buffer, and with gentle stirring (optionally according to the USP paddle method II (USP 23), at 50 rotations per minute).

In some embodiments, a multimodal rate of release under any given conditions (e.g., at pH 6 and/or pH 7) is obtained using populations characterized by different enteric coating thicknesses (e.g., according to any of the respective embodiments described herein), wherein the enteric coatings (e.g., according to any of the respective embodiments described herein) are soluble under such conditions (e.g., at pH 6 and/or pH 7). In such embodiments, the particles with the thicker coatings optionally take more time to dissolve than particles with thinner coatings, such that a population characterized by a relatively thin coating is associated with a relatively early peak in the rate of release, whereas a population characterized by a relatively thick coating is associated with a later peak in the rate of release.

Without being bound by any particular theory, it is believed that oral administration of a drug delivery system in which the release rate from the solid particles is multimodal is particularly suitable for mimicking the effect of release from solid particles in a drug delivery system mimics the effect of multiple oral administrations of PTH (and SNAC) at different times, while avoiding the inconveniences associated with multiple administration. For example, a bimodal release can mimic the effect of two oral administrations, wherein the first peak corresponds to a first oral administration, and a second (later) peak mimics the effect of a second (later) oral administration. Similarly, a trimodal (3 peak) release can mimic the effect of 3 oral administrations, and so forth.

It is further believed that the lower the level of release in a trough separating two peaks, relative to the level of release at the peaks, the more effectively oral administration of the drug delivery system mimics the effect of multiple oral administrations of PTH (with SNAC) at different times.

In some embodiments of any of the embodiments described herein relating to a drug delivery system comprising a plurality of populations of a solid particle, administration of the drug delivery system is considered as more than one administration of a composition comprising PTH and SNAC. For example, oral administration of a drug delivery system which mimics the effect of administration of two different doses of PTH (with SNAC), e.g., characterized by bimodal release, is optionally considered as two oral administrations as described herein; and oral administration of a drug delivery system which mimics the effect of administration of three different doses of PTH (with SNAC), e.g., characterized by trimodal release, is considered as three oral administrations as described herein. Such drug delivery systems may optionally facilitate administration of a composition at least 3 times per day (e.g. according to any of the respective embodiments described herein) in a more convenient manner.

In some embodiments of any of the embodiments described herein relating to a rate of release as a function of time characterized by at least two peaks, at least two of the peaks are separated by a trough which is less than 75 % of a level of the two peaks separated by the trough. In some embodiments, at least two of the peaks are separated by a trough which is less than 50 % of a level of the two peaks separated by the trough. In some embodiments, at least two of the peaks are separated by a trough which is less than 25 % of a level of the two peaks separated by the trough. In some embodiments, at least two of the peaks are separated by a trough which is less than 10 % of a level of the two peaks separated by the trough.

In some embodiments of any of the embodiments described herein relating to a rate of release as a function of time characterized by at least two peaks, at least two of the peaks are separated by at least 30 minutes. In some embodiments, the two peaks are separated by at least 60 minutes. In some embodiments, the two peaks are separated by at least 2 hours. In some embodiments, the two peaks are separated by at least 4 hours. In some embodiments, the two peaks are separated by at least 6 hours.

In some embodiments of any of the embodiments described herein relating to populations characterized by different enteric coatings, at least a portion of the different enteric coatings are characterized by different pH-dependent solubility profiles. In some embodiments, each of the populations in the drug delivery system is characterized by a different pH-dependent solubility profile than the other populations.

Herein, a "pH-dependent solubility profile" refers to the pH values at which a substance (e.g., an enteric coating) is water-soluble, as defined herein.

In some embodiments of any of the embodiments described herein relating to enteric coatings characterized by different pH-dependent solubility profiles, the enteric coatings are characterized by differences in the lowest pH value (in a range of from 5 to 8) at which each enteric coating is water-soluble (as defined herein). In some embodiments, the differences in the lowest pH value (in a range of from 5 to 8) at which each enteric coating is water-soluble is at least 0.2 pH unit (e.g., wherein an enteric coating is water-soluble at pH 6.0, and another enteric coating is water-insoluble at a pH of up to at least 6.2). In some embodiments, the differences in the lowest pH value at which each enteric coating is water-soluble is at least 0.5 pH unit (e.g., wherein an enteric coating is water-soluble at pH 6.0, and another enteric coating is water-insoluble at a pH of up to at least 6.5). In some embodiments, the differences in the lowest pH value at which each enteric coating is water-soluble is at least 1 pH unit (e.g., wherein an enteric coating is water-soluble at pH 5.5, and another enteric coating is water-insoluble at a pH of up to at least 6.5). In some embodiments, the differences in the lowest pH value at which each enteric coating is water-soluble is at least 1.5 pH unit (e.g., wherein an enteric coating is water-soluble at pH 5.5, and another enteric coating is water-insoluble at a pH of up to at least 7.0).

Without being bound by any particular theory, it is believed that populations which are characterized by dissolution profile differences in the lowest pH value (in a range of from 5 to 8) at which each enteric coating is water-soluble will release core contents (e.g., PTH and SNAC) at different times following oral administration, as an orally administered object is typically subjected, over the course of hours, to a gradually increasing the pH in the gastrointestinal tract, beginning with a highly acidic pH in the stomach, followed by a slightly acidic pH in the proximal portion of the intestines, and later by an approximately neutral pH in the distal portion of the intestines. It is further believed that for each population, release of the core contents will be effected primarily at the time and location the gradually increasing pH reaches the lowest pH value (in a range of from 5 to 8) at which each enteric coating is water-soluble.

In some embodiments of any of the embodiments described herein relating to populations characterized by different pH-dependent solubility profiles, the drug delivery system further comprises populations characterized by enteric coatings with different enteric coating thicknesses (e.g., according to any of the respective embodiments described herein).

In some embodiments of any of the embodiments described herein relating to populations characterized by different pH-dependent solubility profiles, the populations are characterized by enteric coatings with substantially the same enteric coating thicknesses (e.g., according to any of the respective embodiments described herein).

In some embodiments of any one of the embodiments described herein, the drug delivery system is formulated such that absorption of the PTH as a function of time (e.g., the pharmacokinetic profile) following oral administration is characterized by at least two peaks in a level of PTH in the blood.

In such embodiments, the time until each of the first two peaks may be defined as the first Tmax and second Tmax respectively.

In some embodiments of any one of the embodiments described herein, the first Tmax is no more than 30 minutes and the second Tmax is at least 60 minutes. In some embodiments, the second Tmax is at least 2 hours. In some embodiments, the second Tmax is at least 4 hours. In some embodiments, the second Tmax is at least 6 hours. In some embodiments, the second Tmax is at least 8 hours.

In some embodiments of any one of the embodiments described herein, the first Tmax is no more than 60 minutes and the second Tmax is at least 2 hours (120 minutes). In some embodiments, the second Tmax is at least 4 hours. In some embodiments, the second Tmax is at least 6 hours. In some embodiments, the second Tmax is at least 8 hours.

In some embodiments of any one of the embodiments described herein, the first Tmax is no more than 2 hours, and the second Tmax is at least 4 hours. In some embodiments, the second Tmax is at least 6 hours. In some embodiments, the second Tmax is at least 8 hours.

In some embodiments of any of the embodiments described herein relating to a pharmacokinetic profile characterized by at least two peaks, at least two of the peaks are separated by a trough which is less than 75 % of a level of the two peaks separated by the trough. In some embodiments, at least two of the peaks are separated by a trough which is less than 50 % of a level of the two peaks separated by the trough. In some embodiments, at least two of the peaks are separated by a trough which is less than 25 % of a level of the two peaks separated by the trough. In some embodiments, at least two of the peaks are separated by a trough which is less than 10 % of a level of the two peaks separated by the trough.

In some embodiments of any of the embodiments described herein relating to a pharmacokinetic profile characterized by at least two peaks, at least two of the peaks are separated by at least 30 minutes. In some embodiments, the two peaks are separated by at least 60 minutes. In some embodiments, the two peaks are separated by at least 2 hours. In some embodiments, the two peaks are separated by at least 4 hours. In some embodiments, the two peaks are separated by at least 6 hours.

The following describes exemplary compositions and/or methods or treatments employing same, which may provide the desired pharmacokinetic properties as described herein in any one of the respective embodiments and any combinations thereof.

### Protective agents:

In some embodiments according to any of the aspects of the embodiments described herein, the method or treatment further comprises oral administration of a protective agent.

In some embodiments according to any of the aspects of the embodiments described herein, the composition for oral administration of PTH further comprises a protective agent. In some embodiments, the composition is formulated as one or more unit dosage forms (e.g., according to any of the respective embodiments described herein). The unit dosage form(s) may be formulated in any form suitable for oral administration, including solid and/or liquid forms. In some embodiments, the unit dosage form is a solid unit dosage form. In some embodiments, the composition is formulated as a tablet.

Herein, the term "protective agent" refers to an agent capable of protecting the PTH and/or SNAC against enzymes and/or acid in the gastrointestinal tract. For example, a protease inhibitor can protect PTH from activity of a protease, and an antacid can protect SNAC (e.g., by reducing conversion of SNAC from a carboxylate salt to a carboxylic acid form) and/or PTH from stomach acid (e.g., acid-induced denaturation).

In some embodiments, of any of the embodiments described herein, the composition comprises at least one antacid compound (e.g., according to any of the respective embodiments described herein). In some such embodiments, the composition further comprises at least one protease inhibitor (e.g., according to any of the respective embodiments described herein).

Without being bound by any particular theory, it is believed that compositions comprising PTH and SNAC are significantly affected by inactivation of the SNAC upon contact with stomach acid, which converts SNAC from a soluble carboxylate salt to an insoluble carboxylic acid. The inactivation of SNAC reduces the absorption of the PTH, thereby potentially reducing the efficacy of the composition. In addition, protease inhibitors used to protect PTH from proteolysis may also be at least partially inactivated upon contact with stomach acid, which may further reduce the efficacy of such compositions. It is further believed that the ability of protease inhibitors to protect PTH against protease activity in the digestive system is limited, because much of the PTH is inactivated by proteases before the proteases are inhibited by the protease inhibitor.

In some embodiments, of any of the embodiments described herein, the composition is in a form of a homogeneous mixture, such that a protective agent (optionally an antacid compound) is uniformly dispersed among the SNAC and therapeutically active agent (and optionally any additional ingredient present).

The present inventors have further designed unit dosage forms so as to release a protective agent prior to release of the compound which the protective agent is intended to protect, for example, releasing an antacid for reducing acidity in a vicinity of an orally administered composition prior to exposure of SNAC and/or PTH to stomach acid, and/or releasing a protease inhibitor for inhibiting proteases prior to exposure of PTH to proteases.

In some embodiments, of any of the embodiments described herein, the composition is formulated as a unit dosage form comprising a core and an external layer. The core comprises PTH and SNAC; and the external layer comprises at least one protective agent.

In some of any of the embodiments described herein, the protective agent is a protease inhibitor, according to any of the respective embodiments described herein.

In some of any of the embodiments described herein, the protective agent is an antacid compound, according to any of the respective embodiments described herein.

In some of any of the embodiments described herein, the unit dosage form comprises at least one protective agent which is an antacid compound (according to any of the respective embodiments described herein) and at least one protective agent which is a protease inhibitor (according to any of the respective embodiments described herein).

Herein throughout, the term "antacid compound" refers to any pharmaceutically acceptable compound capable of neutralizing stomach acid (e.g., HCl in aqueous solution), preferably wherein one mole of antacid compound is capable of neutralizing at least 0.5 mole of HCl, and more preferably capable of neutralizing at least 1 mole of HCl. The PTH, SNAC and protease inhibitors described herein are excluded from the scope of the phrase "antacid compound", even though they may exhibit some ability to neutralize stomach acid, in some embodiments of the invention.

Examples of antacid compounds which may be used in any one of the embodiments described herein relating to one or more antacid compounds (in accordance with any of the aspects of embodiments of the invention described herein), include, without limitation, calcium carbonate, calcium gluconate, calcium citrate, sodium carbonate, sodium bicarbonate, sodium gluconate, sodium citrate, sodium hydroxide, potassium carbonate, potassium bicarbonate, potassium gluconate, potassium citrate, potassium hydroxide, magnesium carbonate, magnesium gluconate, magnesium citrate, magnesium hydroxide, magnesium oxide, aluminum carbonate, aluminum gluconate, aluminum citrate, and aluminum hydroxide.

The unit dosage form may have any shape suitable for orally administered pharmaceutical dosage forms, including, without limitation, any 3-dimensional shape having a substantially rectangular (including substantially square), substantially circular and/or substantially oval cross-section along at least one axis. For example, the unit dosage form may have a substantially box-like shape, having a substantially rectangular cross-section (optionally with rounded corners) along 3 axes; a substantially cylindrical shape, having substantially circular and/or substantially oval cross-section along one axis, and a substantially rectangular cross-section (optionally with rounded corners) along 2 axes; or a substantially spherical or ovoid shape, having a substantially circular and/or substantially oval cross-section along 3 axes.

In some of any one of the embodiments described herein, the external layer comprises one or more protease inhibitors and one or more antacid compounds. In some such embodiments, the external layer consists essentially of one or more protease inhibitors and one or more antacid compounds. Alternatively, in some such embodiments, the external layer comprises a combination of one or more excipients with the protease inhibitor(s) and antacid compound(s).

In some of any one of the embodiments described herein, the external layer comprises one or more protease inhibitors, and is devoid of antacid compounds. In some such embodiments, the external layer consists essentially of one or more protease inhibitors. Alternatively, in some such embodiments, the external layer comprises a combination of one or more excipients with the protease inhibitor(s).

In some of any one of the embodiments described herein, the external layer comprises one or more antacid compounds, and is devoid of protease inhibitors. In some such embodiments, the external layer consists essentially of one or more antacid compounds. Alternatively, in some such embodiments, the external layer comprises a combination of one or more excipients with the antacid compound(s).

Herein throughout, the phrase "devoid of" encompasses the presence of minute amounts of the indicated substance (for example, less than 0.1 weight percent, optionally less than 0.05 weight percent, optionally less than 0.02 weight percent, and optionally less than 0.01 weight percent) as well as the complete absence of the indicated substance.

In some of any one of the embodiments described herein, a concentration (as a weight percentage) of PTH in the external layer is lower than a concentration of PTH in the core. In some of any one of the embodiments described herein, a concentration (as a weight percentage) of PTH in the external layer is less than 50 % of a concentration of PTH in the core. In some embodiments, the concentration in the external layer is less than 20 % of the concentration in the core. In some embodiments, the concentration in the external layer is less than 10 % of the concentration in the core. In some embodiments, the concentration in the external layer is less than 5 % of the concentration in the core. In some embodiments, the concentration in the external layer is less than 2 % of the concentration in the core. In some embodiments, the concentration in the external layer is less than 1 % of the concentration in the core. In some embodiments, the external layer is devoid of PTH.

In some of any one of the embodiments described herein, a concentration (as a weight percentage) of SNAC in the external layer is lower than a concentration of SNAC in the core. In some of any one of the embodiments described herein, a concentration (as a weight percentage) of SNAC in the external layer is less than 50 % of a concentration of SNAC in the core. In some embodiments, the concentration in the external layer is less than 20 % of the concentration in the core. In some embodiments, the concentration in the external layer is less than 10 % of the concentration in the core. In some embodiments, the concentration in the external layer is less than 5 % of the concentration in the core. In some embodiments, the concentration in the external layer is less than 2 % of the concentration in the core. In some embodiments, the concentration in the external layer is less than 1 % of the concentration in the core. In some embodiments, the external layer is devoid of SNAC. In some embodiments, the external layer is devoid of PTH and devoid of SNAC.

In some of any one of the embodiments described herein, the external layer covers the whole surface of the core.

In some of any one of the embodiments described herein, the external layer does not cover the whole surface of the core. In some embodiments wherein the external layer does not cover the whole surface of the core, the external layer is separated into a plurality of unconnected layers (e.g., 2 layers, 3 layers, 4 layers, or more than 4 layers), each of the unconnected layers covering a different region of the surface of the core. In such embodiments, the phrase "external layer" refers collectively to all such unconnected layers. In some embodiments, the external layer is separated into two unconnected layers which cover opposite sides of the core. In alternative embodiments wherein the external layer does not cover the whole surface of the core, the external layer is in a form of a single continuous layer.

In some of any one of the embodiments described herein, the external layer covers at least 30 % of the surface area of the core. In some embodiments, the external layer covers at least 40 % of the surface area of the core. In some embodiments, the external layer covers at least 50 % of the surface area of the core. In some embodiments, the external layer covers at least 60 % of the surface area of the core. In some embodiments, the external layer covers at least 70 % of the surface area of the core. In some embodiments, the external layer covers at least 80 % of the surface area of the core. In some embodiments, the external layer covers at least 90 % of the surface area of the core.

In some embodiments of any one of the embodiments described herein, the protease inhibitor(s) and/or antacid compound(s) in the external layer are distributed homogeneously throughout the external layer.

In some embodiments of any one of the embodiments described herein, the protease inhibitor(s) and/or antacid compound(s) in the external layer are distributed inhomogeneously throughout the external layer.

In some such embodiments, the protease inhibitor(s) and/or antacid compound(s) are within particles (e.g., microspheres containing the protease inhibitor(s) and/or antacid compound(s)), and the external layer further comprises a material (e.g., a filler and/or binder) between the particles.

Alternatively or additionally, in some embodiments, the external layer comprises two or more layers (e.g., concentric layers), wherein each layer within the external layer has a different composition. For example, the external layer may optionally comprise a first layer which comprises one of the protease inhibitor(s) and/or antacid compound(s), a second layer which comprises another of the protease inhibitor(s) and/or antacid compound(s), and optionally one or more additional layers, each comprising different inhibitor(s) and/or antacid compound(s).

In some embodiments of any one of the embodiments described herein, the core further comprises one or more protease inhibitors and/or antacid compounds, in addition to PTH and SNAC.

In some embodiments of any one of the embodiments described herein, the core consists essentially of the PTH and SNAC or a combination of the PTH, SNAC and the protease inhibitor(s) and/or antacid compound(s).

In some embodiments of any one of the embodiments described herein, the core comprises a combination of one or more excipients with the PTH and SNAC (and optionally the protease inhibitor(s) and/or antacid compound(s)).

In some embodiments of any one of the embodiments described herein, the core comprises PTH, SNAC and one or more antacid compounds. In some embodiments, the core consists essentially of a combination of PTH, SNAC and the antacid compound(s). Alternatively, in some embodiments, the core comprises a combination of one or more excipients with PTH, SNAC and antacid compound(s).

In some embodiments of any one of the embodiments described herein, the core comprises PTH, SNAC and one or more protease inhibitors. In some embodiments, the core consists essentially of a combination of PTH, SNAC and the protease inhibitor(s). Alternatively, in some embodiments, the core comprises a combination of one or more excipients with PTH, SNAC and protease inhibitor(s).

In some embodiments of any one of the embodiments described herein, the PTH and/or SNAC in the core are distributed homogeneously throughout the core.

In some embodiments of any one of the embodiments described herein, the PTH and/or SNAC in the core are distributed inhomogeneously throughout the core.

In some such embodiments, the PTH and/or SNAC are within particles (e.g., microspheres containing the PTH and/or SNAC), and the core comprises a material (e.g., a filler and/or binder) between the particles.

Alternatively or additionally, in some embodiments, the core comprises an inner portion and an outer portion (e.g., configured concentrically), wherein each portion within the core has a different composition. For example, the core may optionally comprise an outer portion which comprises PTH and an inner portion which comprises SNAC, or *vice versa.*

In some of any one of the embodiments described herein, the unit dosage form further comprises a coating which coats the outer surface of the external layer described herein, and optionally also a region of a core surface which is not covered by an external layer (in embodiments wherein the external layer does not cover the whole core). In some embodiments, the coating is formed from material which dissolves in at least a portion of the gastrointestinal tract.

In some embodiments of any one of the embodiments described herein, the coating is an enteric coating (e.g., an enteric coating according to any of the respective embodiments described herein).

In some embodiments of any one of the embodiments described herein, the coating dissolves under conditions in the stomach (e.g., in an aqueous environment, optionally only when a low pH is present), thereby exposing the external layer. Such a coating is optionally adapted for altering an appearance of the unit dosage form (e.g., for aesthetic enhancement and/or labeling), to provide flavor and/or mask flavor, and/or to protect the external layer and/or core (e.g., from mechanical insult, air, light and/or liquids).

Dissolution of the unit dosage form in the gastrointestinal system initially comprises primarily dissolution of the external layer (optionally after dissolution of a coating, if present), thereby releasing the protease inhibitor(s) and/or antacid compound(s) in the external layer prior to release of PTH and SNAC from the core.

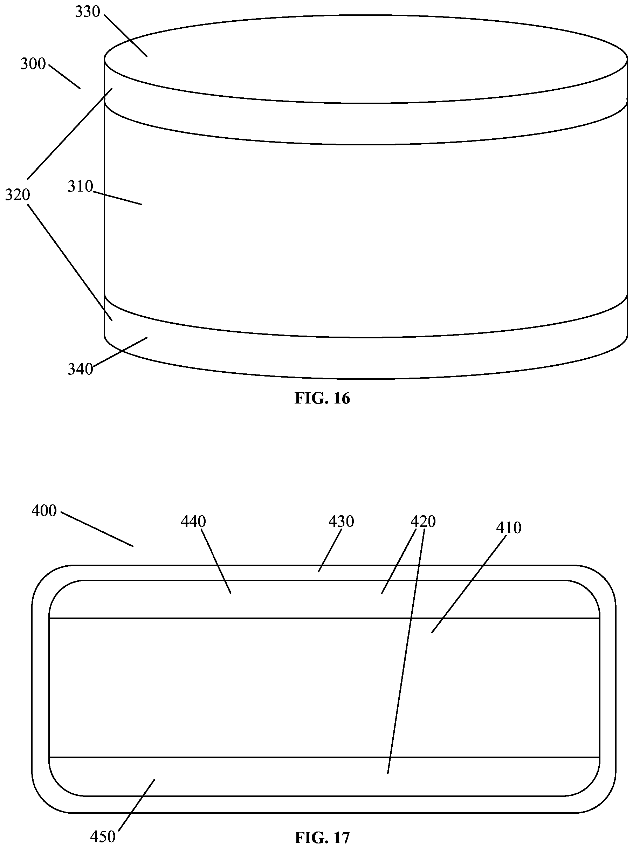

In some embodiments of any one of the embodiments described herein, the unit dosage form is formulated as a tablet. In some embodiments, the unit dosage form is formulated as a multi-layered tablet (e.g., a 3-layered tablet), in which the external layer forms an upper layer and a lower layer, and the core is formulated as a middle layer sandwiched between the upper layer and a lower layer. Exemplary tablets are shown in FIGs. 16 and 17 herein. Any of the multi-layered tablets described herein may optionally prepared according to any technique known in the art for preparing multi-layered tablets (e.g., 3-layered tablet), including, without limitation a technique described by Shende et al. [Int J Drug Delivery 2012, 4:418-426] .

In some embodiments of any one of the embodiments described herein, the unit dosage form consists primarily of the combination of PTH, SNAC, and at least one protective agent (protease inhibitor(s) and/or antacid compound(s)) described herein, that is, at least 50 weight percents of the unit dosage form consists of ingredients selected from the group consisting of PTH, SNAC and at least one protective agent. In some embodiments, at least 60 weight percents of the unit dosage form consists of PTH, SNAC and at least one protective agent. In some embodiments, at least 70 weight percents of the unit dosage form consists of PTH, SNAC and at least one protective agent. In some embodiments, at least 80 weight percents of the unit dosage form consists of PTH, SNAC and at least one protective agent. In some embodiments, at least 90 weight percents of the unit dosage form consists of PTH, SNAC and at least one protective agent. In some embodiments, at least 95 weight percents of the unit dosage form consists of PTH, SNAC and at least one protective agent. In some embodiments, at least 98 weight percents of the unit dosage form consists of PTH, SNAC and at least one protective agent. In some embodiments, the unit dosage form is formulated as a tablet.

In some embodiments of any one of the embodiments described herein, the external layer and core described herein consist primarily of the combination of PTH, SNAC, and at least one protective agent (protease inhibitor(s) and/or antacid compound(s)) described herein, that is, at least 50 weight percents of the total weight of the external layer and core consists of ingredients selected from the group consisting of PTH, SNAC and at least one protective agent. In some embodiments, at least 60 weight percents of the total weight of the external layer and core consists of PTH, SNAC and at least one protective agent. In some embodiments, at least 70 weight percents of the total weight of the external layer and core consists of PTH, SNAC and at least one protective agent. In some embodiments, at least 80 weight percents of the total weight of the external layer and core consists of PTH, SNAC and at least one protective agent. In some embodiments, at least 90 weight percents of the total weight of the external layer and core consists of PTH, SNAC and at least one protective agent. In some embodiments, at least 95 weight percents of the total weight of the external layer and core consists of PTH, SNAC and at least one protective agent.

In some embodiments, at least 98 weight percents of the total weight of the external layer and core consists of PTH, SNAC and at least one protective agent. In some embodiments, the external layer and core are formulated as parts of a tablet. In some embodiments, the tablet is a multi-layered tablet (e.g., 3-layered tablet).

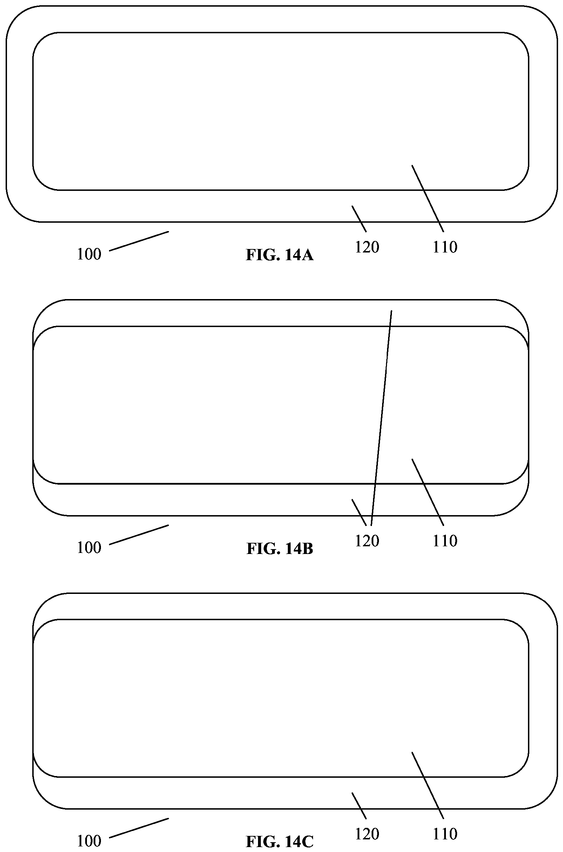

Referring now to the drawings, FIGs. 14A-14C show the structure, in cross-section, of an exemplary unit dosage form **100** according to some related embodiments of the invention. Unit dosage form **100** comprises a core **110** and an external layer **120.** The embodiments shown in FIGs. 14A-14C differ only in that FIG. 14A shows exemplary embodiments in which external layer **120** covers all of core **110;** FIG. 14B shows exemplary embodiments in which external layer **120** is separated into unconnected layers which cover different regions of core **110** (such that external layer **120** does not cover all of core **110**); and FIG. 14C shows exemplary embodiments in which external layer **120** is a single continuous layer which does not cover all of core **110.** Unit dosage form **100** is optionally substantially rectangular in cross-section (as depicted in FIGs. 14A-14C) along at least one axis. However, the cross-section may have a differently shape (e.g., substantially circular and/or substantially oval), and it is to be understood that the shapes depicted in FIGs. 14A-14C are not intended to be limiting.

External layer **120** comprises one or more protease inhibitors and/or antacid compounds, in accordance with any of one of the embodiments described herein relating to a composition of an external layer, and optionally consists essentially of one or more protease inhibitors and/or antacid compounds (e.g., in accordance with one of the respective embodiments described herein). Alternatively, external layer **120** comprises a combination of one or more excipients with the protease inhibitor(s) and/or antacid compound(s) (e.g., in accordance with one of the respective embodiments described herein).

In some embodiments, external layer **120** comprises one or more protease inhibitors (e.g., in accordance with one of the respective embodiments described herein), is optionally devoid of antacid compounds, and optionally consists essentially of one or more protease inhibitors (e.g., in accordance with one of the respective embodiments described herein). Alternatively, external layer **120** comprises a combination of one or more excipients with the protease inhibitor(s) (e.g., in accordance with one of the respective embodiments described herein).

In some embodiments, external layer **120** comprises one or more antacid compounds (e.g., in accordance with one of the respective embodiments described herein), is optionally devoid of protease inhibitors, and optionally consists essentially of one or more antacid compounds (e.g., in accordance with one of the respective embodiments described herein). Alternatively, external layer **120** comprises a combination of one or more excipients with the antacid compound(s) (e.g., in accordance with one of the respective embodiments described herein).

In some embodiments, a concentration (as a weight percentage) of PTH in external layer **120** is less than a concentration of PTH in core **110** (e.g., in accordance with one of the respective embodiments described herein). In some embodiments, external layer **120** is devoid of PTH.

In some embodiments, a concentration (as a weight percentage) of SNAC in external layer **120** is less than a concentration of SNAC in core **110** (e.g., in accordance with one of the respective embodiments described herein). In some embodiments, external layer **120** is devoid of SNAC. In some embodiments, external layer **120** is devoid of PTH and devoid of SNAC.

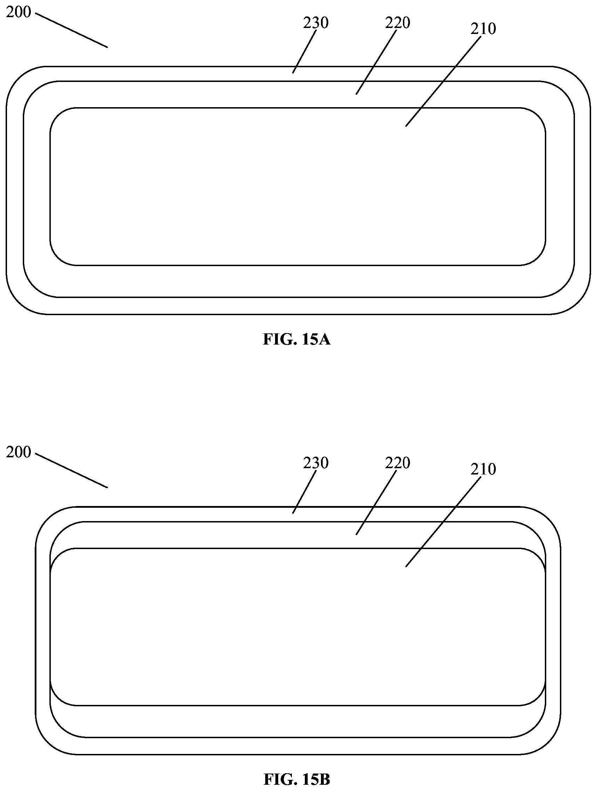

FIGs. 15A-15C show the structure, in cross-section, of an exemplary unit dosage form **200** according to some related embodiments of the invention. Unit dosage form **200** as shown in FIGs. 15A-15C, corresponds to unit dosage form **100** (in any one of the respective embodiments described herein) as shown, respectively, in FIGs. 14A-14C, differing from unit dosage form **100** in that unit dosage form **200** further comprises coating **230.** Unit dosage form **200** comprises a core **210** and an external layer **220,** which correspond, respectively, to core **110** and an external layer **120** of unit dosage form **100,** as described herein, in any one of the respective embodiments.

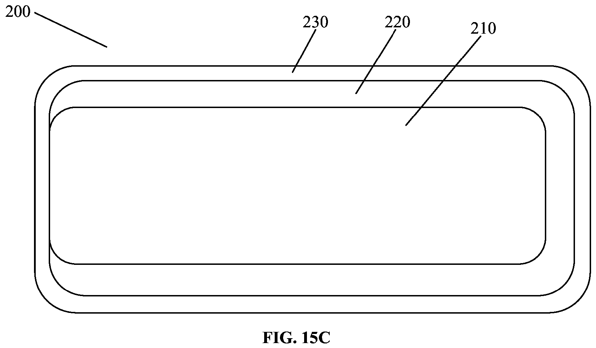

The embodiments shown in FIGs. 15A-15C differ only in that FIG. 15A shows exemplary embodiments in which external layer **220** covers all of core **210;** FIG. 15B shows exemplary embodiments in which external layer **220** is separated into unconnected layers which cover different regions of core **210** (such that external layer **220** does not cover all of core **210**); and FIG. 15C shows exemplary embodiments in which external layer **220** is a single continuous layer which does not cover all of core **210.**

Unit dosage form **200** is optionally substantially rectangular in cross-section (as depicted in FIGs. 15A-15C) along at least one axis. However, the cross-section may have a differently shape (e.g., substantially circular and/or substantially oval), and it is to be understood that the shapes depicted in FIGs. 15A-15C are not intended to be limiting.

Coating **230** has a composition in accordance with any one of the embodiments described herein relating to a coating, and is optionally formed from material which dissolves in at least a portion of the gastrointestinal tract (e.g., in accordance with one of the respective embodiments described herein).

In some embodiments of any one of the embodiments described herein, coating **230** is an enteric coating, as described herein (e.g., in accordance with one of the respective embodiments).

In some embodiments of any one of the embodiments described herein, coating **230** dissolves under conditions in the stomach (e.g., in accordance with one of the respective embodiments described herein), thereby exposing external layer **220.** Coating **230** is optionally adapted for altering an appearance of unit dosage form **200** (e.g., for aesthetic enhancement and/or labeling), to provide flavor and/or mask flavor, and/or to protect external layer **220** and/or core **210** (e.g., from mechanical insult, air, light and/or liquids), e.g., in accordance with one of the respective embodiments described herein).

In some embodiments of any one of the embodiments described herein, coating **230** is an enteric coating (e.g., in accordance with one of the respective embodiments described herein), external layer **220** comprises one or more protease inhibitors (e.g., in accordance with one of the respective embodiments described herein), and core **210** comprises PTH and SNAC, and optionally one or more protease inhibitors (e.g., in accordance with one of the respective embodiments described herein). In some such embodiments, unit dosage form **200** is formulated as a tablet (e.g., optionally as depicted in FIG. 17).

In embodiments wherein coating **230** is an enteric coating, dissolution of the unit dosage form **200** in the gastrointestinal system comprises dissolution of enteric coating **230** in the intestines, followed primarily by dissolution of external layer **220,** thereby releasing the protease inhibitor(s) in the external layer prior to release of PTH and SNAC from core **210.**

In some of any of the embodiments wherein coating **230** is an enteric coating, external layer **220** and/or core **210** is devoid of an antacid.

In some embodiments of any one of the embodiments described herein, coating **230** is a coating which dissolves under gastric conditions (e.g., in accordance with one of the respective embodiments described herein), external layer **220** comprises one or more antacid compounds (e.g., in accordance with one of the respective embodiments described herein), and core **210** comprises PTH and SNAC, and optionally one or more antacid compounds (e.g., in accordance with one of the respective embodiments described herein). In such embodiments, initial dissolution of the unit dosage form **200** in the gastro-intestinal tract primarily comprises dissolution of coating **230** and external layer **220** in the stomach, thereby releasing the antacid compound(s) in the external layer and reducing an acidity in the stomach (e.g., in a vicinity of the unit dosage form) prior to release of PTH and SNAC from core **210.**

In some embodiments of any one of the embodiments wherein coating **230** is a coating which dissolves under gastric conditions, external layer **220** and/or core **210** is devoid of a protease inhibitor.

In some embodiments of any one of the embodiments described herein, unit dosage form **200** is formulated as a coated tablet. In some embodiments, the unit dosage form **200** is formulated as a coated multi-layered tablet (e.g., 3-layered tablet), in which external layer **220** forms an upper layer and a lower layer, and core **210** is formulated as a middle layer sandwiched between the upper layer and a lower layer. An exemplary coated tablet is shown in FIG. 17. Any of the coated multi-layered tablets described herein may optionally prepared using any technique known in the art for preparing multi-layered tablets, followed by coating the tablet using any tablet-coating technique known in the art.

FIG. 16 shows the structure of an exemplary unit dosage form according to some embodiments of the invention, in a form of tablet **300.** Tablet **300** comprises a core **310** and an external layer **320,** which correspond, respectively, to core **110** and external layer **120** of unit dosage form **100,** as described herein in any one of the respective embodiments (e.g., with respect to FIG. 14B).

Tablet **300** is optionally has a substantially circular or substantially oval cross-section in cross-section (as depicted in FIG. 16). However, the tablet may have a differently shape, and it is to be understood that the shape depicted in FIG. 16 is not intended to be limiting.

External layer **320** includes layer **330** on an obverse face (e.g., a circular or oval face) and layer **340** on a reverse face (e.g., a circular or oval face) of tablet **300.** Layers **330** and **340** are optionally unconnected, such that external layer **320** is separated into two unconnected layers, corresponding to external layer **120** in FIG. 14B).

External layer **320** optionally covers at least 50 % of a surface area of core **310,** optionally at least 60 %, optionally at least 70 %, optionally at least 80 %, and optionally at least 90 % of the surface of core **310.**

External layer **320** comprises one or more protease inhibitors and/or antacid compounds, as described for external layer **120** according to any one of the respective embodiments described herein. In some embodiments, external layer **320** comprises one or more antacid compounds (e.g., in accordance with one of the respective embodiments described herein).

In some embodiments of any one of the embodiments described herein, external layer **320** is devoid of protease inhibitors. Optionally, external layer **320** consists essentially of one or more antacid compounds. Alternatively, external layer **320** comprises a combination of one or more excipients with the antacid compound(s) (e.g., in accordance with one of the respective embodiments described herein).

In some embodiments of any one of the embodiments described herein, external layer **320** comprises one or more protease inhibitors in addition to one or more antacid compounds (e.g., in accordance with one of the respective embodiments described herein). Optionally, external layer **320** consists essentially of one or more protease inhibitors and one or more antacid compounds. Alternatively, external layer **320** comprises a combination of one or more excipients with the protease inhibitor(s) and antacid compound(s) (e.g., in accordance with one of the respective embodiments described herein).

Core **310** comprises PTH of the tablet and SNAC, and optionally further comprises one or more protease inhibitors and/or antacid compounds, as described for core **110** according to any one of the respective embodiments described herein. In some embodiments, core **310** comprises one or more antacid compounds (e.g., in accordance with one of the respective embodiments described herein).

Initial dissolution of tablet **300** in the gastrointestinal system primarily comprises dissolution of external layer **320,** thereby releasing the protease inhibitor(s) and/or antacid compound(s) in the external layer prior to release of PTH and SNAC from core **310.**

FIG. 17 shows the structure, in cross-section, of an exemplary unit dosage form according to some embodiments of the invention, in a form of coated tablet **400.** Tablet **400** corresponds to tablet **300** (in any one of the respective embodiments described herein), differing in that tablet **400** further comprises enteric coating **430.** Tablet **400** comprises a core **410,** an external layer **420,** and an enteric coating **430,** which correspond, respectively, to core **210,** external layer **220** and coating **230** of unit dosage form **200,** as described herein, in any one of the respective embodiments (e.g., with respect to FIG. 15B).

Enteric coating **430** may optionally be an enteric coating according to any one of the embodiments described herein relating to an enteric coating, for example, with respect to coating **230** (e.g., in accordance with one of the respective embodiments).

Tablet **400** optionally has a substantially circular or substantially oval cross-section in cross-section (as depicted in FIG. 17). However, the tablet may have a differently shape, and it is to be understood that the shape depicted in FIG. 17 is not intended to be limiting.

External layer **420** includes layer **440** on an obverse face (e.g., a circular or oval face) and layer **450** on a reverse face (e.g., a circular or oval face) of tablet **400.** Layers **440** and **450** are optionally unconnected, such that external layer **420** is separated into two unconnected layers, corresponding to external layer **220** in FIG. 15B).

External layer **420** optionally covers at least 50 % of a surface area of core **410,** optionally at least 60 %, optionally at least 70 %, optionally at least 80 %, and optionally at least 90 % of the surface of core **410.**

External layer **420** comprises one or more protease inhibitors and/or antacid compounds, as described for external layer **120** and/or external layer **220** according to any one of the respective embodiments described herein. In some embodiments of any one of the embodiments described herein, external layer **420** is devoid of antacid compounds. Optionally, external layer **420** consists essentially of one or more protease inhibitors. Alternatively, external layer **420** comprises a combination of one or more excipients with the protease inhibitor(s).

Core **410** comprises PTH of the tablet and SNAC, and optionally further comprises one or more protease inhibitors and/or antacid compounds, as described for core **110** and/or core **210** according to any one of the respective embodiments described herein. In some embodiments, core **410** comprises one or more protease inhibitors. In some embodiments, core **410** is devoid of antacid compounds.

Dissolution of tablet **400** in the gastrointestinal system comprises dissolution of enteric coating **430** in the intestines, followed primarily by dissolution of external layer **420,** thereby releasing the protease inhibitor(s) in the external layer prior to release of PTH and SNAC from core **410.**

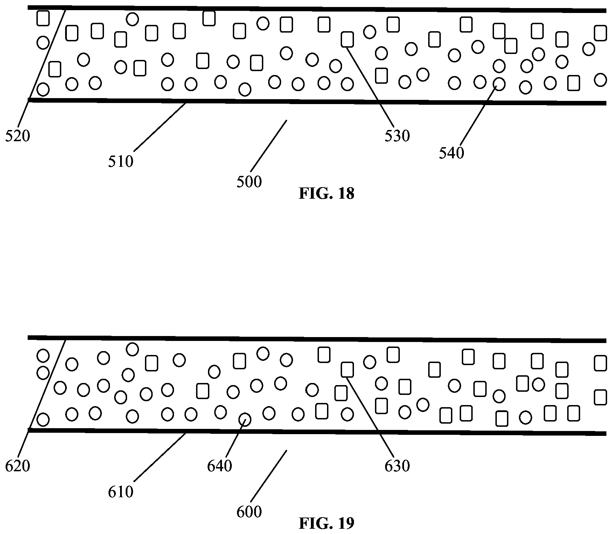

FIG. 18 shows a composition of an exemplary external layer **500** according to some of any one of the embodiments of the invention. External layer **500** corresponds to any external layer described herein (e.g., external layer **120, 220, 320** and/or **420),** in any one of the respective embodiments described herein, and has an inner face **510** which faces a core as described herein, and an outer face **520,** which faces a coating described herein and/or surface of a unit dosage form described herein. External layer **500** comprises a first compound **530** (optionally a single compound, and optionally a combination of compounds) depicted as rectangles, and a second compound **540** (optionally a single compound, and optionally a combination of compounds) depicted as circles. Additional compounds (not shown) may optionally also be comprised by external layer **500.**

The distribution of compounds **530** and **540** is optionally inhomogeneous, such that compound **530** is more concentrated in the vicinity of outer face **520** than in the vicinity of inner face **510,** and/or compound **540** is more concentrated in the vicinity of inner face **510** than in the vicinity of outer face **520,** as depicted in FIG. 18. Thus, a gradient in concentration exists between faces **510** and **520.** In some embodiments, dissolution of external layer **500** results in dissolution of compound **530** preceding dissolution of compound **540.**

Alternatively, the distribution of compounds **530** and **540** is homogeneous, such that no gradient in concentration exists between faces **510** and **520.**

In some of any of the embodiments described herein, compound **530** is one or more antacid compounds (e.g., in accordance with one of the respective embodiments described herein), and compound **540** is one or more protease inhibitor(s) and/or excipient(s) (e.g., in accordance with one of the respective embodiments described herein).

In some of any of the embodiments described herein, compound **530** is one or more protease inhibitors (e.g., in accordance with one of the respective embodiments described herein), and compound **540** is one or more antacid compound(s) and/or excipient(s) (e.g., in accordance with one of the respective embodiments described herein).

FIG. 19 shows a composition of an exemplary external layer **600** according to some of any one of the embodiments of the invention. External layer **600** corresponds to any external layer described herein (e.g., external layer **120, 220, 320, 420** and/or **520),** in any one of the respective embodiments described herein, and has an inner face **610** which faces a core as described herein, and an outer face **620,** which faces a coating described herein and/or surface of a unit dosage form described herein. External layer **600** comprises a first compound **630** (optionally a single compound, and optionally a combination of compounds) depicted as rectangles, and a second compound **640** (optionally a single compound, and optionally a combination of compounds) depicted as circles. Additional compounds (not shown) may optionally also be comprised by external layer **600.**

As depicted in FIG. 19, the distribution of compounds **630** and **640** is optionally inhomogeneous, such that compound **630** is more concentrated in the vicinity of one or more regions of the unit dosage form surface (e.g., the right-hand side of FIG. 19) than in the vicinity of other regions of the unit dosage form surface (e.g., the left-hand side of FIG. 19), and/or compound **540** is more concentrated in the vicinity of one or more regions of the unit dosage form surface (e.g., the left-hand side of FIG. 19) than in the vicinity of other regions of the unit dosage form surface (e.g., the right-hand side of FIG. 19). Thus, a gradient in concentration exists in the plane of external layer **600.**

Alternatively, the distribution of compounds **630** and **640** is homogeneous, such that no gradient in concentration exists in the plane of external layer **600.**

In some of any of the embodiments described herein, compound **630** is one or more antacid compounds (e.g., in accordance with one of the respective embodiments described herein), and compound **640** is one or more protease inhibitor(s) and/or excipient(s) (e.g., in accordance with one of the respective embodiments described herein).

In some of any of the embodiments described herein, compound **630** is one or more protease inhibitors (e.g., in accordance with one of the respective embodiments described herein), and compound **640** is one or more antacid compound(s) and/or excipient(s) (e.g., in accordance with one of the respective embodiments described herein).

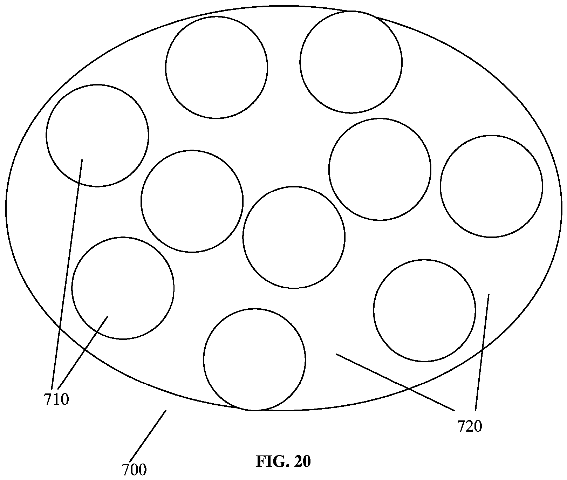

FIG. 20 shows a composition of an exemplary core **700** according to some of any one of the embodiments of the invention. Core **700** corresponds to any external layer described herein (e.g., core **110, 210, 310** and/or **410**), in any one of the respective embodiments described herein, and may optionally be combined with any external layer described herein.

As depicted in FIG. 20, the distribution of one or more compounds in core **700** is optionally inhomogeneous, such that the one or more compounds are concentrated within particles **710** separated at least in part by an interstitial material **720.**

Particles **710** optionally comprise PTH and/or SNAC (e.g., in accordance with one of the respective embodiments described herein), at a concentration which is higher than a concentration of PTH and/or SNAC in interstitial material **720.** Particles **710** may include different species of particles, having different compositions (e.g., one species comprising SNAC, and one species comprising PTH). Particles **710** are optionally in a form of granules and/or microspheres.

Interstitial material **720** is optionally devoid of PTH and/or SNAC. Interstitial material **720** optionally comprises on or more excipients (e.g., in accordance with one of the respective embodiments described herein), such as a filler and/or binder, and optionally consists essentially of one or more excipients.

Alternatively, the distribution of compounds in core **700** is homogeneous.

In some embodiments of any one of the embodiments described herein, at least 50 weight percents of a core described herein (e.g., any one of cores **110, 210, 310** and **410**) consists of SNAC. In some embodiments, at least 60 weight percents of a core described herein (e.g., any one of cores **110, 210, 310** and **410**) consists of SNAC. In some embodiments, at least 70 weight percents of a core described herein (e.g., any one of cores **110, 210, 310** and **410**) consists of SNAC. In some embodiments, at least 80 weight percents of a core described herein (e.g., any one of cores **110, 210, 310** and **410**) consists of SNAC. In some embodiments, at least 90 weight percents of a core described herein (e.g., any one of cores **110, 210, 310** and **410**) consists of SNAC.

Without being bound by any particular theory, it is believed that compositions (e.g., unit dosage forms and/or cores described herein) having a large proportion of SNAC, which is a salt, tend to be readily soluble in aqueous solution, including in gastric fluid, as is desirable according to some embodiments of the invention.

In some embodiments of any one of the embodiments described herein, the unit dosage form (e.g., any one of unit dosage form **100**, unit dosage form **200**, and tablet **300**) is soluble in gastric fluid (as defined herein). In some such embodiments, the unit dosage form does not comprise an enteric coating, thereby facilitating dissolution in gastric fluid. In some embodiments, the unit dosage form dissolves in gastric fluid in no more than 60 minutes. In some embodiments, the unit dosage form dissolves in gastric fluid in no more than 50 minutes. In some embodiments, the unit dosage form dissolves in gastric fluid in no more than 40 minutes. In some embodiments, the unit dosage form dissolves in gastric fluid in no more than 30 minutes. In some embodiments, the unit dosage form dissolves in gastric fluid in no more than 20 minutes. In some embodiments, the unit dosage form dissolves in gastric fluid in no more than 15 minutes. In some embodiments, the unit dosage form dissolves in gastric fluid in no more than 10 minutes. In some embodiments, the unit dosage form dissolves in gastric fluid in no more than 5 minutes.

In some embodiments of any one of the embodiments described herein, the unit dosage form (e.g., any one of unit dosage form **100,** unit dosage form **200,** and tablet **300**) is not soluble in gastric fluid.

In some embodiments of any one of the embodiments described herein, the unit dosage form is formulated such that absorption of the PTH following oral administration of the unit dosage form is characterized by a bioavailability of the PTH which is at least 10 % higher than a bioavailability of the PTH following oral administration of a unit dosage form composition consisting of the core of the aforementioned unit dosage form, without the external layer described herein. In some embodiments, the bioavailability is at least 20 % higher than (120 % of the level of) the bioavailability upon oral administration of the core. In some embodiments, the bioavailability is at least 50 % higher than (150 % of the level of) the bioavailability upon oral administration of the core. In some embodiments, the bioavailability is at least twice (200 % of the level of) the bioavailability upon oral administration of the core. In some embodiments, the bioavailability is at least four-fold (400 % of the level of) the bioavailability upon oral administration of the core. In some embodiments, the bioavailability is at least ten-fold (1000 % of the level of) the bioavailability upon oral administration of the core. In some embodiments, the bioavailability is at least twenty-fold (2000 % of the level of) the bioavailability upon oral administration of the core.

Without being bound by any particular theory, it is believed that the protective agent significantly enhances bioavailability by protecting SNAC and thereby increasing the amount of active SNAC which remains available for enhancing absorption of the PTH; and/or by protecting the PTH and thereby increasing the amount of PTH which remains active upon absorption.

In some embodiments of any one of the embodiments described herein relating to a composition comprising an antacid compound, the composition consists primarily of the combination of PTH, SNAC, and at least one antacid compound described herein, that is, at least 50 weight percents of the composition consists of ingredients selected from the group consisting of a PTH, SNAC and at least one antacid compound. In some embodiments, at least 60 weight percents of the composition consists of PTH, SNAC and at least one antacid compound. In some embodiments, at least 70 weight percents of the composition consists of PTH, SNAC and at least one antacid compound. In some embodiments, at least 80 weight percents of the composition consists of PTH, SNAC and at least one antacid compound. In some embodiments, at least 90 weight percents of the composition consists of PTH, SNAC and at least one antacid compound. In some embodiments, at least 95 weight percents of the composition consists of PTH, SNAC and at least one antacid compound. In some embodiments, at least 98 weight percents of the composition consists of PTH, SNAC and at least one antacid compound. In some embodiments, the composition is formulated as a tablet.

In some embodiments of any one of the embodiments described herein relating to a composition comprising an antacid compound, the composition optionally further comprises at least one protease inhibitor, and at least 50 weight percents of the composition consists of ingredients selected from the group consisting of PTH, SNAC, at least one antacid compound and at least one protease inhibitor. In some embodiments, at least 60 weight percents of the composition consists of PTH, SNAC, at least one antacid compound and at least one protease inhibitor. In some embodiments, at least 70 weight percents of the composition consists of PTH, SNAC, at least one antacid compound and at least one protease inhibitor. In some embodiments, at least 80 weight percents of the composition consists of PTH, SNAC, at least one antacid compound and at least one protease inhibitor. In some embodiments, at least 90 weight percents of the composition consists of PTH, SNAC, at least one antacid compound and at least one protease inhibitor. In some embodiments, at least 95 weight percents of the composition consists of PTH, SNAC, at least one antacid compound and at least one protease inhibitor. In some embodiments, at least 98 weight percents of the composition consists of PTH, SNAC, at least one antacid compound and at least one protease inhibitor. In some embodiments, the composition is formulated as a tablet.

In some embodiments of any one of the embodiments described herein relating to a composition comprising an antacid compound, the composition is formulated such that a bioavailability of the PTH upon oral administration of the composition is at least 10 % higher than a bioavailability of the PTH upon oral administration of a composition comprising the PTH and SNAC without the at least one antacid compound (e.g., being identical in all aspects except for the absence of the antacid compound(s)). In some embodiments, the bioavailability is at least 20 % higher than (120 % of the level of) the bioavailability upon oral administration of a composition comprising the PTH and SNAC without the at least one antacid compound. In some embodiments, the bioavailability is at least 50 % higher than (150 % of the level of) the bioavailability upon oral administration of a composition comprising the PTH and SNAC without the at least one antacid compound. In some embodiments, the bioavailability is at least twice (200 % of the level of) the bioavailability upon oral administration of a composition comprising the PTH and SNAC without the at least one antacid compound. In some embodiments, the bioavailability is at least four-fold (400 % of the level of) the bioavailability upon oral administration of a composition comprising the PTH and SNAC without the at least one antacid compound. In some embodiments, the bioavailability is at least ten-fold (1000 % of the level of) the bioavailability upon oral administration of a composition comprising the PTH and SNAC without the at least one antacid compound. In some embodiments, the bioavailability is at least twenty-fold (2000 % of the level of) the bioavailability upon oral administration of a composition comprising the PTH and SNAC without the at least one antacid compound.

An antacid may be utilized advantageously in combination with PTH and SNAC, without necessarily combining all of the ingredients in a single composition.

In some embodiments of any one of the embodiments described herein, the method or treatment according any of the respective embodiments described herein further comprises co-administering to the subject, an antacid composition comprising at least one antacid compound, as defined herein (e.g., at least one antacid compound described herein), and/or at least one gastric acid secretion inhibitor; and the composition comprising PTH and SNAC (e.g., as described herein according to any of the respective embodiments).

As used herein, the phrase "gastric acid secretion inhibitor" refers to any agent which reduces secretion of acid into the stomach, although it does not necessarily have any effect on acid which has already been secreted. Examples of gastric acid secretion inhibitors which may be used in any of the embodiments described herein relating to an antacid composition include, without limitation, H₂ receptor antagonists, such as cimetidine, famotidine, nizatidine and ranitidine; and proton pump inhibitors, such as omeprazole, lansoprazole, dexlansoprazole, esomeprazole, rabeprazole and ilaprazole.

In some embodiments of any one of the embodiments described herein relating to co-administering an antacid composition, the antacid composition is optionally any antacid composition known in the art (e.g., a commercially available antacid composition).

In some embodiments of any one of the embodiments described herein relating to co-administering an antacid composition, the co-administering comprises administering the antacid composition prior to or concomitantly with the composition comprising PTH and SNAC.

In some embodiments of any one of the embodiments described herein relating to co-administering an antacid composition concomitantly with the composition comprising PTH and SNAC, the antacid composition comprises at least one antacid compound, as defined herein (e.g., in accordance with any of the respective embodiments described herein).

In some embodiments of any one of the embodiments described herein relating to co-administering an antacid composition comprising at least one gastric acid secretion inhibitor, the co-administering comprises administering the antacid composition prior to the composition comprising PTH and SNAC (e.g., in accordance with any of the respective embodiments described herein).

Without being bound by any particular theory, it is believed that antacid compounds as defined herein (compounds capable of neutralizing stomach acid) are generally effective at reducing acidity in the stomach and/or in a region thereof immediately (as neutralization of acid occurs as a relatively rapid chemical reaction) but may have a limited long-term effect due to secretion of additional acid into the stomach, and are therefore particularly effective when administered concomitantly with or shortly (e.g., no more than 90 minutes) prior to the composition comprising PTH and SNAC.

It is further believed that gastric acid secretion inhibitors are generally effective at reducing stomach acidity for a relatively long duration (due to long-term inhibition of gastric acid secretion) but may have a limited effect on acidity immediately after administration due to an absence of a significant effect on acid which is already present in the stomach, and are therefore particularly effective when administered prior to the composition comprising PTH and SNAC.

Herein, the term "concomitantly" refers to an events (e.g., administration of an antacid composition) being within a time period of from 5 minutes before to 5 minutes after another event (e.g., administration of a composition comprising PTH and SNAC), and in some embodiments, within a time period of from one minute before to one minute after the other event.

In some embodiments, concomitant co-administration is effected by swallowing the two compositions simultaneously.

In some embodiments of any one of the embodiments described herein relating to co-administering at least one antacid composition, administering the antacid composition prior to the composition comprising PTH and SNAC comprises administering the antacid composition no more than 5 days prior to the composition comprising PTH and SNAC. In some embodiments, the antacid composition is administered no more than 4 days prior to the composition comprising PTH and SNAC. In some embodiments, the antacid composition is administered no more than 3 days prior to the composition comprising PTH and SNAC. In some embodiments, the antacid composition is administered no more than 2 days prior to the composition comprising PTH and SNAC. In some embodiments, the antacid composition is administered no more than 1 day (24 hours) prior to the composition comprising PTH and SNAC. In some embodiments, the antacid composition comprises a proton-pump inhibitor.

In some embodiments of any one of the embodiments described herein relating to co-administering at least one antacid composition, the antacid composition is administered at least about 1 day (e.g., at least about 24 hours) prior to the composition comprising PTH and SNAC, for example, from about 1 to about 5 days (e.g., about 2 days to about 4 days, optionally about 3 days) prior to the composition comprising PTH and SNAC. In some embodiments, the antacid composition comprises a proton-pump inhibitor.

In some of any of the embodiments described herein in which the antacid composition is optionally administered at least 12 hours prior to the composition comprising PTH and SNAC, the antacid composition comprises a proton-pump inhibitor.

In some embodiments of any one of the embodiments described herein relating to co-administering at least one antacid composition, administering the antacid composition prior to the composition comprising PTH and SNAC comprises administering the antacid composition no more than 16 hours prior to the composition comprising PTH and SNAC. In some embodiments, the antacid composition is administered no more than 12 hours prior to the composition comprising PTH and SNAC. In some embodiments, the antacid composition is administered no more than 10 hours prior to the composition comprising PTH and SNAC. In some embodiments, the antacid composition is administered no more than 8 hours prior to the composition comprising PTH and SNAC. In some embodiments, the antacid composition is administered no more than 6 hours prior to the composition comprising PTH and SNAC. In some embodiments, the antacid composition is administered no more than 4 hours prior to the composition comprising PTH and SNAC. In some embodiments, the antacid composition comprises an H₂ receptor antagonist.

In some embodiments of any one of the embodiments described herein relating to co-administering at least one antacid composition, the antacid composition is administered at least about 2 hours prior to the composition comprising PTH and SNAC, for example, from about 2 to about 10 hours (e.g., 2 to 8 hours, 2 to 6 hours, 2 to 4 hours) prior to the composition comprising PTH and SNAC. In some embodiments, the antacid composition comprises an H₂ receptor antagonist or a proton pump inhibitor. In some embodiments, the antacid composition comprises an H₂ receptor antagonist.

In some of any of the embodiments described herein in which the antacid composition is optionally administered at least 2 hours prior to, but less than 12 hours prior to, the composition comprising PTH and SNAC, the antacid composition comprises an H₂ receptor antagonist.

In some embodiments of any one of the embodiments described herein relating to co-administering at least one antacid composition, administering the antacid composition prior to the composition comprising PTH and SNAC comprises administering the antacid composition no more than 90 minutes prior to the composition comprising PTH and SNAC. In some embodiments, the antacid composition is administered no more than 60 minutes prior to the composition comprising PTH and SNAC. In some embodiments, the antacid composition is administered no more than 30 minutes prior to the composition comprising PTH and SNAC. In some embodiments, the antacid composition is administered no more than 20 minutes prior to the composition comprising PTH and SNAC. In some embodiments, the antacid composition is administered no more than 10 minutes prior to the composition comprising PTH and SNAC. In some embodiments, the antacid composition comprises an antacid compound (as defined herein).

In some embodiments of any one of the embodiments described herein relating to co-administering at least one antacid composition, the composition comprising PTH and SNAC is essentially the same as any one of the compositions described herein comprising PTH, SNAC and antacid compound(s), with the exception that no antacid compound is present.

In some embodiments, the composition comprising PTH and SNAC and/or the antacid composition further comprises at least one protease inhibitor (e.g., one or more protease inhibitors as described herein).

In some embodiments, the composition comprising PTH and SNAC and/or the antacid composition is formulated as a unit dosage form. The unit dosage form may be formulated in any form suitable for oral administration, including solid and/or liquid forms. In some embodiments, the unit dosage form (e.g., a unit dosage form of the composition comprising PTH and SNAC) is a solid unit dosage form. In some embodiments, the unit dosage form (e.g., a unit dosage form of the composition comprising PTH and SNAC) is formulated as a tablet.

In some embodiments, the composition comprising PTH and SNAC and the antacid composition (e.g., in solid form) are each soluble in gastric fluid (as defined herein). In some embodiments, the compositions each dissolve in gastric fluid in no more than 60 minutes. In some embodiments, the compositions each dissolve in gastric fluid in no more than 50 minutes. In some embodiments, the compositions each dissolve in gastric fluid in no more than 40 minutes. In some embodiments, the compositions each dissolve in gastric fluid in no more than 30 minutes. In some embodiments, the compositions each dissolve in gastric fluid in no more than 20 minutes. In some embodiments, the compositions each dissolve in gastric fluid in no more than 15 minutes. In some embodiments, the compositions each dissolve in gastric fluid in no more than 10 minutes. In some embodiments, the compositions each dissolve in gastric fluid in no more than 5 minutes.

In some embodiments, neither the composition comprising the therapeutically active agent and SNAC nor the antacid composition (e.g., in solid form) are soluble in gastric fluid (as defined herein).

In some embodiments of any one of the embodiments described herein relating to co-administering at least one antacid composition, absorption of PTH following the co-administration is characterized by a bioavailability of the PTH which is at least 10 % higher than a bioavailability of the PTH following oral administration of the composition comprising the PTH and SNAC without co-administering the antacid composition. In some embodiments, the bioavailability is at least 20 % higher than (120 % of the level of) the bioavailability without co-administering the antacid composition. In some embodiments, the bioavailability is at least 50 % higher than (150 % of the level of) the bioavailability without co-administering the antacid composition. In some embodiments, the bioavailability is at least twice (200 % of the level of) the bioavailability without co-administering the antacid composition. In some embodiments, the bioavailability is at least four-fold (400 % of the level of) the bioavailability without co-administering the antacid composition. In some embodiments, the bioavailability is at least ten-fold (1000 % of the level of) the bioavailability without co-administering the antacid composition. In some embodiments, the bioavailability is at least twenty-fold (2000 % of the level of) the bioavailability without co-administering the antacid composition.

Any one or more of the antacid compounds described herein may be used in any one of the embodiments described herein which utilize an antacid compound.

In some embodiments, the at least one antacid compound is selected from the group consisting of calcium carbonate, calcium gluconate, calcium citrate, sodium carbonate, sodium bicarbonate, sodium gluconate, sodium citrate, sodium hydroxide, potassium carbonate, potassium bicarbonate, potassium gluconate, potassium citrate, potassium hydroxide, magnesium carbonate, magnesium gluconate, magnesium citrate, magnesium oxide and magnesium hydroxide.

In some embodiments, the at least one antacid compound is selected from the group consisting of calcium carbonate, calcium gluconate, sodium carbonate, sodium bicarbonate, sodium citrate, sodium hydroxide, potassium carbonate, potassium bicarbonate, potassium citrate, potassium hydroxide, magnesium carbonate, magnesium hydroxide, magnesium oxide, aluminum carbonate, and aluminum hydroxide.

In some embodiments, the at least one antacid compound the at least one antacid compound is selected from the group consisting of calcium carbonate, calcium citrate, sodium bicarbonate, sodium hydroxide, magnesium carbonate, magnesium citrate, magnesium hydroxide, aluminum carbonate, and aluminum hydroxide.

In some embodiments, the at least one antacid compound is selected from the group consisting of calcium carbonate, sodium carbonate, sodium bicarbonate, potassium bicarbonate, magnesium carbonate, magnesium hydroxide, magnesium oxide and aluminum hydroxide.

In some embodiments of any one of the embodiments described herein relating to an antacid compound, a total amount of antacid compound(s) administered according to any of the respective embodiments of a method or treatment described herein (e.g., in a core of a unit dosage form described herein and/or in a unit dosage form described herein), is such that the at least one antacid compound comprises at least 0.00001 molar equivalent of base. In some embodiments, the at least one antacid compound comprises at least 0.00003 molar equivalent of base. In some embodiments, the at least one antacid compound comprises at least 0.0001 molar equivalent of base. In some embodiments, the at least one antacid compound comprises at least 0.0003 molar equivalent of base. In some embodiments, the at least one antacid compound comprises at least 0.001 molar equivalent of base. In some embodiments, the at least one antacid compound comprises at least 0.002 molar equivalent of base. In some embodiments, the at least one antacid compound comprises at least 0.003 molar equivalent of base.

In some embodiments, the at least one antacid compound comprises at least 0.005 molar equivalent of base. In some embodiments, the at least one antacid compound comprises at least 0.01 molar equivalent of base. In some embodiments, the at least one antacid compound comprises no more than 0.03 molar equivalent of base.

Herein, 1 molar equivalent of base refers to an amount of a basic compound (e.g., an antacid compound described herein) capable of neutralizing 1 mole of HCl (e.g., in an aqueous solution). In determining molar equivalents of base in antacid compounds described herein, each mole of hydroxide ion and/or bicarbonate ion is considered to be capable of neutralizing 1 mole of HCl, each mole of carbonate ion is considered to be capable of neutralizing 2 moles of HCl, and each mole of citrate ion (if fully deprotonated) is considered to be capable of neutralizing 3 moles of HCl.

In some embodiments of any one of the embodiments described herein relating to an antacid compound, a total amount of antacid compound(s) administered according to any of the respective embodiments of a method or treatment described herein (e.g., in a core of a unit dosage form described herein and/or in a unit dosage form described herein), is at least 0.5 mg. In some embodiments, the amount of antacid compound(s) is at least 1 mg. In some embodiments, the amount of antacid compound(s) is at least 2 mg. In some embodiments, the amount of antacid compound(s) is at least 5 mg. In some embodiments, the amount of antacid compound(s) is at least 10 mg. In some embodiments, the amount of antacid compound(s) is at least 25 mg. In some embodiments, the amount of antacid compound(s) is at least 50 mg. In some embodiments, the amount of antacid compound(s) is at least 100 mg. In some embodiments, the amount of antacid compound(s) is at least 200 mg. In some embodiments, the amount of antacid compound(s) is at least 300 mg. In some embodiments, the amount of antacid compound(s) is at least 400 mg. In some embodiments, the amount of antacid compound(s) is at least 500 mg. In some embodiments, the amount of antacid compound(s) is at least 750 mg. In some embodiments, the amount of antacid compound(s) is at least 1 gram.

### Casing:

In some embodiments of any one of the embodiments described herein, the composition comprising PTH and SNAC (according to any of the respective embodiments described herein) forms a part of a drug delivery system comprising a casing and the composition comprising PTH and SNAC contained within the casing.

The casing according to such embodiments of the invention comprises at least two components being in conjunction, and at least one retainer for maintaining the conjunction of the aforementioned components under gastric conditions, the casing being configured such that upon removal of the retainer, the casing is breached.

Herein, the term "casing" refers to a structure which encloses an inner volume and separates the inner volume from a surrounding environment. The term "casing" encompasses structures of any shape, including deformable structures which readily change shape (e.g., casings formed from a soft substance, for example, in the form of a soft pouch), as well as rigid structures, provided that the ability to separates the inner volume from a surrounding environment is maintained.

Herein, the term "retainer" refers to a component of the casing which is characterized by a structure and chemical composition suitable for performing the abovementioned function of maintaining the conjunction of the aforementioned components under gastric conditions. The casing may optionally comprise one retainer or a plurality of retainers (e.g., 2 retainers, 3 retainers, 4 retainers, more than 4 retainers). By maintaining conjunction of the aforementioned components under gastric conditions, the retainer optionally provides control over release of the PTH in the composition by, *inter **alia,*** preventing release in the stomach, while allowing release in the intestines.

For simplicity, a retainer is described herein throughout in the singular (e.g., "a retainer", "the retainer"). Use of the singular should not be interpreted as meaning that only one retainer is present and/or that only one retainer is referred to by any given description, unless this is explicitly indicated. Rather, in the absence of any indication to the contrary, a description of a retainer (in the singular) according to any one of the embodiments described herein is to be interpreted as referring to any one or more retainer(s) in a casing, and optionally to each retainer in the casing (in embodiments wherein the casing has more than one retainer).

Herein, the abovementioned at least two components being in conjunction are also referred to herein, for the sake of brevity, as the "casing components". This use of the phrase "casing components" is not intended to suggest in any way that the casing does not comprise other components. For example, the casing comprises at least one component, e.g., the retainer described herein, which is not referred to herein as a "casing component".

Herein, the phrase "conjunction" refers to a state wherein two or more objects meet in a form of physical contact, overlapping and/or close proximity.

The components in conjunction may optionally be different portions of a single component, wherein the different portions can be separated from one another upon removal of a retainer. For example, in some embodiments, the components in conjunction form different edges of a flexible component which is folded upon itself, such that breaching of the casing (e.g., upon removal of a retainer) can be effected by unfolding of the flexible component, to thereby separate the edges.

In some embodiments, a conjunction is such that casing components in conjunction are linked to one another.

In some embodiments, the casing components in conjunction form a capsule shell. The casing components may optionally be formed from any substance known in the art to be suitable for forming a capsule shell for pharmaceutical use. In some embodiments, the casing comprises two casing components which fit together to form a capsule shell, for example, wherein each casing component is one half of a capsule shell. In some embodiments, casing comprises more than two casing components (e.g., 3 or 4 casing components) which fit together to form a capsule shell.

Examples of mechanisms by which a conjunction may optionally be maintained by one or more retainers include, without limitation, adhering to casing components, optionally to each of the casing components (e.g., such that casing components are linked by adhesion to the same retainer); blocking movement of casing components, optionally blocking movement of each of the casing components (e.g., wherein casing components are not necessarily linked, but are physically restrained from separating from one another); and clamping casing components.

Herein, the term "clamping" refers to an act of holding objects together by applying a pressure which presses an object against another object.

Clamping may maintain casing components in conjunction, for example, by opposing movement of a casing component in a direction perpendicular to a surface of another casing component (e.g., by pressing a casing component in the opposite direction, namely, towards the surface), and/or by opposing movement (e.g., sliding) of a casing component in a direction parallel to a surface of another casing component (e.g., by increasing friction between the two components).

In some of any of the embodiments described herein, a retainer is an adhesive (e.g., a glue) which maintains at least two casing components in conjunction (e.g., in the form of a capsule shell) by adhering to the casing components. Optionally the adhesive is present at an interface between the casing components. Alternatively or additionally, the adhesive adheres to the casing components at a location other than an interface between the casing components, for example, at an external and/or internal surface of the casing (e.g., a capsule shell surface).

Herein, the terms "breach" and "breached", and variations thereof, refer to formation of an opening in the casing which connects the inner volume of the casing to a surrounding environment, and which is sufficiently large to allow escape of the PTH contained within the casing.

In some embodiments, breaching of the casing upon removal of the retainer(s) of the casing is such that a breach formed in the casing has an area which is at least 10 % of an area of the surface of the casing prior to breaching of the casing. In some embodiments, a breach formed in the casing has an area which is at least 20 % of an area of the surface of the casing prior to breaching of the casing. In some embodiments, a breach formed in the casing has an area which is at least 30 % of an area of the surface of the casing prior to breaching of the casing. In some embodiments, a breach formed in the casing has an area which is at least 40 % of an area of the surface of the casing prior to breaching of the casing. In some embodiments, a breach formed in the casing has an area which is at least 50 % of an area of the surface of the casing prior to breaching of the casing.

Herein, "removal" of a retainer refers to a hypothetical situation in which the casing is altered only in that the retainer is absent. Although such a situation is hypothetical, rather than a real-world physical process, it is useful for describing the structure of the casing herein according to many embodiments of the invention.

It is to be appreciated that a retainer may be partially degraded or otherwise altered without being removed, in a manner which results in breaching of the casing, optionally essentially the same extent of breaching as would occur upon removal of the retainer. When such cases are described herein, the partial degradation and/or alteration of the retainer is not referred to as "removal" of the retainer.

In some of any of the embodiments described herein, breaching of the casing upon removal of a retainer is effected by separation of casing components.

In some of any of the embodiments described herein, a retainer forms an integral part of the casing, such that absence of the retainer *per se* (i.e., without any additional change in the casing, such as movement of the casing components) forms a breach in the casing. In some embodiment, further breaching of the casing is effected by separation of casing components.

In some of any of the embodiments described herein, absence of a retainer *per se* (i.e., without any additional change in the casing, such as movement of the casing components) does not form a breach in the casing, but rather, removal of the retainer effects breaching of the casing, for example, by allowing separation of casing components.

In some of any of the embodiments described herein, absence of a retainer *per se* (i.e., without any additional change in the casing, such as movement of the casing components) does not form a breach in the casing which is at least 30 % of an area of the surface of the casing prior to breaching of the casing (e.g., no breach is formed, or a breach is formed which is less than 30 % of the area of the casing surface). In some embodiments, absence of a retainer *per se* does not form a breach in the casing which is at least 20 % of an area of the surface of the casing prior to breaching of the casing.

In some embodiments, absence of a retainer *per se* does not form a breach in the casing which is at least 10 % of an area of the surface of the casing prior to breaching of the casing. In some embodiments, absence of a retainer *per se* does not form a breach in the casing which is at least 5 % of an area of the surface of the casing prior to breaching of the casing. In some embodiments, absence of a retainer *per se* does not form a breach in the casing which is at least 2 % of an area of the surface of the casing prior to breaching of the casing.

Optionally, each of the casing components is in conjunction with each of the other casing component(s) in the casing.

Alternatively, the casing components may be divided into a plurality of sets (e.g., 2 sets, 3 sets, 4 sets, or more than 4 sets), wherein each of the casing components in each set is in conjunction with each of the other casing component(s) in the same set, but not necessarily in conjunction with casing components of other sets. Any one casing component may belong to one such set or to more than one such set. For example, if a casing comprises 3 casing components - components I, II and III - and component II is in conjunction with components I and III, whereas components I and III are not in conjunction with one another, the casing can be considered as having two sets of casing components, one set consisting of components I and II, and the other set consisting of components II and III.

Optionally, a retainer (optionally each retainer in a casing) maintains one set of casing components in conjunction (wherein each of the casing components is in conjunction with each of the other casing components in the set).

Alternatively, any one retainer may optionally maintain two or more sets of casing components (as described herein) in conjunction.

Optionally, any one set of casing components in conjunction is maintained in conjunction by a single retainer.

Alternatively, two or more retainers may optionally act together to maintain the same set of casing components in conjunction.

In some embodiments wherein two or more retainers maintain the same set of casing components in conjunction, the casing is configured such that the casing is breached upon removal of any one of the aforementioned retainers.

In some embodiments wherein two or more retainers maintain the same set of casing components in conjunction, the casing is configured such that the casing is not breached upon removal of one retainer, but rather, breached only upon removal of two or more of the aforementioned retainers, and optionally only upon removal of each of the aforementioned retainers.

In some embodiments of any one of the embodiments described herein, the drug delivery system is configured such that an internal force induces separation of the casing components upon removal of the retainer.

Herein, the term "internal force" refers to a force applied by one or more components of the drug delivery system, including the casing and/or a substance within the casing. For example, a component under tension and/or compression may apply a force upon neighboring components or substances, and the drug delivery system may optionally be configured such that such forces induce separation of the casing components.

Herein, to "induce" separation of casing components means that the force will tend to cause separation of casing components upon removal of the retainer.

Optionally, the retainer is configured so as to oppose the force inducing separation (until it is removed or otherwise ceases to oppose the force).

An internal force may optionally be present in the drug delivery system at any time (e.g., any time from the manufacturing of the drug delivery system onwards) or alternatively, the internal force is present only under certain conditions (e.g., exposure to an aqueous liquid, such as in the gastrointestinal tract). An internal force may optionally be applied by at least a part of the casing and/or a substance contained within the casing.

In some embodiments of any one of the embodiments described herein, the drug delivery system is not configured such that an internal force induces separation of the casing components upon removal of the retainer. Rather, separation of the casing components is induced, for example, by random movement, fluid flow in the intestines, peristalsis, and/or other external forces.

In some embodiments of any one of the embodiments described herein, the retainer forms a portion of an external surface of the casing, such that at least a portion of the retainer is exposed to the environment surrounding the casing.

In some embodiments of any one of the embodiments described herein, the retainer is adhered to an external surface of at least two casing components, and optionally forming a portion of an external surface of the casing, thereby maintaining conjunction of the casing components.

In some embodiments, a presence of the retainer at the external surface allows the retainer to be sensitive to the environment, for example, such that in a certain environment, the retainer ceases to maintain conjunction of casing components (e.g., due to dissolution of at least a portion of the retainer, as described herein).

In some embodiments of any one of the embodiments described herein, the retainer comprises an enteric polymer (as defined herein).

The pH dependency of solubility of an enteric polymer allows the enteric polymer to serve as a solid substance in the stomach, as well as under dry conditions (e.g., prior to oral administration), while dissolving in at least a portion of the intestines. Thus, the structure of a retainer comprising an enteric polymer is affected by the location of the casing in the gastrointestinal tract.

In some embodiments of any one of the embodiments described herein, the enteric polymer is soluble in an aqueous solution at pH 5.5. In some such embodiments, dissolution of the enteric polymer is effected soon after the drug delivery system reaches the intestines, for example, in the duodenum.

In some embodiments of any one of the embodiments described herein, the enteric polymer is not soluble in an aqueous solution at pH 5.5, and is soluble in an aqueous solution at pH 6.0. In some such embodiments, dissolution of the enteric polymer is effected relatively soon after the drug delivery system reaches the intestines, for example, in the duodenum.

In some embodiments of any one of the embodiments described herein, the enteric polymer is not soluble in an aqueous solution at pH 5.5 or 6.0, and is soluble in an aqueous solution at pH 6.5. In some such embodiments, dissolution of the enteric polymer is effected in the small intestines (e.g., in the jejunum), although optionally not in the duodenum.

In some embodiments of any one of the embodiments described herein, the enteric polymer is not soluble in an aqueous solution at either pH 5.5, 6.0 or 6.5, and is soluble in an aqueous solution at pH 7.0 and/or at pH 7.5. In some such embodiments, dissolution of the enteric polymer is effected in the ileum or colon, and optionally not in the duodenum or jejunum.

In some embodiments of any one of the embodiments described herein, the retainer consists essentially of an enteric polymer. In such embodiments, the whole retainer is potentially soluble under intestinal conditions. However, such a retainer may cease to be functional (e.g., cease to be capable of maintaining a conjunction of casing components) upon partial dissolution of the retainer, e.g., well before the whole retainer has dissolved.

In some embodiments of any one of the embodiments described herein, the retainer comprises an enteric polymer as well as at least one substance other than enteric polymer (e.g., a substance which is water-insoluble at any pH in the gastrointestinal tract). In some such embodiments, the enteric polymer and other substance(s) in the retainer are configured such that dissolution of the enteric polymer in the retainer results in the retainer ceasing to be functional (e.g., ceasing to be capable of maintaining a conjunction of casing components).

In some embodiments of any one of the embodiments described herein, a retainer may cease to be functional (e.g., cease to be capable of maintaining a conjunction of casing components) by separating (e.g., due to degradation) into two or more disconnected portions, for example, wherein a portion of a retainer attached to one casing component ceases to be connected to a portion of the retainer attached to another casing component.

In some embodiments of any one of the embodiments described herein, a retainer may cease to be functional (e.g., cease to be capable of maintaining a conjunction of casing components) by being degraded so as to be breached (without necessarily being degraded into two or more disconnected portions), for example, wherein a retainer forms an integral part of the casing, such that a breach formed in the retainer *per se* forms a breach in the casing.

In some embodiments of any one of the embodiments described herein, a retainer may cease to be functional (e.g., cease to be capable of maintaining a conjunction of casing components) by becoming disconnected (e.g., due to degradation) from one or more casing components, for example, wherein a retainer is adhered to the casing component (prior to degradation).

In some embodiments of any one of the embodiments described herein, a retainer may cease to be functional (e.g., cease to be capable of maintaining a conjunction of casing components) by being deformed (e.g., due to degradation), for example, losing a shape necessary for maintaining a conjunction of casing components (e.g., a shape suitable for clamping casing components).

In some embodiments of any one of the embodiments described herein, the casing comprises a tubular structure.

Herein, the phrase "tubular structure" refers to a structure (e.g., an open cylinder or topological equivalent thereof) having an outer surface, an inner surface which surrounds an inner volume, and two openings on substantially opposite sides of the tubular structure which connect the inner volume to the surroundings of the tubular structure. Optionally, the tubular structure has a substantially circular and/or substantially oval cross-section in a plane perpendicular to an axis between the two openings. However, alternative shapes are also encompassed by the phrase "tubular structure".

Optionally, the two openings of the tubular structure are capped so as to form the casing.

Herein, the term "capped" refers to a presence of a structure (also referred to herein as a "cap") which covers an opening in another structure (e.g., a tubular structure), thereby closing the opening. A cap may optionally be a retainer and/or casing component described herein. Optionally, a cap has a concave surface into which surrounds the opening, for example, wherein the end of a tubular structure fits within a concave surface of a cap.

In some embodiments of any one of the embodiments described herein, at least one opening of a tubular structure described herein is capped by a retainer. In some such embodiments, removal of a retainer breaches the casing by exposing an opening of the tubular structure. In some embodiments, the two openings of the tubular structure are each capped by a retainer (e.g., two separate retainers).

In some embodiments of any one of the embodiments described herein, the casing comprises a flexible component (e.g., as described herein) which comprises a flexible sheet folded into a tubular structure.

Herein, the phrase "flexible sheet" refers to a 3-dimensional structure which is sufficiently thin in one dimension to allow the sheet to be folded into a tubular structure.

In embodiments relating to a flexible sheet folded into a tubular structure, different sides of the sheet which become in conjunction upon folding into a tubular structure are considered herein to be different casing components in conjunction.

Optionally, separation of casing components is effected by unfolding of the flexible sheet (e.g., thereby destroying the tubular structure).

Additionally or alternatively, separation of casing components is effected by a mechanism other than unfolding the flexible sheet, for example, by exposing an opening of the tubular structure (e.g., by at least partial displacement of one or two caps capping the tubular structure).

In embodiments relating to a flexible sheet folded into a tubular structure, each end of the tubular structure is capped by a retainer (e.g., as described herein).

Optionally, separation of casing components is effected by unfolding of the flexible sheet upon removal or one the retainers and/or both of the retainers capping the tubular structure.

In some embodiments of any one of the embodiments described herein, the drug delivery system further comprises a substance which swells upon contact with water, the substance being contained within the casing, and the casing being configured such that upon contact of the casing with an aqueous liquid (e.g., fluid in the stomach and/or intestines), the substance swells. Swelling of the substance may optionally be utilized to generate an internal force described herein. By swelling in contact with water, the substance may optionally provide control over release of the composition comprising PTH and SNAC by, *inter alia*, facilitating release in a time-dependent manner, for example, wherein a degree of swelling (and optionally an internal force) is correlated with the time of exposure to an aqueous liquid (e.g., time from oral administration).

In some embodiments of any one of the embodiments described herein, the casing is water-permeable in at least a portion thereof, such that upon contact with an aqueous liquid, water permeates the casing and causes the substance which swells upon contact with water to swell. In some embodiments, the casing is water-permeable in a portion of the casing which is adjacent to the substance which swells upon contact with water. In some embodiments, the casing is water-permeable only in a portion of the casing which is adjacent to the substance which swells upon contact with water, other portions of the casing being water-impermeable.

In some embodiments of any one of the embodiments described herein, a water-permeable portion of a casing contains at least one perforation, the perforation allowing permeation of water. In some embodiments the portion comprises a plurality of perforations. The perforations are preferably of a size which allows water to permeate but which is sufficiently small to prevent escape of substances (e.g., the substance which swells upon contact with water, the PTH, the SNAC) from within the casing. Techniques for forming small perforations in a drug delivery system are known in the art, and include, for example, laser perforation.

In some embodiments of any one of the embodiments described herein, the casing contains a first compartment comprising the substance which swells upon contact with water, and a second compartment comprising the composition comprising PTH and SNAC. In some embodiments, breaching of the casing upon removal of the retainer is effected by separation of casing components, and the casing is configured such that separation of the casing components breaches the casing in a region containing the second compartment, that is, the breach is effected in the vicinity of the composition comprising PTH and SNAC.

In some embodiments, the first compartment and second compartment are separated by a barrier which limits or prevents contact between contents of the two compartments (e.g., between the substance which swells upon contact with water and the composition comprising PTH and SNAC). In some embodiments, the barrier is movable upon swelling of the substance which swells upon contact with water. In some embodiments, upon swelling of the substance, the barrier moves such that the first compartment expands, and the second compartment shrinks, thereby compressing the composition comprising PTH and SNAC.

In some embodiments, the casing is configured such that the composition comprising PTH and SNAC in the second compartment does not come into contact, prior to breaching of the casing, with the substance which swells upon contact with water, the water-permeable portion of the casing, or a retainer (which is optionally on an external surface of the casing). Without being bound by any particular theory, it is believed that such embodiments are particularly suitable for avoiding potential incompatibility between the PTH and/or SNAC and the substances which provide specific chemical and/or physical properties utilized for controlled release (e.g., a substance which swells upon contact with water, a water-permeable portion of the casing, and/or a retainer which is sensitive to location in the gastrointestinal tract).

In some embodiments, compression of the composition comprising PTH and SNAC results in an internal force on at least a portion of the casing which encloses the second compartment. Optionally, such an internal force facilitates a sudden formation of a relatively large breach (e.g., a breach of a size described herein), thereby allowing for rapid release of the PTH and SNAC.

In some embodiments, compression of the composition comprising PTH and SNAC results in movement of the composition comprising PTH and SNAC towards a region where a breach is formed in the casing upon removal of a retainer, for example, in towards a region adjacent to the retainer. Optionally, such movement of the composition comprising PTH and SNAC facilitates rapid release of the PTH and SNAC once the breach is formed.

A variety of substances which swell upon contact with water, and which are suitable for use in a drug delivery system, are known in the art, and may optionally be used in embodiments of the invention. Such substances are commonly referred to in the art as "disintegrants". Any substance known in the art as a "disintegrant" (including the term "superdisintegrant") suitable for use in a drug delivery system is encompassed herein by the phrase "substance which swells upon contact with water". Examples of such substances include, without limitation, povidone, crospovidone, croscarmellose (e.g., croscarmellose sodium), carboxymethylcellulose (e.g., calcium carboxymethylcellulose, sodium carboxymethylcellulose), hydroxypropyl methylcellulose, starch (e.g., corn starch, potato starch, wheat starch, tapioca starch, rice starch), modified starch (e.g., sodium starch glycolate) and silicon dioxide (e.g., colloidal silicon dioxide).

In some embodiments of any one of the embodiments described herein, the substance which swells upon contact with water is water-insoluble at 37 °C. Examples of water insoluble substances which swell upon contact with water include, without limitation, cross-linked hydrophilic polymers such as crospovidone and croscarmellose, as well as starch and derivatives thereof. Water-insolubility may optionally be beneficial in reducing escape of the substance from the casing (e.g., via a perforation described herein), which could reduce the degree of swelling of the substance within the casing.

As discussed herein, except for at least a portion of a retainer, the various portions of the casing, for example, casing components and barriers according to respective embodiments described herein, as well as portions of a retainer (e.g., a retainer which comprises an enteric polymer described herein but does not consist of an enteric polymer), may optionally be prepared from any of a wide variety of substances, including relatively inert substances.

In some embodiments of any one of the embodiments described herein, such portions of the casing comprise a polymeric substance, and optionally consist of a polymeric substance. In some embodiments, the polymeric substance is a hydrophobic polymeric substance. Examples of hydrophobic polymeric substances include, without limitation, ethyl cellulose and other hydrophobic cellulose ethers, polyvinyl acetate, polyethylene, poly(methyl methacrylate), poly(ethyl methacrylate), poly(methyl acrylate), poly(ethyl acrylate) and copolymers thereof (e.g., poly(ethylene-co-vinyl acetate), poly(ethyl acrylate-co-methyl methacrylate)).

In some embodiments of any one of the embodiments described herein, the hydrophobic polymeric substance is characterized in that at a pH of 7.0, the polymeric substance is both water-insoluble and does not absorb more than 20 weight percents of water (weight of absorbed water relative to weight of substance). In some embodiments, the hydrophobic polymeric substance is characterized in that it does not absorb more than 10 weight percents of water at pH 7.0. In some embodiments, the hydrophobic polymeric substance is characterized in that it does not absorb more than 5 weight percents of water at pH 7.0. In some embodiments, the hydrophobic polymeric substance is characterized in that it does not absorb more than 2 weight percents of water at pH 7.0. In some embodiments, the hydrophobic polymeric substance is characterized in that it does not absorb more than 1 weight percents of water at pH 7.0.

Without being bound by any particular theory, it is believed that hydrophobic polymeric substances tend to be relatively inert due, for example, to a deficiency in capability to form non-covalent bonds such as hydrogen bonds and ionic bonds (this deficiency typically being associated with hydrophobicity).

In some embodiments of any one of the embodiments described herein, the casing is formed from at least one substance (e.g., polymeric substance) which is water-insoluble at a pH of 7.0, with the optional exception of any enteric polymer described herein which is water-soluble at pH 7.0. It is to be appreciated that the aforementioned substance which is water-insoluble at a pH of 7.0 may optionally be enteric polymers which are soluble only at a pH above 7.0. In such embodiments, such portions of the casing comprise a polymeric substance, and optionally consist of a polymeric substance.

Optionally, the substance comprises a mixture of water-insoluble and water-soluble polymers, such that the mixture dissolves only slowly in aqueous solution (e.g., when the percentage of water-soluble polymer in the mixture is small). Such substances are considered herein to be water-insoluble if less than 1 gram dissolves in 1 liter of aqueous solution within 24 hours at 37 °C (with gentle stirring).

Drug delivery systems described herein may optionally be formulated to provide desired pharmacokinetics, for example, by selecting an appropriate configuration of the casing, shape and/or thickness of a retainer, pH dependence of an enteric polymer in a retainer, an amount and/or type of substance which swells upon contact with water, and/or a water-permeability of a water-permeable portion of the casing described herein.

In some embodiments of any one of the embodiments described herein, the drug delivery system is formulated such that absorption of the PTH occurs rapidly once the casing is breached. It is to be appreciated that rapid release of the PTH and SNAC allows for control over the location of release in the gastrointestinal tract, because gradual release from a formulation will generally result in PTH and SNAC being released over a long portion of the intestinal tract, due to movement of the formulation through the gastrointestinal tract.

Without being bound by any particular theory, it is believed that drug delivery systems according to some embodiments described herein are particularly suitable for obtaining rapid release of PTH and SNAC and absorption of PTH (following a controlled delay prior to breaching of the casing), because the breach may optionally be large enough to allow for rapid release (and subsequent absorption) of the entire amount of PTH and SNAC in the drug delivery system, whereas alternative methodologies (e.g., for releasing agents in the intestines rather than in the stomach), such as use of gradually disintegrating and/or dissolving coatings and/or compositions, may result in a relatively gradual release of PTH and SNAC through a small opening in a gradually disintegrating/dissolving coating and/or gradual disintegration/dissolution of a composition comprising PTH and SNAC.

In some embodiments of any one of the embodiments described herein, the drug delivery system enhances the efficacy of delivery (e.g., as reflected by bioavailability) of the PTH, as compared to administration of the contents of the composition comprising PTH and SNAC without the casing described herein. Enhancement of the efficacy may be, for example, due to protection of the PTH, SNAC and/or protease inhibitor from gastric conditions.

In some embodiments of any one of the embodiments described herein, the drug delivery system is formulated such that absorption of the PTH following oral administration of the drug delivery system is characterized by a bioavailability of the PTH which is at least 20 % higher than (120 % of the level of) a bioavailability of the PTH following oral administration of the composition comprising PTH and SNAC (according to any of the respective embodiments described herein) without the casing of the drug delivery system, that is, the same composition as that within the casing of the drug delivery system. In some embodiments, the bioavailability is at least 50 % higher than (150 % of the level of) the bioavailability upon oral administration without the casing. In some embodiments, the bioavailability is at least twice (200 % of the level of) the bioavailability upon oral administration without the casing. In some embodiments, the bioavailability is at least four-fold (400 % of the level of) the bioavailability upon oral administration without the casing. In some embodiments, the bioavailability is at least ten-fold (1000 % of the level of) the bioavailability upon oral administration without the casing. In some embodiments, the bioavailability is at least twenty-fold (2000 % of the level of) the bioavailability upon oral administration without the casing.

### Miscellaneous definitions:

Herein and in the art, the term "hypoparathyroidism" refers to a disorder characterized by decreased production of parathyroid hormone, typically due to decreased function of the parathyroid glands, for example, due to absence or damage to the parathyroid glands (e.g., due to surgery, autoimmune invasion, magnesium deficiency, hemochromatosis, absence of the glands at birth, and/or various genetic disorders). Examples of symptoms of low parathyroid hormone levels include, without limitation, low blood calcium level (which may lead to seizures, irregular heart beat and/or airway spasms), paresthesia, muscle cramps and tetany.

The term "polypeptide" as used herein encompasses native polypeptides (e.g., degradation products, synthetically synthesized polypeptides and/or recombinant polypeptides), including, without limitation, native proteins, fragments of native proteins and homologs of native proteins and/or fragments thereof; as well as peptidomimetics (typically, synthetically synthesized polypeptides) and peptoids and semipeptoids which are polypeptide analogs, which may have, for example, modifications rendering the polypeptides (e.g., PTH and/or protease inhibitor) more stable while in a body or more capable of penetrating into cells. Such modifications include, but are not limited to N terminus modification, C terminus modification, peptide bond modification, backbone modifications, and residue modification.

Methods for preparing peptidomimetic compounds are well known in the art and are specified, for example, in Quantitative Drug Design, C.A. Ramsden Gd., Chapter 17.2, F. Choplin Pergamon Press (1992).
Further details in this respect are provided herein below.

Peptide bonds (-CO-NH-) within the polypeptide (e.g., PTH and/or protease inhibitor) may be substituted, for example, by N-methylated amide bonds (-N(CH₃)-CO-), ester bonds (-C(=O)-O-), ketomethylene bonds (-CO-CH₂-), sulfinylmethylene bonds (-S(=O)-CH₂-), α-aza bonds (-NH-N(R)-CO-), wherein R is any alkyl (e.g., methyl), amine bonds (-CH₂-NH-), sulfide bonds (-CH₂-S-), ethylene bonds (-CH₂-CH₂-), hydroxyethylene bonds (-CH(OH)-CH₂-), thioamide bonds (-CS-NH-), olefinic double bonds (-CH=CH-), fluorinated olefinic double bonds (-CF=CH-), retro amide bonds (-NH-CO-), peptide derivatives (-N(R)-CH2-CO-), wherein R is the "normal" side chain, naturally present on the carbon atom.

These modifications can occur at any of the bonds along the polypeptide chain and even at several (2-3) bonds at the same time.

Natural aromatic amino acids, Trp, Tyr and Phe, may be substituted by non-natural aromatic amino acids such as 1,2,3,4-tetrahydroisoquinoline-3-carboxylic acid (Tic), naphthylalanine, ring-methylated derivatives of Phe, halogenated derivatives of Phe or O-methyl-Tyr.

The polypeptides of some embodiments of the invention (e.g., PTH and/or a protease inhibitor described herein) may also include one or more modified amino acids or one or more non-amino acid monomers (e.g. fatty acids, complex carbohydrates etc.).

The term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Tables 1 and 2 below list naturally occurring amino acids (Table 1), and non-conventional or modified amino acids (e.g., synthetic, Table 2) which can be used with some embodiments of the invention.

**Table 1**

| ***Amino Acid*** | ***Three-Letter Abbreviation*** | ***One-letter Symbol*** |
|---|---|---|
| Alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |
| Any amino acid as above | Xaa | X |

**Table 2**

| ***Non-conventional amino acid*** | ***Code*** | ***Non-conventional amino acid*** | ***Code*** |
|---|---|---|---|
| ornithine | Orn | hydroxyproline | Hyp |
| α-aminobutyric acid | Abu | aminonorbornyl-carboxylate | Norb |
| D-alanine | Dala | aminocyclopropane-carboxylate | Cpro |
| D-arginine | Darg | N-(3-guanidinopropyl)glycine | Narg |
| D-asparagine | Dasn | N-(carbamylmethyl)glycine | Nasn |
| D-aspartic acid | Dasp | N-(carboxymethyl)glycine | Nasp |
| D-cysteine | Dcys | N-(thiomethyl)glycine | Ncys |
| D-glutamine | Dgln | N-(2-carbamylethyl)glycine | Ngln |
| D-glutamic acid | Dglu | N-(2-carboxyethyl)glycine | Nglu |
| D-histidine | Dhis | N-(imidazolylethyl)glycine | Nhis |
| D-isoleucine | Dile | N-(1-methylpropyl)glycine | Nile |
| D-leucine | Dleu | N-(2-methylpropyl)glycine | Nleu |
| D-lysine | Dlys | N-(4-aminobutyl)glycine | Nlys |
| D-methionine | Dmet | N-(2-methylthioethyl)glycine | Nmet |
| D-ornithine | Dorn | N-(3-aminopropyl)glycine | Norn |
| D-phenylalanine | Dphe | N-benzylglycine | Nphe |
| D-proline | Dpro | N-(hydroxymethyl)glycine | Nser |
| D-serine | Dser | N-(1-hydroxyethyl)glycine | Nthr |
| D-threonine | Dthr | N-(3-indolylethyl) glycine | Nhtrp |
| D-tryptophan | Dtrp | N-(*p*-hydroxyphenyl)glycine | Ntyr |
| D-tyrosine | Dtyr | N-(1-methylethyl)glycine | Nval |
| D-valine | Dval | N-methylglycine | Nmgly |
| D-N-methylalanine | Dnmala | L-N-methylalanine | Nmala |
| D-N-methylarginine | Dnmarg | L-N-methylarginine | Nmarg |
| D-N-methylasparagine | Dnmasn | L-N-methylasparagine | Nmasn |
| D-N-methylasparatate | Dnmasp | L-N-methylaspartic acid | Nmasp |
| D-N-methylcysteine | Dnmcys | L-N-methylcysteine | Nmcys |
| D-N-methylglutamine | Dnmgln | L-N-methylglutamine | Nmgln |
| D-N-methylglutamate | Dnmglu | L-N-methylglutamic acid | Nmglu |
| D-N-methylhistidine | Dnmhis | L-N-methylhistidine | Nmhis |
| D-N-methylisoleucine | Dnmile | L-N-methylisolleucine | Nmile |
| D-N-methylleucine | Dnmleu | L-N-methylleucine | Nmleu |
| D-N-methyllysine | Dnmlys | L-N-methyllysine | Nmlys |
| D-N-methylmethionine | Dnmmet | L-N-methylmethionine | Nmmet |
| D-N-methylomithine | Dnmorn | L-N-methylomithine | Nmorn |
| D-N-methylphenylalanine | Dnmphe | L-N-methylphenylalanine | Nmphe |
| D-N-methylproline | Dnmpro | L-N-methylproline | Nmpro |
| D-N-methylserine | Dnmser | L-N-methylserine | Nmser |
| D-N-methylthreonine | Dnmthr | L-N-methylthreonine | Nmthr |
| D-N-methyltryptophan | Dnmtrp | L-N-methyltryptophan | Nmtrp |
| D-N-methyltyrosine | Dnmtyr | L-N-methyltyrosine | Nmtyr |
| D-N-methylvaline | Dnmval | L-N-methylvaline | Nmval |
| L-norleucine | Nle | L-N-methylnorleucine | Nmnle |
| L-norvaline | Nva | L-N-methylnorvaline | Nmnva |
| L-ethylglycine | Etg | L-N-methyl-ethylglycine | Nmetg |
| L-t-butylglycine | Tbug | L-N-methyl-t-butylglycine | Nmtbug |
| L-homophenylalanine | Hphe | L-N-methyl-homophenylalanine | Nmhphe |
| α-naphthylalanine | Anap | N-methyl-α-naphthylalanine | Nmanap |
| penicillamine | Pen | N-methylpenicillamine | Nmpen |
| γ-aminobutyric acid | Gabu | N-methyl-γ-aminobutyrate | Nmgabu |
| cyclohexylalanine | Chexa | N-methyl-cyclohexylalanine | Nmchexa |
| cyclopentylalanine | Cpen | N-methyl-cyclopentylalanine | Nmcpen |
| α-amino-α-methylbutyrate | Aabu | N-methyl-α-amino-α-methylbutyrate | Nmaabu |
| α-aminoisobutyric acid | Aib | N-methyl-α-aminoisobutyrate | Nmaib |
| D-α-methylarginine | Dmarg | L-α-methylarginine | Marg |
| D-α-methylasparagine | Dmasn | L-α-methylasparagine | Masn |
| D-α-methylaspartate | Dmasp | L-α-methylaspartate | Masp |
| D-α-methylcysteine | Dmcys | L-α-methylcysteine | Mcys |
| D-α-methylglutamine | Dmgln | L-α-methylglutamine | Mgln |
| D-α-methyl glutamic acid | Dmglu | L-α-methylglutamate | Mglu |
| D-α-methylhistidine | Dmhis | L-α-methylhistidine | Mhis |
| D-α-methylisoleucine | Dmile | L-α-methylisoleucine | Mile |
| D-α-methylleucine | Dmleu | L-α-methylleucine | Mleu |
| D-α-methyllysine | Dmlys | L-α-methyllysine | Mlys |
| D-α-methylmethionine | Dmmet | L-α-methylmethionine | Mmet |
| D-α-methylornithine | Dmorn | L-α-methylornithine | Morn |
| D-α-methylphenylalanine | Dmphe | L-α-methylphenylalanine | Mphe |
| D-α-methylproline | Dmpro | L-α-methylproline | Mpro |
| D-α-methylserine | Dmser | L-α-methylserine | Mser |
| D-α-methylthreonine | Dmthr | L-α-methylthreonine | Mthr |
| D-α-methyltryptophan | Dmtrp | L-α-methyltryptophan | Mtrp |
| D-α-methyltyrosine | Dmtyr | L-α-methyltyrosine | Mtyr |
| D-α-methylvaline | Dmval | L-α-methylvaline | Mval |
| N-cyclobutylglycine | Ncbut | L-α-methylnorvaline | Mnva |
| N-cycloheptylglycine | Nchep | L-α-methylethylglycine | Metg |
| N-cyclohexylglycine | Nchex | L-α-methyl-t-butylglycine | Mtbug |
| N-cyclodecylglycine | Ncdec | L-α-methyl-homophenylalanine | Mhphe |
| N-cyclododecylglycine | Ncdod | α-methyl-α-naphthylalanine | Manap |
| N-cyclooctylglycine | Ncoct | α-methylpenicillamine | Mpen |
| N-cyclopropylglycine | Ncpro | α-methyl-y-aminobutyrate | Mgabu |
| N-cycloundecylglycine | Ncund | α-methyl-cyclohexylalanine | Mchexa |
| N-(2-aminoethyl)glycine | Naeg | α-methyl-cyclopentylalanine | Mcpen |
| N-(2,2-diphenylethyl)glycine | Nbhm | N-(N-(2,2-diphenylethyl) carbamylmethyl-glycine | Nnbhm |
| N-(3,3-diphenylpropyl)glycine | Nbhe | N-(N-(3,3-diphenylpropyl) carbamylmethyl-glycine | Nnbhe |
| 1-carboxy-1-(2,2-diphenyl ethylamino)cyclopropane | Nmbc | 1,2,3,4-tetrahydroisoquinoline-3-carboxylic acid | Tic |
| phosphoserine | pSer | phosphothreonine | pThr |
| phosphotyrosine | pTyr | O-methyl-tyrosine | |
| 2-aminoadipic acid | | hydroxylysine | |

The polypeptides of some embodiments of the invention (e.g., PTH and/or a protease inhibitor described herein) are preferably utilized in a linear form, although it will be appreciated that in cases where cyclization does not severely interfere with polypeptide characteristics, cyclic forms of the polypeptide can also be utilized.

In some embodiments of any one of the embodiments described herein, the polypeptide (e.g., PTH and/or protease inhibitor) is water-soluble.

Herein throughout, the term "soluble" refers to a compound having a solubility of at least 1 gram per liter in an indicated solvent at 37 °C.

For example, the term "water-soluble" refers to a compound having a solubility of at least 1 gram per liter in an aqueous solution at pH 7, unless another pH is explicitly indicated.

Herein throughout, the term "insoluble" refers to a compound having a solubility of less than 1 gram per liter in an indicated solvent at 37 °C. For example, the term "water-insoluble" refers herein to a compound having a solubility of less than 1 gram per liter in an aqueous solution at pH 7, unless another pH is explicitly indicated.

Water-soluble polypeptides preferably include one or more (non-natural or natural) polar amino acids, including but not limited to serine and threonine which are capable of increasing polypeptide water-solubility due to their hydroxyl-containing side chain. Optionally, a homolog of a polypeptide is selected so as to be more water-soluble than the parent polypeptide, for example, by replacing one or more amino acids in the polypeptide with polar amino acids.

The polypeptides of some embodiments of the invention (e.g., PTH and/or a protease inhibitor described herein) may be synthesized by any techniques that are known to those skilled in the art of peptide synthesis. For solid phase peptide synthesis, a summary of the many techniques may be found in J. M. Stewart and J. D. Young, Solid Phase Peptide Synthesis, W. H. Freeman Co. (San Francisco), 1963 and J. Meienhofer, Hormonal Proteins and Peptides, vol. 2, p. 46, Academic Press (New York), 1973. For classical solution synthesis see G. Schroder and K. Lupke, The Peptides, vol. 1, Academic Press (New York), 1965.

In general, these methods comprise the sequential addition of one or more amino acids or suitably protected amino acids to a growing polypeptide chain. Normally, either the amino or carboxyl group of the first amino acid is protected by a suitable protecting group. The protected or derivatized amino acid can then either be attached to an inert solid support or utilized in solution by adding the next amino acid in the sequence having the complimentary (amino or carboxyl) group suitably protected, under conditions suitable for forming the amide linkage. The protecting group is then removed from this newly added amino acid residue and the next amino acid (suitably protected) is then added, and so forth. After all the desired amino acids have been linked in the proper sequence, any remaining protecting groups (and any solid support) are removed sequentially or concurrently, to afford the final polypeptide compound. By simple modification of this general procedure, it is possible to add more than one amino acid at a time to a growing chain, for example, by coupling (under conditions which do not racemize chiral centers) a protected tripeptide with a properly protected dipeptide to form, after deprotection, a pentapeptide and so forth. Further description of peptide synthesis is disclosed in U.S. Pat. No. 6,472,505.

A preferred method of preparing the polypeptide compounds of some embodiments of the invention (e.g., PTH and/or a protease inhibitor described herein) involves solid phase peptide synthesis.

Large scale polypeptide synthesis is described by Andersson et al. [Biopolymers 2000; 55:227-250].

Herein, a "homolog" of a given polypeptide (e.g., PTH(1-84) or a fragment thereof) refers to a polypeptide that exhibits at least 80 % homology, preferably at least 90 % homology, and more preferably at least 95 % homology, and more preferably at least 98 % homology to the given polypeptide. In some embodiments, a homolog of a given polypeptide further shares a therapeutic activity with the given polypeptide. The percentage of homology refers to the percentage of amino acid residues in a first polypeptide sequence which match a corresponding residue of a second polypeptide sequence to which the first polypeptide is being compared.

Generally, the polypeptides are aligned to give maximum homology. A variety of strategies are known in the art for performing comparisons of amino acid or nucleotide sequences in order to assess degrees of identity, including, for example, manual alignment, computer assisted sequence alignment and combinations thereof.

A number of algorithms (which are generally computer implemented) for performing sequence alignment are widely available, or can be produced by one of skill in the art. Representative algorithms include, e.g., the local homology algorithm of Smith and Waterman (Adv. Appl. Math., 1981, 2: 482); the homology alignment algorithm of Needleman and Wunsch (J. Mol. Biol., 1970, 48: 443); the search for similarity method of Pearson and Lipman (Proc. Natl. Acad. Sci. (USA), 1988, 85: 2444); and/or by computerized implementations of these algorithms (e.g., GAP, BESTFIT, FASTA, and TFASTA in the Wisconsin Genetics Software Package Release 7.0, Genetics Computer Group, 575 Science Dr., Madison, Wis.). Readily available computer programs incorporating such algorithms include, for example, BLASTN, BLASTP, Gapped BLAST, PILEUP, CLUSTALW etc. When utilizing BLAST and Gapped BLAST programs, default parameters of the respective programs may be used. Alternatively, the practitioner may use non-default parameters depending on his or her experimental and/or other requirements (see for example, the Web site having URL www(dot)ncbi(dot)nlm(dot)nih(dot)gov).

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an" and "the" include plural references unless the context clearly dictates otherwise. For example, the term "a protease inhibitor" may include a plurality of protease inhibitors, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non-limiting fashion.

### MATERIALS AND METHODS

### Materials:

8-Aminocaprylic acid was obtained from Alfa-Aesar.

Magnesium stearate was obtained from Sigma-Aldrich.

O-acetylsalicyloyl chloride was obtained from Sigma-Aldrich.

Soybean trypsin inhibitor (SBTI) was obtained from Sigma-Aldrich.

Teriparatide was purchased from Bachem.

Sodium bicarbonate was obtained from Merck.

SNAC (sodium 8-N-(2-hydroxybenzoyl)aminocaprylate) was prepared by reacting O-acetylsalicyloyl chloride with 8-aminocaprylic acid.

### Powder blend and tablet preparation:

Teriparatide tablets were prepared by thoroughly mixing teriparatide with the other ingredients of the formulation by geometric dilution in a mortar with a pestle. Cylindrical tablets were prepared by direct compression of the formulation ingredients using a manually operated single punch tablet press (Korsch, Germany) fitted with an 8 mm biconvex punch and die set, and applying pressure for 20 seconds.

### Dissolution and disintegration measurement:

Dissolution and disintegration was measured using a tablet dissolution/disintegration tester (PJ-3, China). The time required for the full dissolution/disintegration of the tablet was determined. The USP paddle method II (USP 23) was used for dissolution measurement. Rotation speed was 50 or 100 rotations per minute (as indicated below for each experiment) and the volume of the indicated dissolution medium was 900 ml, maintained at 37 °C. Disintegration measurement was performed according to USP protocol using 800 ml of medium maintained at 37 °C. Full dissolution was defined as complete absence of visible formulation at the glass bottom. Full disintegration was defined as a state wherein any residue of the tablet remaining on the screen of the test apparatus is a soft mass having no palpably firm core.

### EXAMPLE 1

### Phase I clinical trial of orally administered parathyroid hormone (PTH)

A Phase I clinical study of exemplary oral formulations comprising teriparatide (parathyroid hormone (1-34)) was conducted at the Hadassah Clinical Research Center. 42 healthy volunteers were included throughout the study.

The formulation was composed of teriparatide (200, 400, 680, 1400 or 1800 µg), SNAC (sodium 8-N-(2-hydroxybenzoyl) aminocaprylate), soybean trypsin inhibitor and magnesium stearate.

Tablets were administered in the morning after an 8-hour overnight fast and immediately followed by 150 ml of water. At each visit a standard meal was provided 3 hours after drug administration. Patients did not eat or drink alcoholic or caffeinated beverages.

To determine parathyroid hormone(1-34) concentrations, blood samples (4 ml each) were drawn via an indwelling catheter from the forearm vein at predetermined time points. The cannula was flushed with 1.5 ml normal saline after each sampling. In addition, to avoid sample dilution, 1 ml of blood was drawn and discarded before the next sample. The blood samples were taken at following times: baseline (predose), 15 minutes, 30 minutes, 45 minutes, 60 minutes, 75 minutes, 90 minutes, 105 minutes, 2 hours, 3 hours, 4 hours and 5 hours post-administration. Each blood sample was collected into a single tube containing EDTA (ethylenediaminetetraacetic acid) and placed on ice. Within 15 minutes of blood collection, samples were centrifuged for 10 minutes at 4 °C (2500 rotations per minute) and the plasma was separated and divided into two or three aliquots. Each aliquot was transferred into appropriately labeled polypropylene tubes and stored at approximately -20 °C pending analysis. PTH levels were measured using an IDS-iSYS automated assay for the measurement of intact PTH in human plasma or serum. The results of the assay do not include levels of PTH(1-84) such as endogenous PTH.

Oral administration of teriparatide was performed as described hereinabove at doses of 200, 400, 680, 1400 or 1800 µg. The Cmax of PTH(1-34) for each orally administered dose was compared with the Cmax of PTH(1-34) for subcutaneous injection of 20 µg teriparatide.

As shown in FIG. 1, the Cmax of PTH(1-34) following oral administration was proportional to the dose, with oral administration of roughly 750 µg teriparatide providing a Cmax equivalent to that of subcutaneous administration of 20 µg teriparatide.

As shown in FIG. 2, average PTH(1-34) levels upon oral administration of 1800 µg teriparatide and subcutaneous administration of 20 µg teriparatide were characterized by similar Cmax values.

It is to be appreciated that a pharmacokinetic profile for an individual administration (e.g., as represented by FIG. 1) is characterized by a narrower and higher curve than the curve shown in FIG. 2, because averaging data from different measurements (e.g., as shown in FIG. 2) results in a broader and lower curve, due to slight variations in Tmax between individual administrations.

In addition, plasma levels of cAMP, a known marker of PTH activity, were determined in order to confirm biological activity of administered PTH. cAMP plasma levels were measured following oral administration of 680 µg teriparatide or subcutaneous injection of 20 µg teriparatide, as described hereinabove.

As shown in FIG. 3, oral administration of 680 µg teriparatide and subcutaneous administration of 20 µg teriparatide increased plasma cAMP levels to a similar degree. This result confirms that the orally administered PTH exhibits biological activity.

These results indicate that oral administration of PTH results in biologically active increases in PTH levels.

### EXAMPLE 2

### Phase II clinical trial of orally administered parathyroid hormone (PTH)

A Phase IIa multi-center clinical study of an exemplary oral formulation comprising teriparatide was conducted on 19 patients with established primary hypoparathyroidism (PHP) (for more than one year). PHP was postsurgical in 13 patients, autoimmune in 5 patients and hereditary in 1 patient.

The average age of the patients was 44.5 years (range 20.3 to 71 years). At enrollment, the patients were treated with an average of 3.64 grams calcium supplement per day (range 1-10.8 grams per day) and 1.1 µg alphacalcidol supplement per day (range 0-2 µg per day). Patients with low hemoglobin, estimated glomerular filtration rate (eGFR) of less than 40 ml per minute per 1.73m², liver enzymes at least 3 times the upper limit, drug or alcohol abuse, kidney/urinary tract stones, 25-hydroxycholecalciferol (25(OH)D) serum levels of less than 20 ng/ml or BMI outside the range of 18-230 kg/m² were excluded from the study.

The formulation was composed of teriparatide (750 µg per dose), SNAC, soybean trypsin inhibitor and magnesium stearate.

Teriparatide was administered 4 times per day, prior to meals, for 16 weeks. Following the first two doses, plasma samples were taken for pharmacokinetic analysis by determining teriparatide serum levels according to procedures described in Example 1. On the first day of treatment during follow up visits at the end of weeks 1, 2, 3, 4, 6, 8, 10, 12 and 16, serum calcium, phosphorus, albumin, and creatinine were evaluated, and dose adjustment of supplemental calcium medications was performed based on albumin adjusted calcium (ACa) levels. 24 hour urine samples were collected at the end of weeks 8 and 16. Quality of Life was (QoL) was monitored using a standard questionnaire.

The treatment was safe and well tolerated - 17 subjects completed the study (during which over 8000 doses were administered) with no related adverse events and with high (95.6 %) adherence (one subject withdrew consent on day 1; one subject was excluded due to hypercalcemia prior to treatment).

As shown in FIG. 4, the calcium intake was gradually and significantly (p < 0.01) decreased from the end of week 3, while maintaining albumin adjusted calcium at a mean level of 8.15 mg/dL (range 6.97-9.14 md/dL) vs. 7.92 md/dL (range 7.2-8.99 md/dL) at baseline (initiation of treatment). As further shown therein, after 16 weeks, average reduction of calcium intake was 37 %, representing a reduction of 1278 ± 880 mg (from 3692 mg to 2414 mg).

Only one of the 17 subjects who completed the study had urinary calcium above the normal range at the end of 16 weeks. Of the remaining 16 subjects, 13 subjects sustained an average decrease of 34 % in urinary calcium (from 209 mg per 24 hours to 136 mg per 24 hours), and 3 subjects exhibited urinary calcium levels with sustained elevations but which remained more than 30 % below the upper limit of normal (between 106 and 160 mg per 24 hours).

As shown in FIG. 5, serum phosphorus levels were significantly (p < 0.02) reduced after each dose of orally administered teriparatide, throughout the 16 weeks of the study, to an average level of 4.3 mg/dL (range 2.9-5.2 mg/dL). As further shown therein, pre-dose serum phosphorus levels were reduced following 2 weeks of treatment with the orally administered teriparatide.

As shown in FIGs. 6 and 7, PTH(1-34) serum levels were about half is high when teriparatide was orally administered after a meal (second administration on day 1) than when orally administered prior to a meal (on an empty stomach).

The results for a sample case, a 54-year old female suffering symptomatic hypocalcemia who received constant alphacalcidol supplementation (1.5 µg per day), are presented below.

As shown in FIG. 8, calcium intake and urinary calcium levels were both reduced by over 50 % during the course of the 16-week treatment of the patient with orally administered teriparatide.

As shown in FIG. 9, treatment of the patient with orally administered teriparatide gradually increased ACa levels and gradually decreased serum phosphate levels.

In addition, the treatment with orally administered teriparatide increased the patient's total serum levels of 25-hydroxycholecalciferol (25(OH)D) from 32.2 ng/ml at baseline to 36.7 ng/ml at the end of week 4 (although alphacalcidol supplementation was constant, as mentioned hereinabove), and the patient's Quality of Life health score increased from 70 to 80 (data not shown).

As shown in FIG. 10, the patient's PTH(1-34) serum levels were about half is high when teriparatide was orally administered after a meal (second administration on day 1) than when orally administered prior to a meal (on an empty stomach).

These results indicate that the orally administered parathyroid hormone was effective in reducing hypocalcemia in hypoparathyroidism patients, as well as associated conditions such as elevated urinary calcium levels and hyperphosphatemia.

### EXAMPLE 3

### Effect of ethyl cellulose on dissolution of teriparatide tablets

300 mg tablets consisting of parathyroid hormone (PTH) in the form of teriparatide (1 mg), SNAC (sodium 8-N-(2-hydroxybenzoyl)aminocaprylate), soybean trypsin inhibitor (SBTI) and magnesium stearate (a lubricant) were prepared as described hereinabove, with no ethyl cellulose (EC), and with 41.67 or 62.5 weight percents granulated ethyl cellulose (EC). The composition of each formulation is presented in Table 3 below.

The time to full dissolution of each tablet was measured as described hereinabove, using a phosphate buffer solution (pH 6.8) as medium and a rotation speed of 100 rotations per minute.

**Table 3: Composition (by weight) of exemplary tablet formulations**

| **Formulation** | **PTH (mg)** | **SNAC + SBTI + Mg stearate (mg)** | **EC (mg)** |
|---|---|---|---|
| **A** | 1 | 299 | 0 |
| **B** | 1 | 174 | 125 |
| **C** | 1 | 11.5 | 187.5 |

As shown in FIG. 11, ethyl cellulose in the formulation decreased the rate of dissolution in a dose-dependent manner, wherein the formulation with 62.5 weight percents ethyl cellulose dissolved after only 90 minutes, as compared to less than 10 minutes for the formulation without ethyl cellulose.

### EXAMPLE 4

### Effect of ethyl cellulose on disintegration and dissolution of teriparatide tablets

Additional tablets were prepared, as described hereinabove, using ethyl cellulose in the form of a fine powder (100 FP premium), rather than the granulated ethyl cellulose used in the tablets described hereinabove. The tablets consisted of parathyroid hormone (PTH) in the form of teriparatide (0.67 mg), SNAC, SBTI and magnesium stearate, with 30 weight percents ethyl cellulose (EC) or with no ethyl cellulose (EC).

Tablets with 30 weight percents ethyl cellulose were prepared with a weight of 190 mg, whereas tablets without ethyl cellulose were prepared with a weight of 133.4 mg. In this experiment, the amounts of each ingredient (other than ethyl cellulose), rather than the total weight of the tablet, was nearly identical in each of the two tablet formulations. The composition of each formulation is presented in Table 4 below.

**Table 4: Composition (by weight percentage) of exemplary tablet formulations**

| **Formulation** | **PTH (%)** | **SNAC + SBTI + Mg stearate (%)** | **EC (%)** |
|---|---|---|---|
| **ST** | 0.5 | 99.5 | 0 |
| **STEC-30** | 0.35 | 69.65 | 30 |

The time to full disintegration and to full dissolution of each tablet was measured as described hereinabove, at both pH 2 and pH 6, using a rotation speed of 50 rotations per minute. The medium at pH 2 was simulated gastric medium without pepsin, and the medium at pH 6 was double distilled water adjusted to pH 6 with NaOH. The pH of each medium was measured immediately after the disintegration/dissolution test, and it was ascertained that the disintegration/dissolution of the tablet did not significantly affect the pH of the medium.

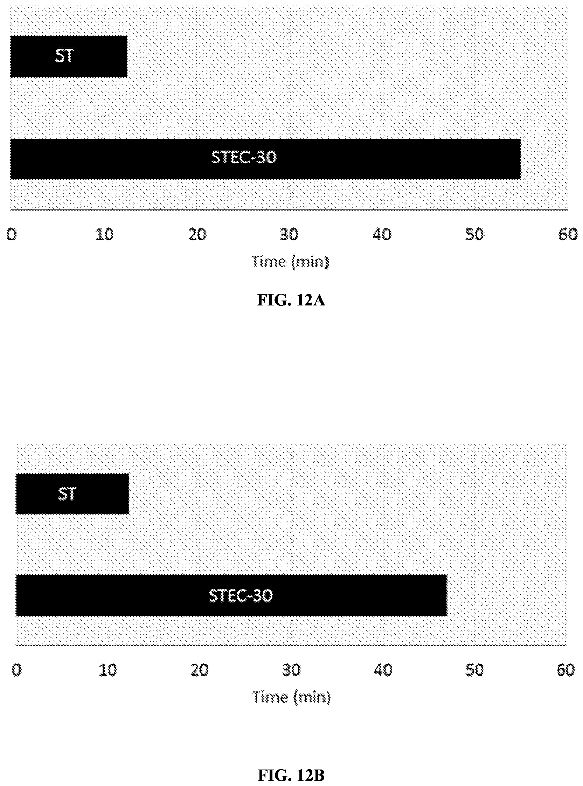

As shown in FIGs. 12A and 12B, ethyl cellulose in the formulation considerably decreased the rate of tablet dissolution at both pH 2 and pH 6. At both pH values, ethyl cellulose increased the time until full dissolution from about 12 minutes to about 50 minutes.

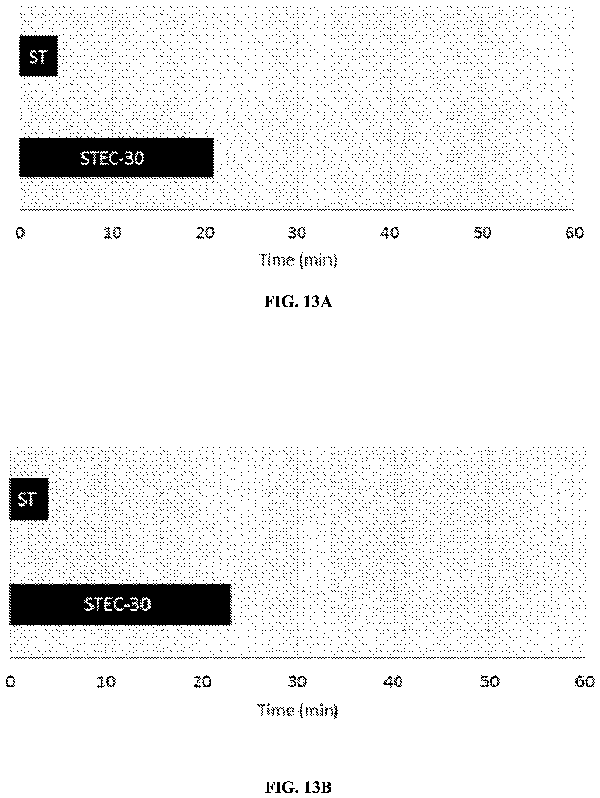

As shown in FIGs. 13A and 13B, ethyl cellulose in the formulation considerably decreased the rate of tablet disintegration at both pH 2 and pH 6. At both pH values, ethyl cellulose increased the time until full disintegration from about 4 minutes to more than 20 minutes.

This result indicates that the slowing of both disintegration and dissolution by ethyl cellulose is a pH-independent phenomenon, and is effected under both conditions of the stomach (pH about 2) and of the intestines (pH about 6).

As further shown in FIGs. 11, 12A and 12B, 30 weight percents of ethyl cellulose in the form of a fine powder lengthened the dissolution time by about four-fold to about 50 minutes (FIGs. 12A and 12B), whereas 41.67 weight percents of granulated ethyl cellulose powder lengthened the dissolution time by about threefold to about 25 minutes (FIG. 11). These results indicate that smaller grains (in the form of a fine powder) of ethyl cellulose are more effective than larger grains of ethyl cellulose in slowing dissolution.

### EXAMPLE 5

### Casing containing parathyroid hormone and swellable substance

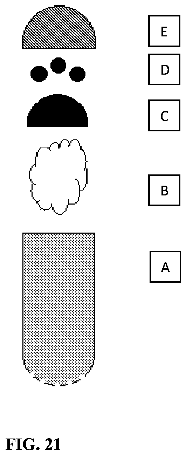

A drug delivery system comprising a casing encapsulating active ingredients, parathyroid hormone and SNAC (sodium 8-N-(2-hydroxybenzoyl)aminocaprylate) (*per se*, or formulated as a pharmaceutical composition according to any of the respective embodiments described herein) according to some embodiments of the invention is optionally assembled as depicted in FIG. 21.

A first casing component (A) is filled with substance which swells upon contact with water (B), followed by a barrier (C), and active ingredients (parathyroid hormone and SNAC) (D), optionally in granular form. A second casing component (E) is then placed in contact with first casing component (A), thereby encapsulating (B), (C) and (D). A layer of enteric polymer (not shown) as described herein in any of the respective embodiments, is then formed over at least a portion of an external surface of casing components (A) and (E). Adhesion of the layer to at least a portion of each of components (A) and (E) prevents separation of components (A) and (B). Assembly may be performed such that active ingredients (D) do not come into contact with any substance of the drug delivery system other than casing component(s) (A) and/or (E) and barrier (C).

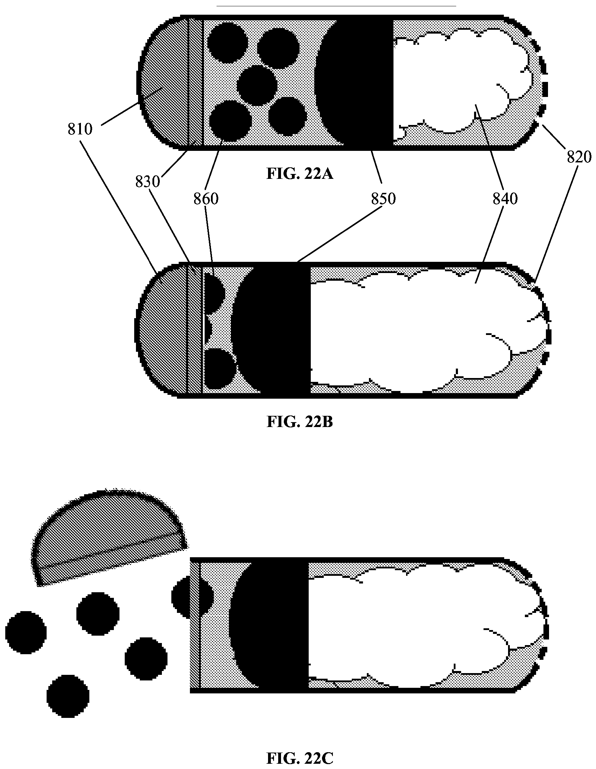

FIGs. 22A-22C show a mechanism of release of active ingredients (parathyroid hormone and SNAC) from a drug delivery system (optionally assembled as described hereinabove and in FIG. 21) according to some embodiments of the invention.

FIG. 22A depicts a drug delivery system according to some embodiments of the invention, prior to administration. The drug delivery system comprises a first casing component **820** which is water-permeable in at least a portion thereof (optionally a perforated portion), as well as second casing component **810** which is in contact with first casing component **820.** Second casing component **810** is preferably water-impermeable. The region where components **810** and **820** are in contact is coated by a layer **830** of enteric polymer (as described herein in any of the respective embodiments), which is adhered to an external surface of both components **810** and **820,** thereby preventing separation of components **810** and **820** from one another. Encapsulated by casing components **810** and **820** are substance **840** which swells upon contact with water, barrier **850,** and active ingredients (parathyroid hormone and SNAC) **860,** optionally in granular form.

FIG. 22B depicts a drug delivery system shown in FIG. 22A, following contact with stomach contents subsequent to oral administration. The drug delivery system comprises the same components as described hereinabove, but differs in that substance **840** is expanded due to permeation of aqueous liquid in the stomach through casing component **820** (optionally via one or more perforations in component **820).** As a result of expansion of substance **840,** barrier **850** is displaced towards active ingredients **860,** which are optionally displaced towards casing component **810** and/or compressed (e.g., reduction of volume of voids in a vicinity of active ingredients **860)** by displacement of barrier **850.** Expansion of substance **840** optionally results in a force being applied to casing component **810,** the force being transmitted by displacement of barrier **850** and by active ingredients **860** (e.g., by resistance of active ingredients **860** to compression). Layer **830** of enteric polymer remains substantially intact, as the enteric polymer does not dissolve in the acidic environment of the stomach, thereby continuing to prevent separation of components **810** and **820** from one another, even in the abovementioned optional presence of a force applied to casing component **810.**

FIG. 22C depicts a drug delivery system shown in FIGs. 22A and 22B, following contact with intestinal contents subsequent to oral administration. The layer of enteric polymer is ruptured as a result of dissolution of enteric polymer in the mildly acidic or non-acidic intestinal environment (e.g., pH of at least 5.5), and optionally further facilitated by the abovementioned force applied to casing component **810.** Upon rupture, the layer no longer prevents separation of the casing components from one another. The casing components separate, and the active ingredients, parathyroid hormone and SNAC, are released rapidly from the casing.

Prior to separation of the casing components and release of the parathyroid hormone and SNAC, the parathyroid hormone and SNAC optionally do not contact any substance in the drug delivery system other than any substance of the drug delivery system other than casing component(s) **810** and/or **820** and barrier **850.**

Casing components **810, 820,** (A) and/or (E) are optionally composed of a polymeric substance which is water-insoluble at a pH of 7.0, optionally a hydrophobic polymeric substance, such as ethyl cellulose. Additionally or alternatively, casing components **810, 820,** (A) and/or (E) are composed of a polymer which is water-insoluble at a pH of 7.0, optionally a hydrophobic polymer, mixed with a small amount of hydrophilic polymer, the mixture dissolving only slowly in aqueous solution.

Substance **840** and/or substance (B) are optionally a substance recognized in the art as a disintegrant suitable for pharmaceutical use.

Barrier **850** and/or barrier (C) are optionally composed of a polymeric substance which is water-insoluble at a pH of 7.0, optionally a hydrophobic polymeric substance, such as ethyl cellulose. Additionally or alternatively, barrier **850** and/or barrier (C) are composed of a polymer which is water-insoluble at a pH of 7.0, optionally a hydrophobic polymer, mixed with a small amount of hydrophilic polymer, the mixture dissolving only slowly in aqueous solution.

The abovementioned enteric polymer (e.g., of layer **830)** is optionally poly(methacrylic acid-co-ethyl acrylate) (optionally with about a 1:1 ratio of methacrylic acid to ethyl acrylate), for example, Eudragit^{®} L100-55.

The abovementioned polymeric substance or polymer which is water-insoluble at a pH of 7.0 is optionally composed of an enteric polymer which dissolves at a pH of above 7.0 (e.g., in a colon).

The active ingredients optionally comprise SNAC in an amount according to any of the respective embodiments described herein, and a therapeutically effective amount of parathyroid hormone, optionally parathyroid hormone (1-34) (e.g., teriparatide), optionally in an amount according to any of the respective embodiments described herein.

### EXAMPLE 6

### Casing containing parathyroid hormone formed from sheet folded into tubular structure

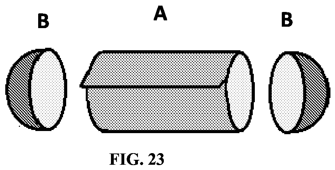

FIG. 23 shows the structure of a casing according to some embodiments of the invention.

A polymeric sheet (A) is folded into a tubular structure. Caps (B) comprising an enteric polymer (as described herein in any of the respective embodiments), optionally consisting essentially of an enteric polymer, are attached at both ends of the tubular structure, thereby forming a closed casing and preventing unfolding of sheet (A) of the tubular structure. Attachment of the caps is optionally effected by an adhesive. Alternatively or additionally, attachment is effected by friction associated with pressure applied to an inner surface of a cap by sheet (A), optionally pressure associated with resistance of the sheet to folding.

The active ingredients parathyroid hormone and SNAC (sodium 8-N-(2-hydroxybenzoyl)aminocaprylate) (not shown) are placed in the casing, optionally prior to prior to attachment of both caps, and optionally subsequent to attachment of one cap and prior to attachment of the second cap.

Upon partial and/or complete dissolution of enteric polymer in one or both caps (B) in an intestinal environment, sheet (A) unfolds, and the parathyroid hormone and SNAC are thereby released rapidly from the casing.

Sheet (A) is optionally composed of a polymeric substance which is water-insoluble at a pH of 7.0, optionally a hydrophobic polymeric substance, such as ethyl cellulose. Additionally or alternatively, sheet (A) is composed of a polymer which is water-insoluble at a pH of 7.0, optionally a hydrophobic polymer, mixed with a small amount of hydrophilic polymer, the mixture dissolving only slowly in aqueous solution.

The abovementioned enteric polymer is optionally poly(methacrylic acid-co-ethyl acrylate) (optionally with about a 1:1 ratio of methacrylic acid to ethyl acrylate), for example, Eudragit^{®} L100-55.

The abovementioned polymeric substance or polymer which is water-insoluble at a pH of 7.0 is optionally composed of an enteric polymer which dissolves at a pH of above 7.0 (e.g., in a colon).

The active ingredients optionally comprise SNAC in an amount according to any of the respective embodiments described herein, and a therapeutically effective amount of parathyroid hormone, optionally parathyroid hormone (1-34) (e.g., teriparatide), optionally in an amount according to any of the respective embodiments described herein.

### EXAMPLE 7

### Effect of antacid on release profile of composition comprising parathyroid hormone and SNAC

Two tablet formulations were prepared, having the same amounts of SNAC, trypsin inhibitor and teriparatide (parathyroid hormone (1-34)), wherein one formulation further contained 100 mg sodium bicarbonate and the other formulation did not contain sodium bicarbonate. The tablets were in a form of a homogeneous mixture.

Each tablet formulation was subjected to a dissolution test in 100 ml of simulated gastric buffer (without pepsin), pH 2.0, at 37 °C, according to USP 23 Apparatus 2 (paddle) with 50 rotations per minute. The amount of released SNAC in each sample was determined chromatographically, using an HPLC apparatus with Cosmosil^{™} 5 C18-MS-II (4.6 ID x 250 mm) column. Mobile phase consisted of 50 % acetonitrile and 50 % phosphoric acid solution (0.1 %). Flow rate was 1ml/minute and injection volume was 25 µl. Amount of released SNAC was calculated as a percentage of the amount of SNAC in the formulation.

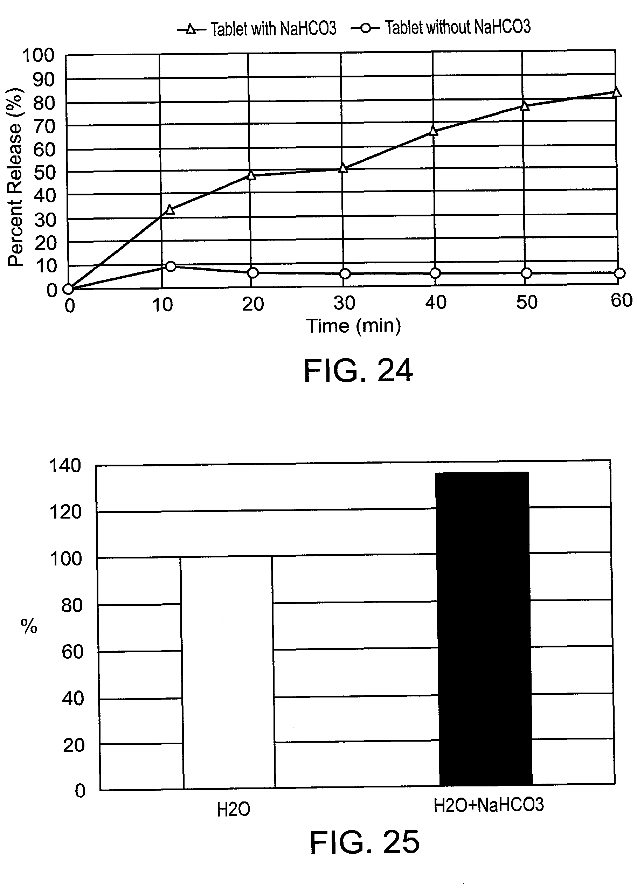

As shown in FIG. 24, sodium bicarbonate considerably enhanced the dissolution of SNAC in tablets, and preserved the soluble fraction of SNAC.

These results indicate that formulation with an antacid such as sodium bicarbonate can considerably enhance the effect of the absorption enhancer SNAC when treating hypoparathyroidism by oral administration PTH.

### EXAMPLE 8

### Effect of antacid on pharmacokinetic profile of orally administered parathyroid hormone (PTH)

An open label comparative pharmacokinetic study was performed on ten healthy volunteers. On different visits, each volunteer received the same oral tablet containing 0.75 mg of teriparatide, a recombinant form of parathyroid hormone (1-34) (PTH(1-34)). In the first visit, the tablet was administered with 150 ml water, whereas in the second visit the tablet was administered with 150 ml of 3 mg/ml sodium bicarbonate aqueous solution.

The formulation was composed of teriparatide (0.75 mg), SNAC (sodium 8-N-(2-hydroxybenzoyl) aminocaprylate), soybean trypsin inhibitor (SBTI) and a small amount of magnesium stearate.

Tablets were administered in the morning after an 8-hour overnight fast. At each visit a standard meal was provided 3 hours after drug administration. Patients did not eat nor drink alcoholic or caffeinated beverages. There was a two week period between the two visits.

To determine PTH(1-34) concentrations, blood samples were drawn and PTH(1-34) levels were measured using an IDS-iSYS automated assay, using procedures described in Example 1 hereinabove. Relative absorption was determined based on the AUC (area under curve) parameter.

As shown in FIG. 25, co-administration with sodium bicarbonate solution increased absorption of PTH(1-34) from an orally administered formulation by about 35 %, in comparison with co-administration of the formulation with water.

These results indicate that co-administration with an antacid enhances the ability of SNAC to promote absorption of orally administered PTH for treatment of hypoparathyroidism.

Citation or identification of any reference in this application shall not be construed as an admission that such reference is available as prior art to the present invention. To the extent that section headings are used, they should not be construed as necessarily limiting.

## Claims

1. A pharmaceutical composition for use in the treatment of hypoparathyroidism by administration of the composition to a subject in need thereof via oral administration, the composition comprising:
teriparatide;
at least one protease inhibitor; and
SNAC (sodium 8-N-(2-hydroxybenzoyl)aminocaprylate),
wherein said composition is for administration via oral administration at least three times per day, and said oral administration is effected at least 4 hours after the most recent food intake.

2. The composition for use according to claim 1, wherein said at least one protease inhibitor comprises at least one trypsin inhibitor.

3. The composition for use according to claim 2, wherein said at least one trypsin inhibitor is selected from the group consisting of lima bean trypsin inhibitor, aprotinin, soybean trypsin inhibitor and ovomucoid trypsin inhibitor.

4. The composition for use according to claim 2, wherein said at least one trypsin inhibitor comprises soybean trypsin inhibitor.

5. The composition for use according to any one of claims 1 to 4, wherein said treatment comprises administration via oral administration of said teriparatide in an amount 400 µg to 20 mg per day.

6. The composition for use according to any one of claims 1 to 5, wherein said treatment comprises administration via oral administration of said teriparatide in an amount in a range of from 100 to 3000 µg.

7. The composition for use according to claim 6, wherein said amount is in a range of from 750 to 3000 µg.

8. The composition for use according to any one of claims 1 to 7, wherein said composition is for administration via oral administration three or four times per day.

9. The composition for use according to any one of claims 1 to 8, wherein the composition is formulated as a tablet.

## Patentansprüche

1. Pharmazeutische Zusammensetzung zur Verwendung bei der Behandlung von Hypoparathyreoidismus durch Verabreichung der Zusammensetzung an einen Probanden, der dieser bedarf, mittels oraler Verabreichung, wobei die Zusammensetzung umfasst:
Teriparatid;
mindestens einen Protease-Inhibitor und
SNAC (Natrium-8-N-(2-Hydroxybenzoyl-aminocaprylat),
wobei die besagte Zusammensetzung zur Verabreichung mittels oraler Verabreichung mindestens dreimal täglich ist und die besagte orale Verabreichung mindestens 4 Stunden nach der jüngsten Nahrungsaufnahme ausgeführt wird.

2. Zusammensetzung zur Verwendung nach Anspruch 1, wobei der besagte mindestens eine Protease-Inhibitor mindestens einen Trypsin-Inhibitor umfasst.

3. Zusammensetzung zur Verwendung nach Anspruch 2, wobei der besagte mindestens eine Trypsin-Inhibitor aus der Gruppe bestehend aus Limabohnen-Trypsin-Inhibitor, Aprotinin, Sojabohnen-Trypsin-Inhibitor und Ovomucoid-Trypsin-Inhibitor ausgewählt ist.

4. Zusammensetzung zur Verwendung nach Anspruch 2, wobei der besagte mindestens eine Trypsin-Inhibitor Sojabohnen-Trypsin-Inhibitor umfasst.

5. Zusammensetzung zur Verwendung nach einem der Ansprüche 1 bis 4, wobei die besagte Behandlung eine Verabreichung mittels oraler Verabreichung des besagten Teriparatids in einer Menge von 400 µg bis 20 mg täglich umfasst.

6. Zusammensetzung zur Verwendung nach einem der Ansprüche 1 bis 5, wobei die besagte Behandlung eine Verabreichung mittels oraler Verabreichung des besagten Teriparatids in einer Menge in einem Bereich von 100 bis 3000 µg umfasst.

7. Zusammensetzung zur Verwendung nach Anspruch 6, wobei die besagte Menge in einem Bereich von 750 bis 3000 µg liegt.

8. Zusammensetzung zur Verwendung nach einem der Ansprüche 1 bis 7, wobei die besagte Zusammensetzung zur Verabreichung mittels oraler Verabreichung drei- oder viermal täglich ist.

9. Zusammensetzung zur Verwendung nach einem der Ansprüche 1 bis 8, wobei die Zusammensetzung als eine Tablette formuliert ist.

## Revendications

1. Composition pharmaceutique destinée à être utilisée dans le traitement de l'hypoparathyroïdie par administration de la composition à un sujet en ayant besoin par administration par voie orale, la composition comprenant :
du tériparatide ;
au moins un inhibiteur de protéase ; et
du SNAC (8-N-(2-hydroxybenzoyl)aminocaprylate de sodium), dans laquelle ladite composition est destinée à une administration par administration par voie orale au moins trois fois par jour, et ladite administration par voie orale est effectuée au moins 4 heures après la prise de nourriture la plus récente.

2. Composition destinée à être utilisée selon la revendication 1, dans laquelle ledit au moins un inhibiteur de protéase comprend au moins un inhibiteur de trypsine.

3. Composition destinée à être utilisée selon la revendication 2, dans laquelle ledit au moins un inhibiteur de trypsine est choisi parmi le groupe constitué par un inhibiteur de trypsine de haricot de Lima, l'aprotinine, un inhibiteur de trypsine de graines de soja et un inhibiteur de trypsine d'ovomucoïde.

4. Composition destinée à être utilisée selon la revendication 2, dans laquelle ledit au moins un inhibiteur de trypsine comprend un inhibiteur de trypsine de graines de soja.

5. Composition destinée à être utilisée selon l'une quelconque des revendications 1 à 4, dans laquelle ledit traitement comprend une administration par administration par voie orale dudit tériparatide dans une quantité de 400 µg à 20 mg par jour.

6. Composition destinée à être utilisée selon l'une quelconque des revendications 1 à 5, dans laquelle ledit traitement comprend une administration par administration par voie orale dudit tériparatide dans une quantité dans une plage allant de 100 à 3 000 µg.

7. Composition destinée à être utilisée selon la revendication 6, dans laquelle ladite quantité est dans une plage allant de 750 à 3 000 µg.

8. Composition destinée à être utilisée selon l'une quelconque des revendications 1 à 7, dans laquelle ladite composition est destinée à une administration par administration par voie orale trois ou quatre fois par jour.

9. Composition destinée à être utilisée selon l'une quelconque des revendications 1 à 8, dans laquelle la composition est formulée comme un comprimé.
